# EUROPEAN PATENT APPLICATION

(11) **EP 4 071 239 A1**
(43) Date of publication of application: **12.10.2022**
(21) Application number: 20906474.0
(22) Date of filing: 25.12.2020
(51) Int. Cl.: C12N 5/0789, A61K 35/28, A61P 7/00, A61P 43/00, C12N 5/07

(54) **METHOD FOR CULTURING HEMATOPOIETIC STEM CELLS**

(30) Priority: 26.12.2019 JP 2019237030
(71) Applicant: Sumitomo Pharma Co., Ltd., Osaka 541-0045 (JP); NEXTGEM INC., Tokyo 141-0021 (JP)
(72) Inventor: NAKANE Atsushi, Kobe-shi, Hyogo 650-0047 (JP); NAGATA Hidetaka, Osaka-shi, Osaka 554-0022 (JP); ASANO Shigehiro, Osaka-shi, Osaka 554-0022 (JP); MIYANISHI Masanori, Kobe-shi, Hyogo 650-0047 (JP); SUDA Hitoshi, Osaka-shi, Osaka 554-0022 (JP); SHIODA Yusuke, Osaka-shi, Osaka 554-0022 (JP)
(74) Representative: Hoffmann Eitle
(86) International application number: PCT/JP2020/048917
(87) International publication number: WO 2021/132627

(57) **Abstract**

An object of the present invention is to provide a method for culturing one or more hematopoietic stem cells applicable to hematopoietic stem cell transplantation, and a method for producing such one or more hematopoietic stem cells. The method of the present invention for culturing one or more hematopoietic stem cells comprises: culturing a cell population including hematopoietic stem cells in a culture medium containing at least one compound represented by formula (1) or a salt thereof:

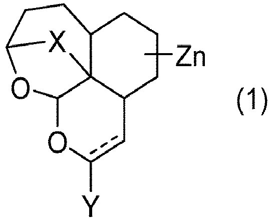

## Description

### Technical Field

The present invention relates to a method for culturing a hematopoietic stem cell. The present invention also relates to a method for producing a hematopoietic stem cell. Furthermore, the present invention relates to a culture medium for hematopoietic stem cells for growing hematopoietic stem cells ex vivo.

### Background Art

Hematopoietic stem cells (HSCs) are primarily present in the bone marrow, defined as blood cells having self-renewal capacity and multipotency, and used for hematopoietic stem cell transplantation and the like. However, hematopoietic stem cell transplantation suffers from shortage of high-quality bone marrow sources. Purification of hematopoietic stem cells of high quality (e.g., long-term hematopoietic stem cells) followed by in vitro large-scale growth thereof is contemplated as one of solutions for that problem, whereas no specific marker that enables purification of long-term hematopoietic stem cells (long-term HSCs: LT-HSCs) has not been known, and no method for culturing them has been established either.

Meanwhile, homeobox B5 (Hoxb5) has been recently identified as a marker that is expressed specifically in mouse long-term hematopoietic stem cells (Patent Literature 1, Non Patent Literature 1).

An indole derivative has been reported as a compound that accelerates the growth of hematopoietic stem cells (see Patent Literature 2, Non Patent Literature 2), whereas a method of maintenance culture of hematopoietic stem cells, in particular, of long-term hematopoietic stem cells has been needed.

### Citation List

### Patent Literature

Patent Literature 1: U.S. Patent Application Publication No. 2017/0350879
Patent Literature 2: WO 2013/110198

### Non Patent Literature

Non Patent Literature 1: James Y. Chen et al., "Hoxb5 marks long-term haematopoietic stem cells and reveals ahomogenous perivascular niche", Nature, Vol 530, 223-227 (2016)
Non Patent Literature 2: Fares I et al. (2014) Science 345, 1509-1512

### Summary of Invention

### Technical Problem

An object of the present invention is to provide a method for culturing one or more hematopoietic stem cells applicable to hematopoietic stem cell transplantation, and a method for producing such one or more hematopoietic stem cells.

### Solution to Problem

To solve the above problem, the present inventors carried out screening for low-molecular-weight compounds effective for the growth of long-term hematopoietic stem cells by using Hoxb5-positive hematopoietic stem cells isolated from a reporter mouse that expresses a fluorescent protein (mCherry) in the endogenous promoter of a Hoxb5 gene, which is a marker specific to mouse long-term hematopoietic stem cells. The results found that long-term hematopoietic stem cells can be grown with a culture medium containing a compound described later such as artemether, leading to the completion of the present invention.

Specifically, the present invention relates to, for example, the following:
[1] A method for culturing one or more hematopoietic stem cells, comprising:
   culturing a cell population including hematopoietic stem cells in a culture medium containing at least one compound represented by formula (1) or a salt thereof: 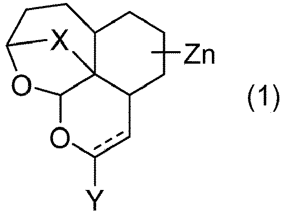 wherein 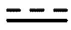

   is a single bond or a double bond;
   X is O-O or O;
   Y is a hydrogen atom, hydroxy, oxo, mercapto, carboxy, carbamoyl, cyano, OR¹, SR¹, SOR¹, SO₂R¹, COR¹, OCOR¹, R², COOR¹, NR³R⁴, NR⁵COR¹, NR⁵SO₂R¹, NR⁵CONR³R⁴, or a halogen;
   Z is attached to a carbon at an arbitrary position of the fused ring, and each Z is independently a C₁₋₆ alkyl;
   n is an integer of 0 to 10;
   R¹ is an optionally substituted alkyl, an optionally substituted cycloalkyl, an optionally substituted aryl, an optionally substituted heteroaryl, or an optionally substituted aliphatic heterocyclic group;
   R² is an optionally substituted alkyl, an optionally substituted alkenyl, an optionally substituted alkynyl, an optionally substituted cycloalkyl, an optionally substituted aryl, an optionally substituted heteroaryl, or an optionally substituted aliphatic heterocyclic group; and
   R³, R⁴, and R⁵ are each independently a hydrogen atom, an optionally substituted alkyl, an optionally substituted cycloalkyl, an optionally substituted aliphatic heterocyclic group, an optionally substituted aryl, or an optionally substituted heteroaryl, wherein R³ and R⁴ can be combined with the adjacent nitrogen atom to form a 4- to 10-membered nitrogen-containing aliphatic heterocyclic ring optionally containing additional one or two atoms selected from an oxygen atom, a nitrogen atom, and a sulfur atom, which constitutes the nitrogen-containing aliphatic heterocyclic ring, and the nitrogen-containing aliphatic heterocyclic ring is optionally substituted.
[2] The method for culturing one or more hematopoietic stem cells according to [1], wherein the compound of the formula (1) is a compound represented by formula (2): 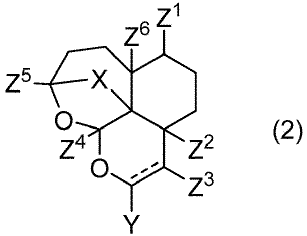 wherein 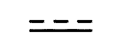

   is a single bond or a double bond;
   X and Y are as defined in the formula (1); and
   Z¹, Z², Z³, Z⁴, Z⁵, and Z⁶ are each independently a hydrogen atom or a C₁₋₆ alkyl.
[3] The method for culturing one or more hematopoietic stem cells according to [2], wherein the compound of the formula (2) is a compound represented by formula (3-1), (3-2), or (3-3): 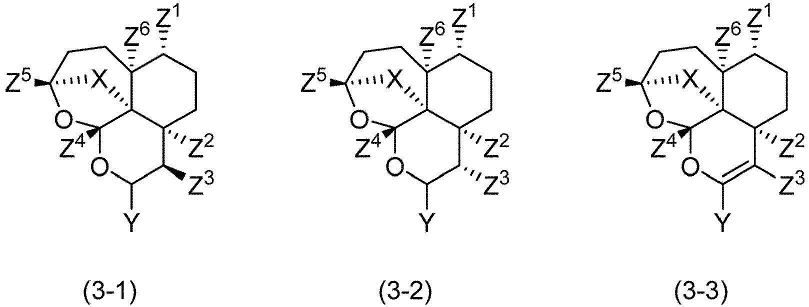 wherein
   X and Y are as defined in the formula (1); and
   Z¹, Z², Z³, Z⁴, Z⁵, and Z⁶ are each independently a hydrogen atom or a C₁₋₆ alkyl.
[4] The method for culturing one or more hematopoietic stem cells according to [3], wherein, in the formula (3-1), (3-2), or (3-3), Z¹, Z², Z³, Z⁴, Z⁵, and Z⁶ are each independently a hydrogen atom or methyl.
[5] The method for culturing one or more hematopoietic stem cells according to [1], wherein the compound of the formula (1) is a compound represented by formula (4-1), (4-2), or (4-3): 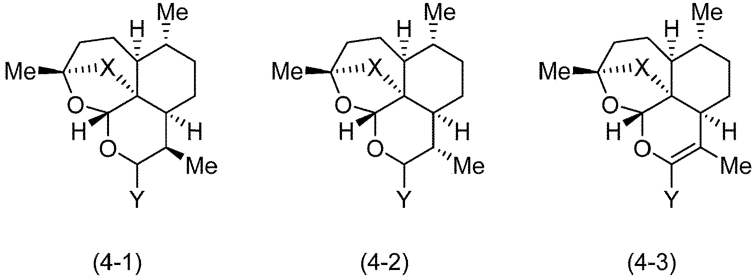 wherein
   X and Y are as defined in the formula (1).
[6] The method for culturing one or more hematopoietic stem cells according to any one of [1] to [5], wherein Y is a hydrogen atom, hydroxy, oxo, carboxy, carbamoyl, OR¹, SR¹, SO₂R¹, COR¹, OCOR¹, R², NR³R⁴, NR⁵COR¹, NR⁵SO₂R¹, or a fluorine atom.
[7] The method for culturing one or more hematopoietic stem cells according to any one of [1] to [6], wherein,
   if 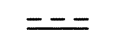

   is a double bond, then Y is a hydrogen atom or hydroxy, and
   if 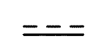

   is a single bond, then
   Y is hydroxy, oxo, carboxy, carbamoyl, OR¹, SR¹, SO₂R¹, OCOR¹, R², NR³R⁴, NR⁵COR¹, or a fluorine atom;
   R¹ is a C₁₋₈ alkyl optionally substituted with one to seven substituents selected from Group 1, a C₃₋₈ cycloalkyl optionally substituted with one to three groups selected from Group 1, phenyl optionally substituted with one to five groups selected from Group 1, a 4- to 10-membered aliphatic heterocyclic group containing one to four endocyclic heteroatoms selected from an oxygen atom, a nitrogen atom, and a sulfur atom and optionally substituted with one to three groups selected from Group 1, or a 5- to 10-membered heteroaryl containing one to three endocyclic heteroatoms selected from an oxygen atom, a nitrogen atom, and a sulfur atom and optionally substituted with one to four groups selected from Group 1;
   R² is a C₁₋₈ alkyl optionally substituted with one to seven substituents selected from Group 2, a C₁₋₈ alkenyl optionally substituted with one to seven substituents selected from Group 2, a C₃₋₈ cycloalkyl optionally substituted with one to three groups selected from Group 1, phenyl optionally substituted with one to five groups selected from Group 1, a 4-to 10-membered aliphatic heterocyclic group containing one to four endocyclic heteroatoms selected from an oxygen atom, a nitrogen atom, and a sulfur atom and optionally substituted with one to three groups selected from Group 1, or a 5- to 10-membered heteroaryl containing one to three endocyclic heteroatoms selected from an oxygen atom, a nitrogen atom, and a sulfur atom and optionally substituted with one to four groups selected from Group 1;
   R³ and R⁴ are each independently a hydrogen atom, a C₁₋₆ alkyl optionally substituted with one to seven substituents selected from Group 1, or phenyl optionally substituted with one to five groups selected from Group 1, wherein R³ and R⁴ can be combined with the adjacent nitrogen atom to form a 3- to 8-membered nitrogen-containing aliphatic heterocyclic ring optionally containing additional one or two atoms selected from oxygen atom, nitrogen atom, or sulfur atom, which constitutes the 3- to 8-membered nitrogen-containing aliphatic heterocyclic ring, and the nitrogen-containing aliphatic heterocyclic ring is optionally substituted with one to three substituents selected from substituents of Group 1 shown below; and
   R⁵ is a hydrogen atom or a C₁₋₃ alkyl:
   [Group 1]
      carboxy, hydroxy, a C₁₋₈ alkoxy, a C₁₋₈ alkylcarbonyl, a C₁₋₈ alkylcarbonyloxy, a C₁₋₈ alkoxycarbonyl, mercapto, a C₁₋₈ alkylsulfanyl, a C₁₋₈ alkylsulfonyl, carbamoyl optionally substituted with one or two C₁₋₈ alkyls, amino optionally substituted with one or two C₁₋₈ alkyls, C₁₋₈ alkylcarbonylamino, C₁₋₈ alkylsulfonylamino, phenyl optionally substituted with one to five groups selected from Group 3, a 5- or 6-membered heteroaryl containing one to three endocyclic heteroatoms selected from an oxygen atom, a nitrogen atom, and a sulfur atom and optionally substituted with one to four groups selected from Group 3, a C₃₋₈ cycloalkyl optionally substituted with one to three groups selected from Group 3, a 4- to 10-membered aliphatic heterocyclic group containing one to four endocyclic heteroatoms selected from an oxygen atom, a nitrogen atom, and a sulfur atom and optionally substituted with one to three groups selected from Group 3, and a halogen;
   [Group 2]
      carboxy, hydroxy, a C₁₋₈ alkoxy, a C₁₋₈ alkylcarbonyl, a C₁₋₈ alkylcarbonyloxy, a C₁₋₈ alkoxycarbonyl, mercapto, a C₁₋₈ alkylsulfanyl, a C₁₋₈ alkylsulfonyl, carbamoyl optionally substituted with one or two C₁₋₈ alkyls, amino, phenyl optionally substituted with one to five groups selected from Group 3, a 5- or 6-membered heteroaryl containing one to three endocyclic heteroatoms selected from an oxygen atom, a nitrogen atom, and a sulfur atom and optionally substituted with one to four groups selected from Group 3, a C₃₋₈ cycloalkyl optionally substituted with one to three groups selected from Group 3, a 4- to 8-membered aliphatic heterocyclic group containing one to four endocyclic heteroatoms selected from an oxygen atom, a nitrogen atom, and a sulfur atom and optionally substituted with one to three groups selected from Group 3, and a halogen; and
   [Group 3]
      carboxy, hydroxy, a C₁₋₈ alkyl, a C₁₋₈ alkoxy, a C₁₋₈ alkylcarbonyl, a C₁₋₈ alkylcarbonyloxy, a C₁₋₈ alkoxycarbonyl, mercapto, a C₁₋₈ alkylsulfanyl, a C₁₋₈ alkylsulfonyl, amino optionally substituted with one or two C₁₋₈ alkyls, carbamoyl optionally substituted with one or two C₁₋₈ alkyls, a C₁₋₈ alkylcarbonylamino, a C₁₋₈ alkylsulfonylamino, and a halogen.
[8] The method for culturing one or more hematopoietic stem cells according to any one of [1] to [7], wherein 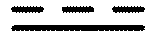
   is a single bond;
   Y is hydroxy, oxo, carboxy, carbamoyl, OR¹, SR¹, SO₂R¹, OCOR¹, R², NR³R⁴, NR⁵COR¹, NR⁵SO₂R¹, or a fluorine atom;
   R¹ is a C₁₋₄ alkyl optionally substituted with one to three groups selected from Group 1', a C₃₋₆ cycloalkyl optionally substituted with one to three groups selected from Group 1', phenyl optionally substituted with one to three groups selected from Group 1', a 4- to 6-membered aliphatic heterocyclic group containing one to three endocyclic heteroatoms selected from an oxygen atom, a nitrogen atom, and a sulfur atom and optionally substituted with one to three groups selected from Group 1', or a 5- or 6-membered heteroaryl containing one to three endocyclic heteroatoms selected from an oxygen atom, a nitrogen atom, and a sulfur atom and optionally substituted with one to three groups selected from Group 1';
   R² is a C₁₋₄ alkyl optionally substituted with one to three groups selected from Group 2', a C₃₋₆ cycloalkyl optionally substituted with one to three groups selected from Group 1', phenyl optionally substituted with one to three groups selected from Group 1', a 4- to 6-membered aliphatic heterocyclic group containing one to three endocyclic heteroatoms selected from an oxygen atom, a nitrogen atom, and a sulfur atom and optionally substituted with one to three groups selected from Group 1', or a 5- or 6-membered heteroaryl containing one to three endocyclic heteroatoms selected from an oxygen atom, a nitrogen atom, and a sulfur atom and optionally substituted with one to three groups selected from Group 1';
   R³ is a hydrogen atom or a C₁₋₃ alkyl;
   R⁴ is a hydrogen atom, a C₁₋₄ alkyl optionally substituted with one to three groups selected from Group 1', a C₃₋₆ cycloalkyl optionally substituted with one to three groups selected from Group 1', phenyl optionally substituted with one to three groups selected from Group 1', a 4- to 6-membered aliphatic heterocyclic group containing one to three endocyclic heteroatoms selected from an oxygen atom, a nitrogen atom, and a sulfur atom and optionally substituted with one to three groups selected from Group 1', or a 5- or 6-membered heteroaryl containing one to three endocyclic heteroatoms selected from an oxygen atom, a nitrogen atom, and a sulfur atom and optionally substituted with one to three groups selected from Group 1',
   wherein R³ and R⁴ can be combined with the adjacent nitrogen atom to form a 3- to 8-membered nitrogen-containing aliphatic heterocyclic ring optionally containing additional one or two atoms selected from an oxygen atom, a nitrogen atom and a sulfur atom, which constitutesthe 3-to 8-membered nitrogen-containing aliphatic heterocyclic ring, and the nitrogen-containing aliphatic heterocyclic ring is optionally substituted with one to three groups selected from substituents of Group 1' shown below; and
   R⁵ is a hydrogen atom:
   [Group 1']
      a fluorine atom, hydroxy, carboxy, amino, a C₁₋₄ alkoxy, a C₁₋₄ alkylcarbonyloxy, phenyl optionally substituted with one to three groups selected from Group 3', a C₃₋₆ cycloalkyl optionally substituted with one to three groups selected from Group 3', and a 4- to 6-membered aliphatic heterocyclic group containing one to three endocyclic heteroatoms selected from an oxygen atom, a nitrogen atom, and a sulfur atom and optionally substituted with one to three groups selected from Group 3';
   [Group 2']
      a fluorine atom, hydroxy, carboxy, amino, a C₁₋₄ alkoxy, a C₁₋₄ alkylcarbonyloxy, a C₁₋₄ alkoxycarbonyl, carbamoyl optionally substituted with one or two C₁₋₈ alkyls, phenyl optionally substituted with one to three groups selected from Group 3', a C₃₋₆ cycloalkyl optionally substituted with one to three groups selected from Group 3', and a 4- to 6-membered aliphatic heterocyclic group containing one to three endocyclic heteroatoms selected from an oxygen atom, a nitrogen atom, and a sulfur atom and optionally substituted with one to three groups selected from Group 3'; and
   [Group 3']
      a fluorine atom, hydroxy, and a C₁₋₄ alkyl.
[9] The method for culturing one or more hematopoietic stem cells according to any one of [1] to [8], wherein X is O-O.
[10] The method for culturing one or more hematopoietic stem cells according to [1], wherein the compound of the formula (1) is a compound selected from the group consisting of the following compounds: artemether;
   artemisinin;
   artenimol;
   artemotil;
   artesunate;
   (3R,5aS,6R,8aS,9R,10R,12S,12aR)-10-fluoro-3,6,9-trimethyldecahydro-12H-3,12-epoxypyrano[4,3-j][1,2]benzodioxepine;
   (3R,5aS,6R,8aS,9R,10S,12R,12aR)-3,6,9-tnmethyl-10-phenyldecahydro-12H-3,12-epoxypyrano[4,3-j][1,2]benzodioxepine;
   (3R,5aS,6R,8aS,9R,10S,12S,12aR)-3,6,9-trimethyl-10-(methylsulfanyl)decahydro-12H-3,12-epoxypyrano[4,3-j][1,2]benzodioxepine;
   (3R,5aS,6R,8aS,9R,10R,12S,12aR)-3,6,9-trimethyl-10-(methylsulfanyl)decahydro-12H-3,12-epoxypyrano[4,3-j][1,2]benzodioxepine;
   (3R,5aS,6R,8aS,9R,10S,12S,12aR)-10-(methanesulfonyl)-3,6,9-trimethyldecahydro-12H-3,12-epoxypyrano[4,3-j][1,2]benzodioxepine; (3R,5aS,6R,8aS,9R,10R,12R,12aR)-3,6,9,10-tetramethyldecahydro-12H-3,12-epoxypyrano[4,3-j][1,2]benzodioxepine;
   (3R,5aS,6R,8aS,9R,10R,12R,12aR)-10-(furan-2-yl)-3,6,9-trimethyldecahydro-12H-3,12-epoxypyrano[4,3-j][1,2]benzodioxepine; (3R,5aS,6R,8aS,9R,10R,12R,12aR)-3,6,9-trimethyldecahydro-12H-3,12-epoxypyrano[4,3-j][1,2]benzodioxepine-10-carboxylic acid;
   4-phenyl-1-[(3R,5aS,6R,8aS,9R,10R,12R,12aR)-3,6,9-trimethyldecahydro-12H-3,12-epoxypyrano[4,3-j][1,2]benzodioxepin-10-yl]-1H-1,2,3-triazole;
   N-[(3R,5aS,6R,8aS,9R,10R,12R,12aR)-3,6,9-trimethyldecahydro-12H-3,12-epoxypyrano[4,3-j][1,2]benzodioxepin-10-yl]acetamide;
   N-[(3R,5aS,6R,8aS,9R,10R,12R,12aR)-3,6,9-trimethyldecahydro-12H-3,12-epoxypyrano[4,3-j][1,2]benzodioxepin-10-yl]methanesulfonamide;
   (3R,5aS,6R,8aS,9R,10R,12R,12aR)-N,N,3,6,9-pentamethyldecahydro-12H-3,12-epoxypyrano[4,3-j][1,2]benzodioxepin-10-amine;
   4-[(3R,5aS,6R,8aS,9R,10R,12R,12aR)-3,6,9-trimethyldecahydro-12H-3,12-epoxypyrano[4,3-j][1,2]benzodioxepin-10-yl]morpholine;
   (3R,5aS,6R,8aS,9R,10S,12R,12aR)-3,6,9-trimethyldecahydro-12H-3,12-epoxypyrano[4,3-j][1,2]benzodioxepin-10-yl acetate;
   (3R,5aS,6R,8aS,9R,10S,12R,12aR)-3,6,9-trimethyldecahydro-12H-3,12-epoxypyrano[4,3-j][1,2]benzodioxepine-10-carboxamide;
   (3R,5aS,6R,8aS,9R, 10R, 12R,12aR)-3,6,9-trimethyldecahydro-12H-3,12-epoxypyrano[4,3-j][1,2]benzodioxepine-10-carboxamide;
   1-[(3R,5aS,6R,8aS,9R,10S,12R,12aR)-3,6,9-trimethyldecahydro-12H-3,12-epoxypyrano[4,3-j][1,2]benzodioxepin-10-yl]methanamine;
   1-[(3R,5aS,6R,8aS,9R,10R, 12R, 12aR)-3,6,9-trimethyldecahydro-12H-3,12-epoxypyrano[4,3-j][1,2]benzodioxepin-10-yl]methanamine;
   (3R,5aS,6R,8aS,9R,10S,12R,12aR)-3,6,9-trimethyl-10-[(propan-2-yl)oxy]decahydro-12H-3,12-epoxypyrano[4,3-j][1,2]benzodioxepine;
   (3R,5aS,6R,8aS,9R,10S,12R,12aR)-10-(cyclohexyloxy)-3,6,9-trimethyldecahydro-12H-3,12-epoxypyrano[4,3-j][1,2]benzodioxepine;
   (3R,5aS,6R,8aS,9R,10S,12R,12aR)-3,6,9-trimethyl-10-phenoxydecahydro-12H-3,12-epoxypyrano[4,3-j][1,2]benzodioxepine;
   (3R,5aS,6R,8aS,9R,10S,12R, 12aR)-3,6,9-trimethyl-10-(2,2,2-trifluoroethoxy)decahydro-12H-3,12-epoxypyrano[4,3-j][1,2]benzodioxepine;
   (3R,5aS,6R,8aS,9R,10S,12R,12aR)-10-(2-methoxyethoxy)-3,6,9-trimethyldecahydro-12H-3,12-epoxypyrano[4,3-j][1,2]benzodioxepine;
   (3R,5aS,6R,8aS,9R,10S,12R,12aR)-10-(benzyloxy)-3,6,9-trimethyldecahydro-12H-3,12-epoxypyrano[4,3-j][1,2]benzodioxepine;
   (3R,5aS,6R,8aS,9R,10R,12R,12aR)-3,6,9-trimethyl-10-phenoxydecahydro-12H-3,12-epoxypyrano [4,3-j][1,2]benzodioxepine;
   2-{[(3R,5aS,6R,8aS,9R,10S,12R,12aR)-3,6,9-trimethyldecahydro-12H-3,12-epoxypyrano[4,3-j][1,2]benzodioxepin-10-yl]oxy}ethyl acetate;
   2-{[(3R,5aS,6R,8aS,9R,10R,12R,12aR)-3,6,9-trimethyldecahydro-12H-3,12-epoxypyrano[4,3-j][1,2]benzodioxepin-10-yl]oxy}ethyl acetate;
   (3R,5aS,6R,8aS,12R,12aR)-3,6,9-trimethyl-3,4,5,5a,6,7,8,8a-octahydro-12H-3,12-epoxypyrano[4,3-j][1,2] benzodioxepine;
   (3R,3aS,6R,6aS,9S,10aS,10bR)-3,6,9-trimethyloctahydro-10aH-9,10b-epoxypyrano[4,3,2-jk][2]benzoxepin-2(3H)-one; and
   (3R,5aS,6R,8aS,9R,10R,12R,12aR)-10-methoxy-3,6,9-trimethyldecahydro-12H-3,12-epoxypyrano[4,3-j][1,2]benzodioxepine.
   [10-2] The method for culturing one or more hematopoietic stem cells according to [1], wherein the compound of the formula (1) is a compound selected from the group consisting of the following compounds:
      artemether;
      artemisinin;
      artenimol;
      artemotil;
      artesunate
      (3R,5aS,6R,8aS,9R,10S,12R,12aR)-3,6,9-trimethyldecahydro-12H-3,12-epoxypyrano[4,3-j][1,2]benzodioxepine-10-carboxamide;
      (3R,5aS,6R,8aS,9R,10R,12R,12aR)-3,6,9-trimethyldecahydro-12H-3,12-epoxypyrano[4,3-j][1,2]benzodioxepine-10-carboxamide;
      1-[(3R,5aS,6R,8aS,9R,10S,12R,12aR)-3,6,9-trimethyldecahydro-12H-3,12-epoxypyrano[4,3j][1,2]benzodioxepin-10-yl]methanamine;
      1-[3R,(5aS,6R,8aS,9R,10R,12R,12aR)-3,6,9-trimethyldecahydro-12H-3,12-epoxypyrano[4,3-j][1,2]benzodioxepin-10-yl]methanamine; and
      (3R,5aS,6R,8aS,9R,10S,12R,12aR)-10-(2-methoxyethoxy)-3,6,9-trimethyldecahydro-12H-3,12-epoxypyrano[4,3-j][1,2]benzodioxepine.
[11] The method for culturing one or more hematopoietic stem cells according to [1], wherein the compound of the formula (1) is selected from the group consisting of artemether, artemisinin, artenimol, artemotil, and artesunate.
[12] The method for culturing one or more hematopoietic stem cells according to any one of [1] to [11], wherein the hematopoietic stem cell is a CD34-positive cell.
[13] The method for culturing one or more hematopoietic stem cells according to any one of [1] to [12], wherein the hematopoietic stem cell is a long-term hematopoietic stem cell.
[14] The method for culturing one or more hematopoietic stem cells according to any one of [1] to [12], wherein the hematopoietic stem cell is a Hoxb5-positive mouse hematopoietic stem cell.
[15] The method for culturing one or more hematopoietic stem cells according to any one of [1] to [14], wherein the proportion of hematopoietic stem cells to the total cell count in the cell population after culture is 10% or more of the proportion of hematopoietic stem cells to the total cell count in the cell population before culture.
[16] The method for culturing one or more hematopoietic stem cells according to any one of [1] to [15], wherein the number of hematopoietic stem cells in the cell population after culture is three or more times the number of hematopoietic stem cells in the cell population before culture.
   [16-2] The method for culturing one or more hematopoietic stem cells according to any one of [1] to [15], wherein the number of hematopoietic stem cells in the cell population after culture is 10 or more times the number of hematopoietic stem cells in the cell population before culture.
[17] The method for culturing one or more hematopoietic stem cells according to any one of [1] to [16], wherein the culture medium is a culture medium further containing UM171 or a derivative thereof.
[18] A method for producing one or more hematopoietic stem cells, comprising:
   a step of preparing a cell population including hematopoietic stem cells; and
   a step of culturing the cell population including hematopoietic stem cells, wherein the cell population including hematopoietic stem cells is cultured by the method for culturing one or more hematopoietic stem cells according to any one of [1] to [17].
[19] A hematopoietic stem cell obtained by culturing by the method for culturing one or more hematopoietic stem cells according to any one of [1] to [17].
[20] A hematopoietic stem cell produced by the method for producing one or more hematopoietic stem cells according to [18] .
[21] A reagent for culturing one or more hematopoietic stem cells, comprising at least one compound represented by the formula (1) or a salt thereof.

### Advantageous Effects of Invention

The culture method of the present invention allows culture and growth of hematopoietic stem cells, in particular, long-term hematopoietic stem cells with the self-renewal capacity and multipotency maintained, consequently allowing production of long-term hematopoietic stem cells suitable for transplantation. Hematopoietic stem cells obtained by the culture method are applicable to hematopoietic stem cell transplantation.

### Brief Description of Drawings

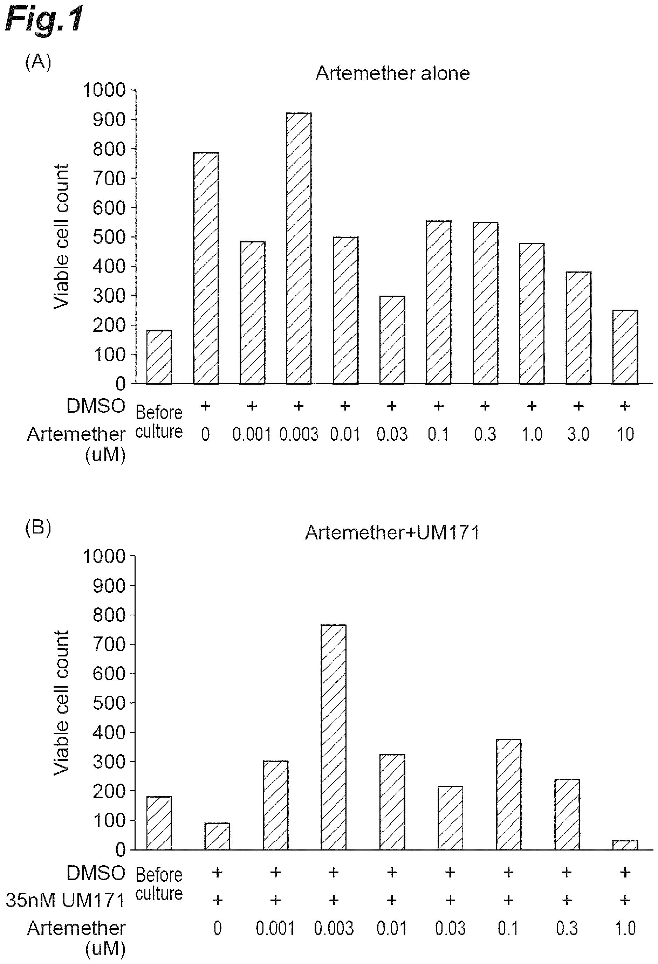

[Figure 1] Figure 1 shows results of culture of cell populations including human hematopoietic stem cells, wherein (A) shows viable cell counts after culture with a culture medium containing artemether alone, and (B) shows those after culture with a culture medium containing UM171 in addition to artemether.

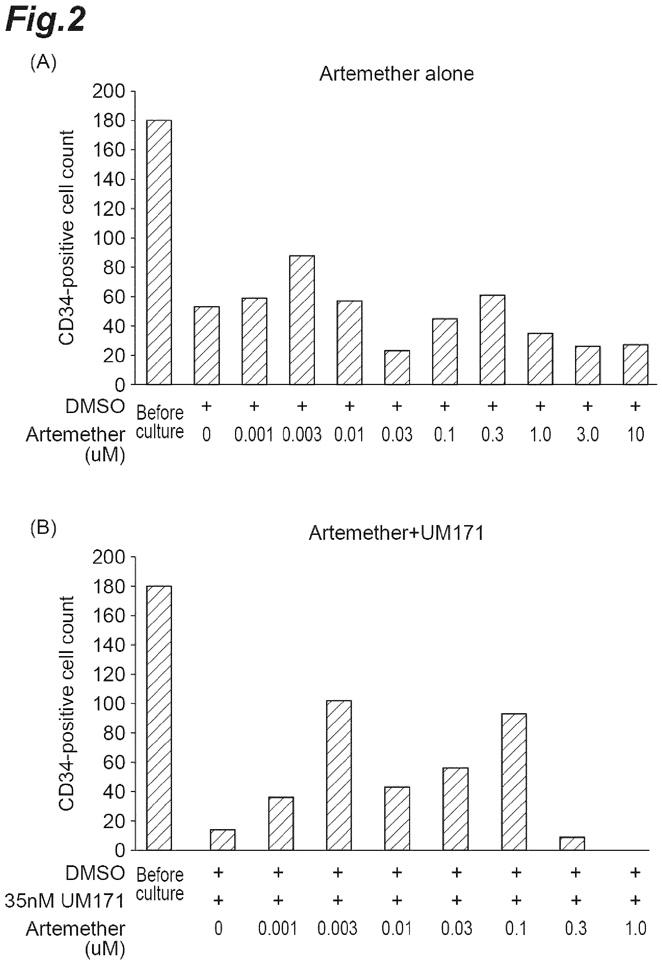

[Figure 2] Figure 2 shows results of culture of cell populations including human hematopoietic stem cells, wherein (A) shows CD34-positive cell counts after culture with a culture medium containing artemether alone, and (B) shows those after culture with a culture medium containing UM171 in addition to artemether.

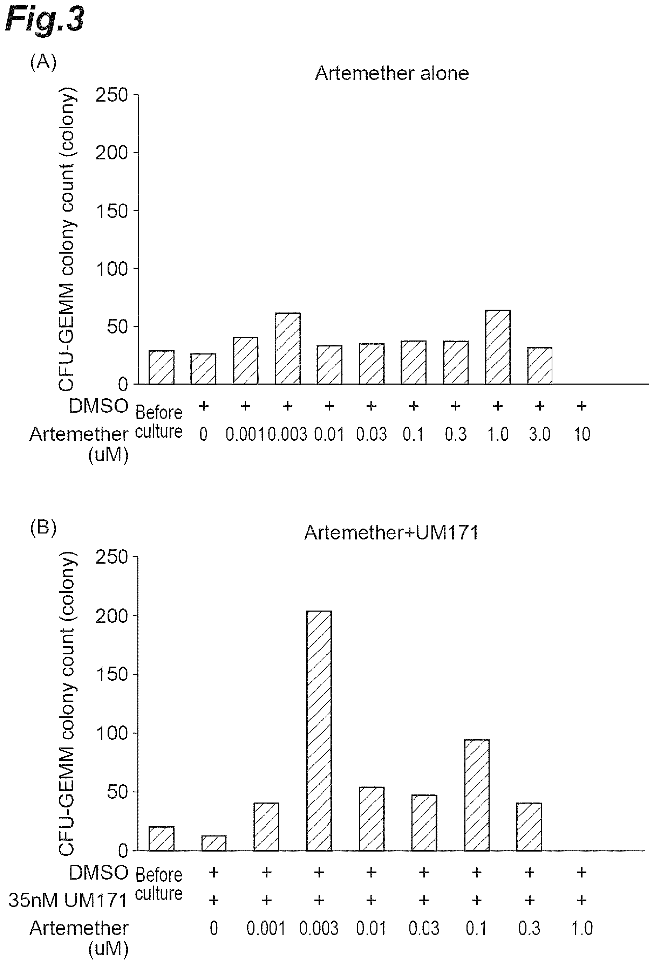

[Figure 3] Figure 3 shows results of culture of cell populations including human hematopoietic stem cells, wherein (A) shows the numbers of CFU-GEMM colonies after culture with a culture medium containing artemether alone, and (B) shows those after culture with a culture medium containing UM171 in addition to artemether.

### Description of Embodiments

### [Culture Method]

In an embodiment, the method of the present invention for culturing one or more hematopoietic stem cells comprises: culturing a cell population including hematopoietic stem cells in a culture medium containing at least one compound represented by formula (1) or a salt thereof.

In an embodiment, the method of the present invention for culturing one or more hematopoietic stem cells comprises: culturing a cell population including hematopoietic stem cells in a culture medium containing at least one compound selected from the group consisting of artemether, artemisinin, artenimol, artemotil, and artesunate or a derivative thereof, or a salt of any of them.

### Hematopoietic Stem Cells

"Hematopoietic stem cells (HSCs)" are blood cells that are primarily present in the bone marrow and have self-renewal capacity and multipotency that allows differentiation into all types of blood cells. Self-renewal is production of a cell having the same functions and characteristics as the original cell has through cell division. Consequently, cells produced through self-renewal of a hematopoietic stem cell have multipotency for all types of blood cells and self-renewal capacity.

Hematopoietic stem cells are classified according to their abilities to maintain hematopoiesis into short-term hematopoietic stem cells and long-term hematopoietic stem cells. In general, long-term hematopoietic stem cells refer to hematopoietic stem cells capable of sustaining self-renewal capacity for a longer period of time than short-term hematopoietic stem cells do, and long-term hematopoietic stem cells have long-term bone marrow reconstruction potential (e.g., ability to reconstruct the bone marrow even in secondary transplantation). By contrast, short-term hematopoietic stem cells can reconstruct the bone marrow in vitro or in primary transplantation, but cannot maintain this ability in secondary transplantation. Secondary transplantation is transplantation of hematopoietic stem cells derived from bone marrow reconstructed through primary transplantation to another individual. Herein, hematopoietic stem cells refer to both short-term hematopoietic stem cells and long-term hematopoietic stem cells, unless specifically stated otherwise.

Differentiation of a hematopoietic stem cell is the phenomenon that a long-term hematopoietic stem cell (long-term HSC: LT-HSC) transforms into a short-term hematopoietic stem cell, the short-term hematopoietic stem cell into a multipotent hematopoietic progenitor cell, the multipotent hematopoietic progenitor cell into an oligopotent hematopoietic progenitor cell, the oligopotent hematopoietic progenitor cell into a unipotent hematopoietic progenitor cell, and the unipotent hematopoietic progenitor cell into a mature cell having unique functions, specifically, a mature blood cell such as an erythrocyte, a leukocyte, a megakaryocyte, and a platelet. Among the series of cell types associated with the differentiation of hematopoietic stem cells, only long-term hematopoietic stem cells and short-term hematopoietic stem cells are cell types having self-renewal capacity, and the cells in the downstream of multipotent hematopoietic progenitor cells are said to have division capacity associated with differentiation but lack self-renewal capacity.

Herein, the term "growth" for hematopoietic stem cells means that the number of hematopoietic stem cells increases, for example, by culturing the hematopoietic stem cells, typically ex vivo. Such growth of hematopoietic stem cells can be achieved by self-renewal of at least some of the hematopoietic stem cells to form hematopoietic stem cells having common characteristics. While hematopoietic stem cells are known to undergo self-renewal in vivo, however, no specific factor that induces the self-renewal has been identified, and it is difficult to induce hematopoietic stem cells ex vivo to undergo self-renewal like that in vivo and allow them to grow. The culture method of the present invention can allow hematopoietic stem cells to grow ex vivo with the self-renewal capacity and multipotency maintained.

Hematopoietic stem cells can be identified by using, as an indicator, an expressed marker, preferably a marker that is expressed specifically in hematopoietic stem cells and a marker expression of which is not found in hematopoietic stem cells (negative marker).

Examples of markers that are expressed specifically in mouse long-term hematopoietic stem cells include Hoxb5 (Non Patent Literature 1). In an embodiment, mouse long-term hematopoietic stem cells may be cells identifiable on the basis of at least one, as an indicator, selected from the group consisting of being Lineage-negative, being c-Kit-positive, being Sca-1-positive, being Flk2-negative, being CD34-negative or weakly CD34-positive, being CD150-positive, and being Hoxb5-positive.

On the other hand, mouse short-term hematopoietic stem cells have been confirmed not to express Hoxb5. In an embodiment, mouse short-term hematopoietic stem cells may be cells identifiable on the basis of at least one, as an indicator, selected from the group consisting of being Lineage-negative, being c-Kit-positive, being Sca-1-positive, being Flk2-negative, being CD34-negative or weakly CD34-positive, being CD150-positive, and being Hoxb5-negative.

Lineage markers collectively refer to antigens expressed in mature hematopoietic cells. Examples of lineage markers include, but are not limited to, Ter-119 (erythrocyte) and B220 (B cell). An example of human lineage markers has been reported, for example, in Notta F et al., Science. 2011 Jul 8; 333 (6039): 218-21, Sugimura R. et al., Nature. 2017 May 25; 545 (7655): 432-438, and Taya Y. et al., Science. 2016 Dec 2; 354 (6316): 1152-1155. Cells not expressing all or some of the lineage markers are occasionally expressed as Lineage-negative (Lin-negative or Lin⁻) cells. In an embodiment, mouse Lineage-negative cells may be cells not expressing, for example, one of Ter-119, B220, CD3, CD4, CD8a, Gr-1, CD11b, and IL-7R.

In an embodiment, human hematopoietic stem cells may be cells identifiable by the presence or absence of expression of at least one marker selected from the group consisting of CD34, CD38, Lineage, CD90, CD45RA, and CD49f, as an indicator (for example, CD34-positive cells; CD34-positive and CD38-negative cells; CD34-positive, CD38-negative, CD90-positive, and CD45RA-negative cells; CD34-positive and CD38-negative cells; CD90-negative and CD45RA-negative cells; CD34-positive, CD38-negative, CD90-negative, and CD45RA-positive cells). Long-term hematopoietic stem cells are believed to be present in a larger proportion in CD34-positive and CD38-negative cell populations among CD34-positive cell populations.

Herein, "positive" cells are cells expressing a specific marker protein, and "negative" cells mean cells not expressing a specific marker protein. For example, being CD34-positive indicates being a cell expressing CD (cluster of differentiation) 34 antigen on the cell surface. Being CD38-negative indicates being a cell not expressing CD38 antigen on the cell surface.

The presence or absence of expression of a specific marker protein can be determined by a method that would be well known to those skilled in the art. In an embodiment, such determination can be performed on the basis of whether significant increase in fluorescence intensity is found when cells are treated with a fluorescence-labeled antibody for a marker of interest, as compared with the case that the cells are treated with a fluorescence-labeled control antibody.

The above-mentioned markers are markers well known to those skilled in the art, and the gene sequences for and the amino acid sequences of the markers can be confirmed with use of GenBank or the like. Examples of the mouse Hoxb5 gene include GenBank Accession No.: 15413 (NC_000077.6), and examples of the mouse Hoxb5 protein include GenBank Accession No.: NP_032294.2. Commercially available products of antibodies or the like for the markers can be obtained, and expression or expression levels of the markers can be detected by using the antibodies.

The self-renewal capacity of hematopoietic stem cells can be determined by a method known to those skilled in the art. For example, such determination can be performed for human hematopoietic stem cells on the basis of increase in cell count identifiable by the presence or absence of expression of any of the above-mentioned markers (for example, CD34-positive and CD38-negative), as an indicator. In an embodiment, the self-renewal capacity of mouse hematopoietic stem cells can be determined on the basis of increase in cKit-positive-Lin-negative-Scal-positive cell count, as an indicator. In addition, the self-renewal capacity of mouse long-term hematopoietic stem cells can be determined. For example, such determination can be performed on the basis of increase in Hoxb5-positive cell count, as an indicator.

The multipotency of hematopoietic stem cells can be determined by subjecting cells obtained by culture to treatment to induce differentiation into at least two or more different types of hematopoietic progenitor cells or blood cells derived from them and confirming the differentiation into desired hematopoietic progenitor cells or blood cells.

A method of transplanting into immunodeficient mice is often used as a method for confirming functions of human hematopoietic stem cells or long-term hematopoietic stem cells. For example, immunodeficient mice which allow almost all types of human mature blood cells to be produced when human hematopoietic stem cells are transplanted (NOD.Cg-Prkdcscid Il2rgtm1Sug/Jic: hereinafter, "NOG mouse", Blood (2012), 100 (9): 1113-1124) can be used. Transplantation of cultured hematopoietic stem cells into such a mouse allows determination of engraftment potential to bone marrow, multipotency, and bone marrow reconstruction potential for the transplanted cells. Further, re-transplantation (secondary transplantation) of bone marrow collected from an immunodeficient mouse for which transplantation has been established into another NOG mouse allows determination of self-renewal capacity and long-term bone marrow reconstruction potential for the transplanted cells. Alternatively, long-term bone marrow reconstruction potential can be confirmed by successful engraftment of transplanted hematopoietic stem cells, which have been transplanted into an immunocompromised NOG mouse exposed to a lethal dose of radiation, for a long period of time (for 1 month or more, preferably for 2 months or more, 3 months or more, or 4 months or more after transplantation). For transplantation of human cells, the engraftment of transplanted cells can be confirmed by detecting cells expressing a human marker in the blood of the NOG mouse. Specifically, the leukocyte common antigen human CD45 can be detected with an antibody or the like for it. The proportion of human white blood cells (for example, human CD45-positive cells) in blood 1 month (preferably 2 months, 3 months, 4 months) after transplantation is needed to be higher than that of a comparative sample. Specifically, if the proportion is maintained or has increased as compared with that of a comparative sample, the long-term hematopoietic stem cells of interest can be confirmed to be maintained or have been amplified. In this context, the comparative sample may be, for example, a cell population obtained by a culture method under culture conditions matched except that the compound of the present invention is not contained, or a cell population before culture. The proportion of human white blood cells (for example, human CD45-positive cells) in blood 1 month (preferably, 2 months, 3 months, 4 months) after transplantation largely depends on experimental conditions including the number of transplanted cells. Accordingly, whether the proportion of human white blood cells (for example, human CD45-positive cells) in blood is high or low does not matter, and the proportion may be, for example, 0.1% or more, 0.5% or more, 1% or more, 5% or more, or 10% or more. The phenomenon that some cells in a recipient are replaced with cells (for example, white blood cells) derived from a donor is occasionally referred to as chimerism (or donor chimerism), and the proportion of cells (for example, white blood cells) derived from a donor is occasionally referred to as the chimerism. Specifically, in transplantation experiment with human cells into the above-mentioned NOG mouse, the phenomenon that some cells in the blood of the mouse are replaced with human white blood cells (for example, human CD45-positive cells) is occasionally referred to as chimerism, and the proportion of human white blood cells is occasionally referred to as the chimerism rate. Cells confirmed to have functions of hematopoietic stem cells through that method can be regarded as cells suitable for actual application to hematopoietic stem cell transplantation.

Thus, the culture methods according to [1] to [11] in the above can be regarded as methods for culturing a cell population that increases the ability to produce mature blood cells when being transplanted into a mammal.

Alternatively, the culture methods according to [1] to [11] in the above can be regarded as methods for culturing a cell population that increases at least one of, preferably all of the abilities of engraftment potential to bone marrow, multipotency, and bone marrow reconstruction potential.

Hematopoietic stem cells in the present specification may be derived from a mammal, and, for example, may be hematopoietic stem cells derived from a human, a monkey, a rat, or a mouse. Hematopoietic stem cells (a cell population including hematopoietic stem cells) can be prepared by a method described in (Step (1)) of [Production Method].

### Cell Population Including Hematopoietic Stem Cells to Be Cultured

The term "cell population" as used herein means a population in which the same type or different types of two or more cells are present. Preferably, the cell population is present in a medium such as a culture medium. Cell populations include cell suspensions and cell aggregates, and it is preferable that the cell population be in the form of a cell suspension or a cell aggregate.

The cell population including hematopoietic stem cells to be cultured is not limited as long as the cell population is a population of two or more cells including hematopoietic stem cells, and may be a cell population including hematopoietic stem cells (long-term hematopoietic stem cells and/or short-term hematopoietic stem cells) alone, or a cell population including hematopoietic stem cells and cells not being hematopoietic stem cells. Herein, the term "cell population including hematopoietic stem cells" refers to both a cell population including hematopoietic stem cells alone and a cell population including hematopoietic stem cells and cells not being hematopoietic stem cells, unless specifically stated otherwise. The hematopoietic stem cells may be preferably long-term hematopoietic stem cells. Consequently, the "cell population including hematopoietic stem cells" to be cultured in the present specification includes long-term hematopoietic stem cells.

Examples of the cells not being hematopoietic stem cells that can be included in the cell population include hematopoietic progenitor cells and mature blood cells.

Hematopoietic progenitor cells include multipotent hematopoietic progenitor cells, oligopotent hematopoietic progenitor cells, and unipotent hematopoietic progenitor cells. The classification, whether cells are multipotent, oligopotent, or unipotent, is that based on the degree of differentiation potential into blood cells.

Multipotent hematopoietic progenitor cells are cells having multipotency that allows differentiation into all types of blood cells, but being incapable of self-renewal. For this reason, although blood cells can be temporarily produced therefrom, depletion of hematopoietic progenitor cells occurs after all the cells have differentiated, resulting in failure in reconstruction of broken bone marrow.

Oligopotent hematopoietic progenitor cells are cells being capable of differentiating into several but not all types of blood cells, but being incapable of self-renewal. Examples thereof include granulocyte/monocyte progenitor cells (CFU-GEMM) and granulocyte/macrophage colony-forming cells (CFU-GM). GEMM stands for the first letters of granulocyte, erythrocyte, monocyte, and megakaryocyte.

Unipotent hematopoietic progenitor cells are cells being capable of differentiating into a single type of blood cells, but lacking self-renewal capacity. Examples of unipotent hematopoietic progenitor cells include erythroid burst-forming cells (BFU-E), which are erythroid progenitor cells.

A colony method (e.g., Ueda T. et al, J. Clin. Invest. (2000) 105: 1013-1021) is traditionally used as a function-based assay method for hematopoietic progenitor cells. The most immature colonies assayed by the colony method (also referred to as CFU assay) are mixed colonies in which erythrocytes and leukocytes coexist (hereinafter, referred to as "CFU-GEMM"). If the colony method is performed with a cell population including hematopoietic stem cells, the hematopoietic stem cells form various colonies through repeated differentiation. Accordingly, if many mixed colonies (CFU-GEMM) are formed from a cell population including specific hematopoietic stem cells, the cell population can be said to include therein many undifferentiated cell fractions including hematopoietic stem cells or long-term hematopoietic stem cells.

Thus, the culture methods according to [1] to [11] in the above can be regarded as methods for culturing one or more cells or cell population having an ability to form a mixed colony (CFU-GEMM) derived from hematopoietic stem cells.

Mature blood cells refer to a mature cell population formed from hematopoietic stem cells through hematopoietic progenitor cells. Examples thereof include erythrocytes, neutrophils, monocytes, eosinophils, basophils, macrophages, platelets, mast cells, T cells, B cells, NK cells, and NKT cells, and these can be obtained by allowing hematopoietic stem cells or hematopoietic progenitor cells in the upstream of the corresponding mature blood cells to differentiate. Those mature blood cells can be discriminated by using a known method with specific markers expressed in the cells. For example, CD33 is known as a marker for monocytes, macrophages, and granulocytes, CD41 as a megakaryocyte/platelet marker, CD235a as an erythrocyte marker, CD 19 as a B cell marker, and CD3 as a T cell marker.

The cell population including hematopoietic stem cells to be cultured in the present specification may be a cell population, for example, collected from blood or bone marrow, or one artificially produced from pluripotent stem cells (for example, iPS cells, ES cells). Examples of specific methods therefor include a method described in [Production Method].

### Compound

In an embodiment, the compound of the present invention is the above-mentioned compound of the formula (1) or a salt thereof, preferable examples thereof include a compound of the formula (2) or the formula (3-1), formula (3-2), or formula (3-2), or a salt thereof, and more preferable examples thereof include a compound of the formula (4-1), formula (4-2), or formula (4-3), or a salt thereof.

In an embodiment, the compound of the present invention is a compound represented by any one of chemical formulas shown below, or a salt thereof. The compounds are each a sesquiterpene lactone compound having antimalarial activity, and thought to be effective even for falciparum malaria with multidrug resistance (Jigang Wang et al., NATURE COMMUNICATIONS 2015 6: 10111 DOI: 10.1038). Among them, artemisinin is a compound separated from and named after annual wormwood (Artemisia annua, Chinese name: Qinghaosu), a plant that belongs to the genus Artemisia and has been traditionally used as a Chinese herbal medicine. 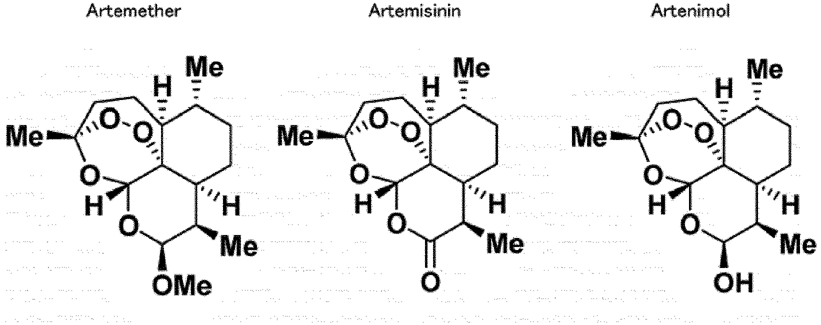 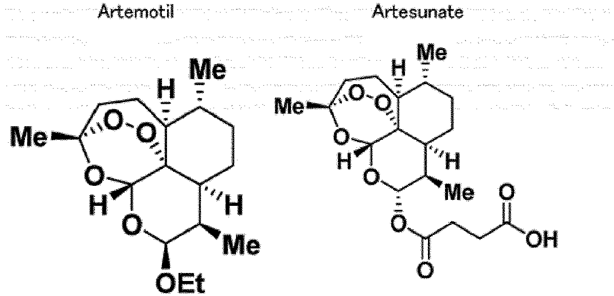 (In each formula, Me represents a methyl group, and Et represents an ethyl group.)

Artemisinin and derivatives thereof have been reported to have anticancer effect (Yin Kwan Wong et al., Med Res Rev 2017; 37: 1492-1517). According to the literature, the IC₅₀ of the antimalarial effect is in the order of several nM, and the IC₅₀ of the anticancer effect is approximately 100 µM. The effects of those compounds on hematopoietic stem cells have not been reported till now.

The compound applicable to the culture method of the present invention may be at least one compound selected from the group consisting of artemether, artemisinin, artenimol, artemotil, and artesunate, or a salt thereof, or a derivative of at least one compound selected from the group consisting of artemether, artemisinin, artenimol, artemotil, and artesunate, or a salt thereof. The compound of the formula (1) can be regarded as a derivative of artemether, artemisinin, artenimol, artemotil, or artesunate.

Each of the compounds of the formula (1), formula (2), formula (3-1), formula (3-2), formula (3-3), formula (4-1), formula (4-2), and formula (4-3), or a salt thereof will be described in the following.

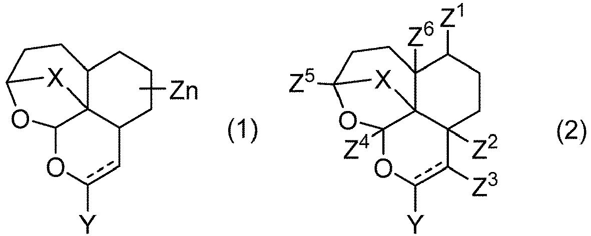

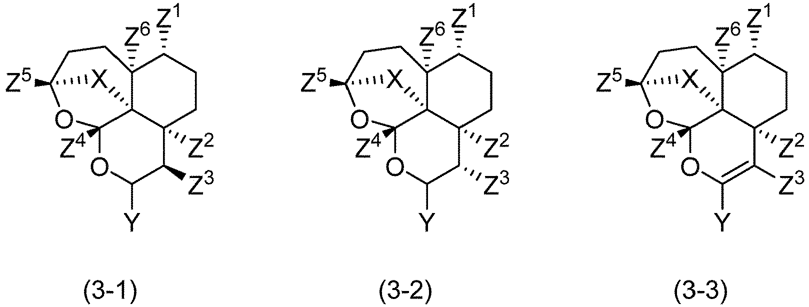

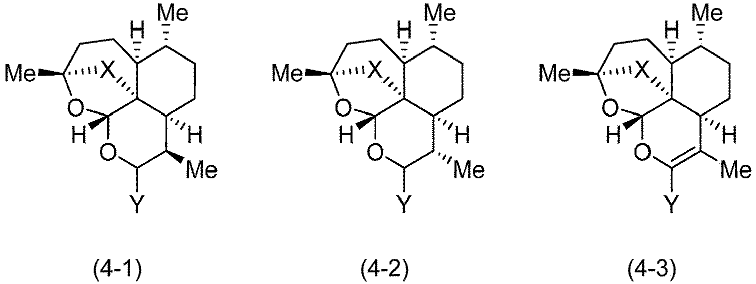

In the formula (1), 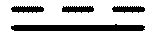
is a single bond or a double bond;
X is O-O or O;
Y is a hydrogen atom, hydroxy, oxo, mercapto, carboxy, carbamoyl, OR¹, SR¹, SOR¹, SO₂R¹, COR¹, OCOR¹, R², COOR¹, NR³R⁴, NR⁵COR¹, NR⁵SO₂R¹, NR⁵CONR³R⁴, or a halogen;
Z is attached to a carbon at an arbitrary position of the fused ring, and each Z is independently a C₁₋₆ alkyl;
n is an integer of 0 to 10;
R¹ is an optionally substituted alkyl, an optionally substituted cycloalkyl, an optionally substituted aryl, an optionally substituted heteroaryl, or an optionally substituted aliphatic heterocyclic group;
R² is an optionally substituted alkyl, an optionally substituted alkenyl, an optionally substituted alkynyl, an optionally substituted cycloalkyl, an optionally substituted aryl, an optionally substituted heteroaryl, or an optionally substituted aliphatic heterocyclic group; and
R³, R⁴, and R⁵ are each independently a hydrogen atom, an optionally substituted alkyl, an optionally substituted cycloalkyl, an optionally substituted aliphatic heterocyclic group, an optionally substituted aryl, or an optionally substituted heteroaryl, wherein R³ and R⁴ can be combined with the adjacent nitrogen atom to form a 3- to 8-membered nitrogen-containing aliphatic heterocyclic ring optionally containing an oxygen atom, a nitrogen atom, or a sulfur atom, additionally constituting one or two rings, and the nitrogen-containing aliphatic heterocyclic ring is optionally substituted.
   n may be an integer of 0 to 10, and is an integer preferably of 0 to 6, more preferably of 0 to 3.

In the formula (2), 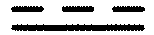
is a single bond or a double bond;
X and Y are as defined in the formula (1); and
Z¹, Z², Z³, Z⁴, Z⁵, and Z⁶ are each independently a hydrogen atom or a C₁₋₆ alkyl. It is preferable that, in the formula (2), Z¹, Z², Z³, Z⁴, Z⁵, and Z⁶ be each independently a hydrogen atom or methyl.

In the formula (3-1), formula (3-2), and formula (3-3),
X and Y are as defined in the formula (1); and
Z¹, Z², Z³, Z⁴, Z⁵, and Z⁶ are each independently a hydrogen atom or a C₁₋₆ alkyl. Here, it is preferable that Z¹, Z², Z³, Z⁴, Z⁵, and Z⁶ be each independently a hydrogen atom or methyl.

In the formula (4-1), formula (4-2), and formula (4-3),

X and Y are as defined in the formula (1).

The number of substituents in a group defined as being "optionally substituted" or "substituted" is not limited as long as the substitution is acceptable. Unless otherwise specified, description of each group is also applied to the case that the group is a part or substituent of another group.

Examples of "halogen" include fluorine, chlorine, bromine, and iodine. "Halogen" is preferably fluorine or chlorine. "Halogen" is more preferably fluorine.

The term "alkyl" means a linear or branched saturated hydrocarbon group, and examples thereof include alkyls having 1 to 25 carbon atoms, and preferable examples thereof include alkyls having 1 to 10 carbon atoms. Examples of more preferable alkyl include "C₁₋₈ alkyl", "C₁₋₆ alkyl", and "C₁₋₄ alkyl".

The term "C₁₋₁₀ alkyl" means a linear or branched saturated hydrocarbon group having 1 to 10 carbon atoms. The same is applied to cases with other numeric characters.

Preferable examples of "C₁₋₀ alkyl" include "C₁₋₈ alkyl", more preferable examples thereof include "C₁₋₆ alkyl", and even more preferable example thereof include "C₁₋₄ alkyl".

Preferable examples of "C₁₋₈ alkyl" include "C₁₋₆ alkyl", and more preferable examples thereof include "C₁₋₄ alkyl".

Preferable examples of "C₁₋₆ alkyl" include "C₁₋₄ alkyl". Specific examples of "C₁₋₄ alkyl" include methyl, ethyl, propyl, 1-methylethyl, butyl, 1,1-dimethylethyl, 1-methylpropyl, and 2-methylpropyl. Specific examples of "C₁₋₆ alkyl" include the specific examples shown above for "C₁₋₄ alkyl", in addition, 4-methylpentyl, 3-methylpentyl, 2-methylpentyl, 1-methylpentyl, and hexyl. Specific examples of "C₁₋₈ alkyl" include the specific examples shown above for "C₁₋₆ alkyl", in addition, heptyl and octyl.

The term "alkenyl" means a linear or branched unsaturated hydrocarbon group having one or more, preferably one to three double bonds, and examples thereof include alkenyls having two to seven carbon atoms. Examples of alkenyl in the present specification include vinyl, allyl, and butadienyl.

The term "alkynyl" means a linear or branched unsaturated hydrocarbon group having one or more, preferably one or two triple bonds, and examples thereof include alkynyls having two to five carbon atoms. Examples of alkynyl in the present specification include ethynyl and propynyl.

The term "cycloalkyl" means saturated cyclic alkyl, and those having partially crosslinked structure are also included. Examples of cycloalkyl include "C₃₋₈ cycloalkyl" and "C₃₋₆ cycloalkyl".

The term "C₃₋₈ cycloalkyl" means cycloalkyl in which the number of carbon atoms constituting the ring is three to eight. Preferable examples of "C₃₋₈ cycloalkyl" include "C₃₋₆ cycloalkyl". Specific examples of "C₃₋₆ cycloalkyl" include cyclopropyl, cyclobutyl, cyclopentyl, and cyclohexyl. Specific examples of "C₃₋₈ cycloalkyl" include the specific examples shown above for "C₃₋₆ cycloalkyl", in addition, cycloheptyl and cyclooctyl.

The term "aryl" means monocyclic or bicyclic aromatic hydrocarbon, and examples thereof include "C₆₋₁₀ aryl".

The term "C₆₋₁₀ aryl" means aryl having 6 to 10 carbon atoms. Specific examples of "C₆₋₁₀ aryl" include phenyl, 1-naphthyl, and 2-naphthyl. Preferable examples of "C₆₋₁₀ aryl" include phenyl.

The term "heteroaryl" means a monocyclic or multicyclic aromatic heterocyclic group containing one to four endocyclic heteroatoms each independently selected from the group consisting of one to four nitrogen atoms, one oxygen atom, and one sulfur atom.

If substituted, heteroaryl may be substituted at an arbitrary carbon atom or nitrogen atom, as long as the resultant is chemically stable.

The term "5- to 10-membered heteroaryl" means monocyclic or bicyclic heteroaryl composed of 5 to 10 atoms. Preferable examples of "5- to 10-membered heteroaryl" include "5- or 6-membered heteroaryl", and specific examples thereof include furyl, thienyl, pyrrolyl, imidazolyl, triazolyl, tetrazolyl, thiazolyl, pyridyl, pyrimidinyl, pyrazinyl, pyridazinyl, isothiazolyl, thiadiazolyl, pyrazolyl, oxazolyl, isoxazolyl, triazinyl, triazolyl, imidazolidinyl, and oxadiazolyl. Specific examples of "5- to 10-membered heteroaryl" include the specific examples shown above for "5- or 6-membered heteroaryl", in addition, indolyl, indazolyl, quinolyl, isoquinolyl, benzofuranyl, benzothienyl, benzooxazolyl, benzothiazolyl, benzisoxazolyl, benzisothiazolyl, benzotriazolyl, benzimidazolyl, and 6,11-dihydrodibenzo[b,e]thiepinyl.

The term "aliphatic heterocyclic group" means a monocyclic or bicyclic, saturated or unsaturated aliphatic heterocyclic group containing, in addition to carbon atoms, one to four heteroatoms each independently selected from the group consisting of one or two nitrogen atoms, one or two oxygen atoms, and one or two sulfur atoms.

If substituted, the aliphatic heterocyclic group may be substituted at an arbitrary carbon atom or nitrogen atom, as long as the resultant is chemically stable.

The term "4- to 10-membered aliphatic heterocyclic group" means an aliphatic heterocyclic group composed of 4 to 10 atoms, and those having partially crosslinked structure, those partially having spiro-structure, and those forming a fused ring with C₆₋₁₀ aryl or C₅₋₁₀ heteroaryl are also included. Preferable examples of "4- to 10-membered aliphatic heterocyclic group" include a 4- to 6-membered monocyclic saturated heterocyclic group. Specific examples of "4- to 6-membered monocyclic saturated heterocyclic group" include azetidinyl, pyrrolidinyl, piperidinyl, piperazinyl, morpholinyl, tetrahydrofuryl, and tetrahydropyranyl. Specific examples of "4- to 10-membered saturated heterocyclic group" include the specific examples shown above for "4- to 6-membered monocyclic saturated heterocyclic group", in addition, azepanyl, oxepanyl, diazepanyl, and homopiperidinyl.

Examples of "4- to 10-membered nitrogen-containing aliphatic heterocyclic ring" include a 4- to 10-membered nitrogen-containing aliphatic heterocyclic ring optionally containing an oxygen atom, a nitrogen atom, or a sulfur atom, additionally constituting one or two rings. The nitrogen-containing aliphatic heterocyclic ring may be monocyclic or bicyclic, and may be a saturated or unsaturated nitrogen-containing aliphatic heterocyclic ring. Preferable examples of "4- to 10-membered nitrogen-containing aliphatic heterocyclic ring" include a 4- to 6-membered monocyclic saturated nitrogen-containing heterocyclic ring. Specific examples of "4- to 6-membered monocyclic saturated nitrogen-containing heterocyclic ring" include azetidine, pyrrolidine, piperidine, piperazine, and morpholine. Specific examples of "4- to 10-membered saturated heterocyclic ring" include the specific examples shown above for "4- to 6-membered monocyclic saturated heterocyclic ring", in addition, azepane, oxepanyl, diazepane, and homopiperidine.

The term "alkoxy" means an oxy group substituted with alkyl, and preferable examples thereof include "C₁₋₈ alkoxy", "C₁₋₆ alkoxy", and "C₁₋₄ alkoxy".

The term "C₁₋₈ alkoxy" means an oxy group substituted with "C₁₋₈ alkyl" shown above. Preferable examples of "C₁₋₈ alkoxy" include "C₁₋₆ alkoxy" and "C₁₋₄ alkoxy". Specific examples of "C₁₋₄ alkoxy" include methoxy, ethoxy, propoxy, 1-methylethoxy, butoxy, 1,1-dimethylethoxy, 1-methylpropoxy, and 2-methylpropoxy. Specific examples of "C₁₋₆ alkoxy" include the specific examples shown above for "C₁₋₄ alkoxy", in addition, pentyloxy, 3-methylbutoxy, 2-methylbutoxy, 2,2-dimethylpropoxy, 1-ethylpropoxy, 1,1-dimethylpropoxy, hexyloxy, 4-methylpentyloxy, 3-methylpentyloxy, 2-methylpentyloxy, 1-methylpentyloxy, 3,3-dimethylbutoxy, 2,2-dimethylbutoxy, 1,1-dimethylbutoxy, and 1,2-dimethylbutoxy. Specific examples of "C₁₋₈ alkoxy" include the specific examples shown above for "C₁₋₆ alkoxy", in addition, heptyloxy and octyloxy.

The term "C₁₋₈ alkylcarbonyl" means a carbonyl group substituted with "C₁₋₈ alkyl" shown above. Preferable examples of "C₁₋₈ alkylcarbonyl" include "C₁₋₆ alkylcarbonyl" and "C₁₋₄ alkylcarbonyl". Specific examples of "C₁₋₄ alkylcarbonyl" include methylcarbonyl, ethylcarbonyl, propylcarbonyl, 1-methylethylcarbonyl, butylcarbonyl, 1,1-dimethylethylcarbonyl, 1-methylpropylcarbonyl, and 2-methylpropylcarbonyl. Specific examples of "C₁₋₆ alkylcarbonyl" include the specific examples shown above for "C₁₋₄ alkylcarbonyl", in addition, 4-methylpentylcarbonyl, 3-methylpentylcarbonyl, 2-methylpentylcarbonyl, 1-methylpentylcarbonyl, and hexylcarbonyl. Specific examples of "C₁₋₈ alkylcarbonyl" include the specific examples shown above for "C₁₋₆ alkylcarbonyl", in addition, heptylcarbonyl and octylcarbonyl.

The term "C₁₋₈ alkylcarbonyloxy" means an oxy group substituted with "C₁₋₈ alkylcarbonyl" shown above. Preferable examples of "C₁₋₈ alkylcarbonyloxy" include "C₁₋₆ alkylcarbonyloxy" and "C₁₋₄ alkylcarbonyloxy". Specific examples of "C₁₋₄ alkylcarbonyloxy" include acetoxy, propanoyloxy, butanoyloxy, 2-methylpropanoyloxy, pentanoyloxy, 2,2-dimethylpropanoyloxy, 2-methylbutanoyloxy, and 3-methylbutanoyloxy. Specific examples of "C₁₋₆ alkylcarbonyloxy" include the specific examples shown above for "C₁₋₄ alkylcarbonyloxy", in addition, hexanoyloxy, 3,3-dimethylbutanoyloxy, 3-methylpentanoyloxy, 4-methylpentanoyloxy, 2,2-dimethylbutanoyloxy, 2,3-dimethylbutanoyloxy, 2-ethylbutanoyloxy, 3-ethylbutanoyloxy, heptanoyloxy, 2-methylhexanoyloxy, 3-methylhexanoyloxy, 4-methylhexanoyloxy, 5-methylhexanoyloxy, 2,2-dimethylpentanoyloxy, 3,3-dimethylpentanoyloxy, 4,4-dimethylpentanoyloxy, 2,3-dimethylpentanoyloxy, 2,4-dimethylpentanoyloxy, 2-ethylpentanoyloxy, 3-ethylpentanoyloxy, and 4-ethylpentanoyloxy. Specific examples of "C₁₋₈ alkylcarbonyloxy" include the specific examples shown above for "C₁₋₆ alkylcarbonyloxy", in addition, heptylcarbonyloxy and octylcarbonyloxy.

The term "C₁₋₈ alkoxycarbonyl" means a carbonyl group substituted with "C₁₋₈ alkoxy" shown above. Preferable examples of "C₁₋₈ alkoxycarbonyl" include "C₁₋₆ alkoxycarbonyl" and "C₁₋₄ alkoxycarbonyl". Specific examples of "C₁₋₄ alkoxycarbonyl" include methoxycarbonyl, ethoxycarbonyl, propoxycarbonyl, 1-methylethoxycarbonyl, butoxycarbonyl, 1,1-dimethylethoxycarbonyl, 1-methylpropoxycarbonyl, and 2-methylpropoxycarbonyl. Specific examples of "C₁₋₆ alkoxycarbonyl" include the specific examples shown above for "C₁₋₄ alkoxycarbonyl", in addition, pentyloxycarbonyl, 3-methylbutoxycarbonyl, 2-methylbutoxycarbonyl, 2,2-dimethylpropoxycarbonyl, 1-ethylpropoxycarbonyl, 1,1-dimethylpropoxycarbonyl, hexyloxycarbonyl, 4-methylpentyloxycarbonyl, 3-methylpentyloxycarbonyl, 2-methylpentyloxycarbonyl, 1-methylpentyloxycarbonyl, 3,3-dimethylbutoxycarbonyl, 2,2-dimethylbutoxycarbonyl, 1,1-dimethylbutoxycarbonyl, and 1,2-dimethylbutoxycarbonyl. Specific examples of "C₁₋₈ alkoxycarbonyl" include the specific examples shown above for "C₁₋₆ alkoxycarbonyl", in addition, heptyloxycarbonyl and octyloxycarbonyl.

The term "C₁₋₈ alkylsulfanyl" means a sulfanyl group substituted with "C₁₋₈ alkyl" shown above. Preferable examples of "C₁₋₈ alkylsulfanyl" include "C₁₋₆ alkylsulfanyl" and "C₁₋₄ alkylsulfanyl". Specific examples of "C₁₋₄ alkylsulfanyl" include methylsulfanyl, ethylsulfanyl, propylsulfanyl, 1-methylethylsulfanyl, butylsulfanyl, 1,1-dimethylethylsulfanyl, 1-methylpropylsulfanyl, and 2-methylpropylsulfanyl. Specific examples of "C₁₋₆ alkylsulfanyl" include the specific examples shown above for "C₁₋₄ alkylsulfanyl", in addition, pentylsulfanyl, 3-methylbutylsulfanyl, 2-methylbutylsulfanyl, 2,2-dimethylpropylsulfanyl, 1-ethylpropylsulfanyl, 1,1-dimethylpropylsulfanyl, hexilsulfanyl, 4-methylpentylsulfanyl, 3-methylpentylsulfanyl, 2-methylpentylsulfanyl, 1-methylpentylsulfanyl, 3,3-dimethylbutylsulfanyl, 2,2-dimethylbutylsulfanyl, 1,1-dimethylbutylsulfanyl, and 1,2-dimethylbutylsulfanyl. Specific examples of "C₁₋₈ alkylsulfanyl" include the specific examples shown above for "C₁₋₆ alkylsulfanyl", in addition, heptylsulfanyl and octylsulfanyl.

The term "C₁₋₈ alkylsulfonyl" means a sulfonyl group substituted with "C₁₋₈ alkyl" shown above. Preferable examples of "C₁₋₈ alkylsulfonyl" include "C₁₋₆ alkylsulfonyl" and "C₁₋₄ alkylsulfonyl". Specific examples of "C₁₋₄ alkylsulfonyl" include methylsulfonyl, ethylsulfonyl, propylsulfonyl, 1-methylethylsulfonyl, butylsulfonyl, 1,1-dimethylethylsulfonyl, 1-methylpropylsulfonyl, and 2-methylpropylsulfonyl. Specific examples of "C₁₋₆ alkylsulfonyl" include the specific examples shown above for "C₁₋₄ alkylsulfonyl", in addition, pentylsulfonyl, 3-methylbutylsulfonyl, 2-methylbutylsulfonyl, 2,2-dimethylpropylsulfonyl, 1-ethylpropylsulfonyl, 1,1-dimethylpropylsulfonyl, hexilsulfonyl, 4-methylpentylsulfonyl, 3-methylpentylsulfonyl, 2-methylpentylsulfonyl, 1-methylpentylsulfonyl, 3,3-dimethylbutylsulfonyl, 2,2-dimethylbutylsulfonyl, 1,1-dimethylbutylsulfonyl, and 1,2-dimethylbutylsulfonyl. Specific examples of "C₁₋₈ alkylsulfonyl" include the specific examples shown above for "C₁₋₆ alkylsulfonyl", in addition, heptylsulfonyl and octylsulfonyl.

The term "C₁₋₈ alkylsulfonylamino" means an amino group substituted with "C₁₋₈ alkylsulfonyl" shown above. Preferable examples of "C₁₋₈ alkylsulfonylamino" include "C₁₋₆ alkylsulfonylamino" and "C₁₋₄ alkylsulfonylamino". Specific examples of "C₁₋₄ alkylsulfonylamino" include methylsulfonylamino, ethylsulfonylamino, propylsulfonylamino, 1-methylethylsulfonylamino, butylsulfonylamino, 1,1-dimethylethylsulfonylamino, 1-methylpropylsulfonylamino, and 2-methylpropylsulfonylamino. Specific examples of "C₁₋₆ alkylsulfonylamino" include the specific examples shown above for "C₁₋₄ alkylsulfonylamino", in addition, pentylsulfonylamino, 3-methylbutylsulfonylamino, 2-methylbutylsulfonylamino, 2,2-dimethylpropylsulfonylamino, 1-ethylpropylsulfonylamino, 1,1-dimethylpropylsulfonylamino, hexilsulfonylamino, 4-methylpentylsulfonylamino, 3-methylpentylsulfonylamino, 2-methylpentylsulfonylamino, 1-methylpentylsulfonylamino, 3,3-dimethylbutylsulfonylamino, 2,2-dimethylbutylsulfonyl, 1,1-dimethylbutylsulfonyl, and 1,2-dimethylbutylsulfonylamino. Specific examples of "C₁₋₈ alkylsulfonylamino" include the specific examples shown above for "C₁₋₆ alkylsulfonylamino", in addition, heptylsulfonylamino and octylsulfonylamino.

The term "C₁₋₈ alkylcarbonylamino" means an amino group substituted with "C₁₋₈ alkylcarbonyl" shown above. Preferable examples of "c₁₋₈ alkylcarbonylamino" include "C₁₋₆ alkylcarbonylamino" and "C₁₋₄ alkylcarbonylamino". Specific examples of "C₁₋₄ alkylcarbonylamino" include acetylamino, propanoylamino, butanoylamino, 2-methylpropanoylamino, pentanoylamino, 2,2-dimethylpropanoylamino, 2-methylbutanoylamino, and 3-methylbutanoylamino. Specific examples of "C₁₋₆ alkylcarbonylamino" include the specific examples shown above for "C₁₋₄ alkylcarbonylamino", in addition, hexanoylamino, 3,3-dimethylbutanoylamino, 3-methylpentanoylamino, 4-methylpentanoylamino, 2,2-dimethylbutanoylamino, 2,3-dimethylbutanoylamino, 2-ethylbutanoylamino, 3-ethylbutanoylamino, heptanoylamino, 2-methylhexanoylamino, 3-methylhexanoylamino, 4-methylhexanoylamino, 5-methylhexanoylamino, 2,2-dimethylpentanoylamino, 3,3-dimethylpentanoylamino, 4,4-dimethylpentanoylamino, 2,3-dimethylpentanoylamino, 2,4-dimethylpentanoylamino, 2-ethylpentanoylamino, 3-ethylpentanoylamino, and 4-ethylpentanoylamino. Specific examples of "C₁₋₈ alkylcarbonylamino" include the specific examples shown above for "C₁₋₆ alkylcarbonylamino", in addition, heptylcarbonylamino and octylcarbonylamino.

Examples of amino optionally substituted with one or two C₁₋₈ alkyls in an embodiment include amino and amino substituted with one or two identical or different groups being "C₁₋₆ alkyl" or "C₁₋₄ alkyl" shown above. Specific examples thereof include amino, methylamino, ethylamino, propylamino, 2-propylamino, butylamino, dimethylamino, diethylamino, methylethylamino, and dipropylamino.

In an embodiment of amino optionally substituted with one or two C₁₋₈ alkyls, two substituents of the amino group can be combined with the adjacent nitrogen atom to form a 4- to 10-membered nitrogen-containing aliphatic heterocyclic ring. Preferable examples of the nitrogen-containing aliphatic heterocyclic ring include piperidine, pyrrolidine, piperazine, and morpholine.

Examples of carbamoyl optionally substituted with one or two C₁₋₈ alkyls in an embodiment include carbamoyl and carbamoyl substituted with one or two identical or different groups being "C₁₋₆ alkyl" or "C₁₋₄ alkyl" shown above. Specific examples thereof include carbamoyl, methylcarbamoyl, ethylcarbamoyl, propylcarbamoyl, 2-propylcarbamoyl, butylcarbamoyl, dimethylcarbamoyl, diethylcarbamoyl, methylethylcarbamoyl, and dipropylcarbamoyl.

In an embodiment of carbamoyl optionally substituted with one or two C₁₋₈ alkyls, two substituents of the carbamoyl group can be combined with the adjacent nitrogen atom to form a 4- to 10-membered nitrogen-containing aliphatic heterocyclic ring. Preferable examples of the nitrogen-containing aliphatic heterocyclic ring include piperidine, pyrrolidine, piperazine, and morpholine.

It is preferable that Y in the formula (1), formula (2), formula (3-1), formula (3-2), formula (3-3), formula (4-1), formula (4-2), or formula (4-3) be a hydrogen atom, hydroxy, oxo, carboxy, carbamoyl, OR¹, SR¹, SO₂R¹, COR¹, OCOR¹, R², NR³R⁴, NR⁵COR¹ , NR^{S}SO₂R¹, or a fluorine atom.

In an embodiment of the present invention, in the formula (1) or formula (2), the bond represented by: 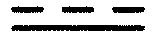 indicates a single bond or a double bond. Here, the bond is a single bond if Y is an oxo group.

In an embodiment of the present invention, in the formula (1) or formula (2),
if 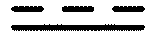
is a double bond, then Y is a hydrogen atom or hydroxy, and
if 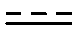
is a single bond, then
Y is hydroxy, oxo, carboxy, carbamoyl, OR¹, SR¹, SO₂R¹, OCOR¹, R², NR³R⁴, NR⁵COR¹ , NR⁵SO₂R¹, or a fluorine atom;
R¹ is a C₁₋₈ alkyl optionally substituted with one to seven substituents selected from Group 1, a 3- to 8-membered cycloalkyl optionally substituted with one to three groups selected from Group 1, phenyl optionally substituted with one to five groups selected from Group 1, a 4-to 10-membered aliphatic heterocyclic group containing one to four endocyclic heteroatoms selected from an oxygen atom, a nitrogen atom, and a sulfur atom and optionally substituted with one to three groups selected from Group 1, or a 5- to 10-membered heteroaryl containing one to three endocyclic heteroatoms selected from an oxygen atom, a nitrogen atom, and a sulfur atom and optionally substituted with one to four groups selected from Group 1;
R² is a C₁₋₈ alkyl optionally substituted with one to seven substituents selected from Group 2, a C₁₋₈ alkenyl optionally substituted with one to seven substituents selected from Group 2, a 3- to 8-membered cycloalkyl optionally substituted with one to three groups selected from Group 1, phenyl optionally substituted with one to five groups selected from Group 1, a 4- to 10-membered aliphatic heterocyclic group containing one to four endocyclic heteroatoms selected from an oxygen atom, a nitrogen atom, and a sulfur atom and optionally substituted with one to three groups selected from Group 1, or a 5- to 10-membered heteroaryl containing one to three endocyclic heteroatoms selected from an oxygen atom, a nitrogen atom, and a sulfur atom and optionally substituted with one to four groups selected from Group 1;
R³ and R⁴ are each independently a hydrogen atom, a C₁₋₆ alkyl optionally substituted with one to seven substituents selected from Group 1, or phenyl optionally substituted with one to five groups selected from Group 1, wherein R³ and R⁴ can be combined with the adjacent nitrogen atom to form a 3- to 8-membered nitrogen-containing aliphatic heterocyclic ring optionally containing an oxygen atom, a nitrogen atom, or a sulfur atom, additionally constituting one or two rings, and the nitrogen-containing aliphatic heterocyclic ring is optionally substituted with one to three substituents selected from substituents of Group 1 shown below; and it is preferable that R⁵ be a hydrogen atom or a C₁₋₃ alkyl.

In an embodiment of the present invention, it is preferable that X be O-O.

In the present specification, if substituted, the alkyl in R¹, R³, R⁴, and R⁵ is optionally substituted with one to seven, preferably one to three substituents selected from Group 1 shown below.

In the present specification, if substituted, the alkyl, alkenyl, or alkynyl in R² is optionally substituted with one to seven, preferably one to three substituents selected from Group 2 shown below.

### [Group 1]

Carboxy, hydroxy, a C₁₋₈ alkoxy, a C₁₋₈ alkylcarbonyl, a C₁₋₈ alkylcarbonyloxy, a C₁₋₈ alkoxycarbonyl, mercapto, a C₁₋₈ alkylsulfanyl, a C₁₋₈ alkylsulfonyl, amino optionally substituted with one or two C₁₋₈ alkyls, carbamoyl optionally substituted with one or two C₁₋₈ alkyls, a C₁₋₈ alkylcarbonylamino, a C₁₋₈ alkylsulfonylamino, phenyl optionally substituted with one to five groups selected from Group 3, a 5- or 6-membered heteroaryl containing one to four endocyclic heteroatoms selected from an oxygen atom, a nitrogen atom, and a sulfur atom and optionally substituted with one to three groups selected from Group 3, a 3- to 8-membered cycloalkyl optionally substituted with one to three groups selected from Group 3, a 4- to 10-membered aliphatic heterocyclic group containing one to four endocyclic heteroatoms selected from an oxygen atom, a nitrogen atom, and a sulfur atom and optionally substituted with one to three groups selected from Group 3, and a halogen;

### [Group 2]

carboxy, hydroxy, a C₁₋₈ alkoxy, a C₁₋₈ alkylcarbonyl, a C₁₋₈ alkylcarbonyloxy, a C₁₋₈ alkoxycarbonyl, mercapto, a C₁₋₈ alkylsulfanyl, a C₁₋₈ alkylsulfonyl, carbamoyl optionally substituted with one or two C₁₋₈ alkyls, amino, phenyl optionally substituted with one to five groups selected from Group 3, a 5- or 6-membered heteroaryl containing one to three endocyclic heteroatoms selected from an oxygen atom, a nitrogen atom, and a sulfur atom and optionally substituted with one to four groups selected from Group 3, a 3- to 8-membered cycloalkyl optionally substituted with one to three groups selected from Group 3, a 4- to 8-membered aliphatic heterocyclic group containing one to four endocyclic heteroatoms selected from an oxygen atom, a nitrogen atom, and a sulfur atom and optionally substituted with one to three groups selected from Group 3, and a halogen; and

### [Group 3]

carboxy, hydroxy, a C₁₋₈ alkyl, a C₁₋₈ alkoxy, a C₁₋₈ alkylcarbonyl, a C₁₋₈ alkylcarbonyloxy, a C₁₋₈ alkoxycarbonyl, mercapto, a C₁₋₈ alkylsulfanyl, a C₁₋₈ alkylsulfonyl, amino optionally substituted with one or two C₁₋₈ alkyls, carbamoyl optionally substituted with one or two C₁₋₈ alkyls, a C₁₋₈ alkylcarbonylamino, a C₁₋₈ alkylsulfonylamino, and a halogen.

If substituted, the cycloalkyl is optionally substituted with one to three, preferably one or two substituents selected from Group 1 shown above. Preferable examples of the substituents of the cycloalkyl group include hydroxy, a C₁₋₈ alkoxy, and a halogen.

If substituted, the aliphatic heterocyclic group is optionally substituted with one to three, preferably one or two substituents selected from Group 1 shown above. Preferable examples of the substituents of the aliphatic heterocyclic group include hydroxy, a C₁₋₈ alkoxy, and a halogen.

If substituted, the aryl is optionally substituted with one to five, preferably one to three substituents selected from Group 1 shown above. Preferable examples of the substituents of the aryl group include hydroxy, a C₁₋₈ alkoxy, and a halogen.

If substituted, the heteroaryl is optionally substituted with one to four, preferably one to three substituents selected from Group 1 shown above. Preferable examples of the substituents of the heteroaryl group include hydroxy, a C₁₋₈ alkoxy, and a halogen.

Group 1, Group 2, and Group 3 are preferably Group 1', Group 2', and Group 3', respectively, in the following:

### [Group 1']

a fluorine atom, hydroxy, carboxy, amino, a C₁₋₄ alkoxy, a C₂₋₄ alkylcarbonyloxy, phenyl optionally substituted with one to three groups selected from Group 3', a 3- to 6-membered cycloalkyl optionally substituted with one to three groups selected from Group 3', and a 4- to 6-membered aliphatic heterocyclic group containing one to three endocyclic heteroatoms selected from an oxygen atom, a nitrogen atom, and a sulfur atom and optionally substituted with one to three groups selected from Group 3';

### [Group 2']

a fluorine atom, hydroxy, carboxy, amino, a C₁₋₄ alkoxy, a C₂₋₄ alkylcarbonyloxy, a C₁₋₄ alkoxycarbonyl, carbamoyl optionally substituted with one or two C₁₋₈ alkyls, phenyl optionally substituted with one to three groups selected from Group 3', a 3- to 6-memberedcycloalkyl optionally substituted with one to three groups selected from Group 3', and a 4- to 6-membered aliphatic heterocyclic group containing one to three endocyclic heteroatoms selected from an oxygen atom, a nitrogen atom, and a sulfur atom and optionally substituted with one to three groups selected from Group 3'; and

### [Group 3']

a fluorine atom, hydroxy, and a C₁₋₄ alkyl.

If substituted, the alkenyl or alkynyl is optionally substituted with one to three, preferably one or two substituents selected from Group 3" in the following.

### [Group 3"]

A fluorine atom.

Preferred as X, Y, Z, Z¹, Z², Z³, Z⁴, Z⁵, Z⁶, R¹, R², R³, R⁴, and R⁵ are as follows; however, the technical scope of the present invention is not limited to the scope of the compound shown in the following.

Preferable examples of Z in the formula (1) include a C₁₋₄ alkyl, more preferable examples thereof include a C₁₋₃ alkyl, and even more preferable examples thereof include methyl and ethyl.

In the formula (1), each group Z may be attached to an arbitrary carbon atom constituting the fused ring, the groups Z may be attached to the same carbon atom or be each attached to a carbon atom belonging to a plurality of rings, as long as the resultant is chemically stable, and the groups Z may be the same or different.

The configuration of the carbon atoms constituting the ring (fused ring) to which groups Z are attached in the formula (1) is not limited as long as the configuration allows the ring to form chemically stable structure, and, for example, specific examples of a group represented by formula (2-1): 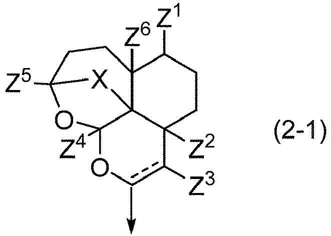 wherein X, Z¹, Z², Z³, Z⁴, Z⁵, and Z⁶ are as defined in the formula (1) or formula (2), include groups represented by the following formula (3-1'), formula (3-2'), and formula (3-3'): 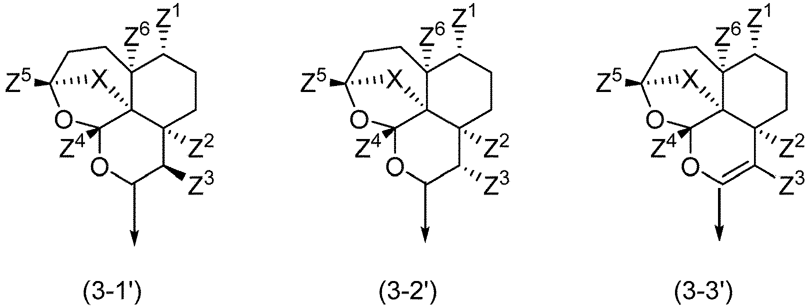 wherein X, Z¹, Z², Z³, Z⁴, Z⁵, and Z⁶ are as defined in the formula (1) or formula (2).

Preferable examples of each of Z¹, Z², Z³, Z⁴, Z⁵, and Z⁶ in the formula (2) include a hydrogen atom and a C₁₋₄ alkyl, more preferable examples thereof include a hydrogen atom and a C₁₋₃ alkyl, and even more preferable examples thereof include a hydrogen atom, methyl, and ethyl.

Examples of the compound of the formula (1), formula (2), formula (3-1), formula (3-2), formula (3-3), formula (4-1), formula (4-2), or formula (4-3) in an embodiment include a compound in which X is O-O.

Examples of the compound of the formula (1), formula (2), formula (3-1), formula (3-2), formula (3-3), formula (4-1), formula (4-2), or formula (4-3) in the case that Y is oxo in an embodiment include a compound represented by formula (2') shown below. While there exists a tautomer represented by formula (2") for the compound of the formula (2'), the present invention includes both the substances as long as they exist in a chemically stable manner: 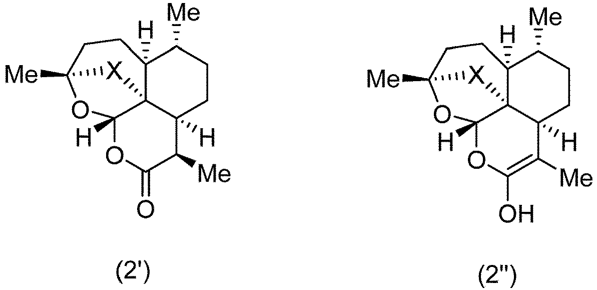 wherein X is as defined in the formula (1).

Preferable examples of the compound of the formula (1), formula (2), formula (3-1), formula (3-2), formula (3-3), formula (4-1), formula (4-2), or formula (4-3) in the case that X is O in an embodiment include the compound represented by the above formula (2') or formula (2").

Embodiments of Y in the formula (1), formula (2), formula (3-1), formula (3-2), formula (3-3), formula (4-1), formula (4-2), or formula (4-3) will be described in the following.

Examples of a preferred embodiment of Y include a hydrogen atom, hydroxy, carboxy, carbamoyl, and a halogen.

If Y is a halogen, Y is preferably a fluorine atom.

Preferable examples of R¹ in the case that Y is OR¹, SR¹, SOR¹, SO₂R¹, or COR¹ include an optionally substituted C₁₋₄ alkyl, optionally substituted phenyl, and an optionally substituted cycloalkyl.

Here, preferable examples of R¹ in an embodiment include an optionally substituted C₁₋₃ alkyl, and more preferable examples thereof include methyl and ethyl, each optionally substituted. Examples of the substituents include an aryl optionally substituted with one or more substituents selected from Group 2 mentioned above such as a halogen, carboxy, amino, a C₁₋₄ alkoxy, and a C₁₋₄ alkylcarbonyloxy, and the substituents may each independently substitute at one or more, or one to three positions. Preferable examples of the halogen include a fluorine atom.

Examples of R¹ in the case that Y is OCOR¹ include an optionally substituted C₁₋₄ alkyl. Examples of the substituents include a halogen, carboxy, amino optionally substituted with one or two identical or different C₁₋₃ alkyls, a C₁₋₄ alkoxy, a C₁₋₄ alkylcarbonyloxy, and an aryl optionally substituted with one or more substituents selected from Group 3 mentioned above, and the substituents may each independently substitute at one or more, or one to three positions. Preferable examples of the halogen include a fluorine atom.

Examples of R¹ in the case that Y is OCOR¹ in an embodiment include a C₁₋₄ alkyl substituted with carboxy.

If Y is NR³R⁴, R³ and R⁴ are preferably each independently a hydrogen atom or a C₁₋₄ alkyl, or R³ and R⁴ can be combined with the adjacent nitrogen atom to form a 4- to 10-membered nitrogen-containing aliphatic heterocyclic ring. Preferable examples of the 4- to 10-membered nitrogen-containing aliphatic heterocyclic ring include a 4- to 6-membered monocyclic saturated nitrogen-containing heterocyclic ring, and specific examples thereof include azetidine, pyrrolidine, piperidine, piperazine, and morpholine.

Examples of R¹ in the case that Y is NR⁵COR¹ or NR⁵SO₂R¹ include an optionally substituted C₁₋₄ alkyl. Examples of the substituents include a halogen, carboxy, amino, a C₁₋₄ alkoxy, a C₁₋₄ alkylcarbonyloxy, and an aryl optionally substituted with one or more substituents selected from Group 3 mentioned above, and the substituents may each independently substitute at one or more, or one to three positions. Preferable examples of the halogen include a fluorine atom.

R⁵ is preferably a hydrogen atom or C₁₋₄ alkyl, and more preferably a hydrogen atom, methyl, or ethyl.

For example, R³ and R⁴ in the case that Y is NR⁵CONR³R⁴ are preferably each independently a hydrogen atom, an optionally substituted C₁₋₄ alkyl, or phenyl optionally substituted with one or more substituents selected from Group 3. Examples of the substituents of the C₁₋₄ alkyl include a halogen, carboxy, amino, a C₁₋₄ alkoxy, a C₁₋₄ alkylcarbonyloxy, and an aryl optionally substituted with one or more substituents selected from Group 3 mentioned above, and the substituents may each independently substitute at one or more, or one to three positions. Preferable examples of the halogen include a fluorine atom. R³ and R⁴ can be combined with the adjacent nitrogen atom to form a 4- to 10-membered nitrogen-containing aliphatic heterocyclic ring. Preferable examples of the 4- to 10-membered nitrogen-containing aliphatic heterocyclic ring include a 4- to 6-membered monocyclic saturated nitrogen-containing heterocyclic ring, and specific examples thereof include azetidine, pyrrolidine, piperidine, piperazine, and morpholine. Preferable examples of the substituents of the above optionally substituted C₁₋₄ alkyl include an aryl, a heteroaryl, a cycloalkyl, and an aliphatic heterocyclic group, each optionally substituted with one or more substituents selected from Group 2 mentioned above, such as a halogen, carboxy, amino, a C₁₋₄ alkoxy, and a C₁₋₄ alkylcarbonyloxy, and the substituents may each independently substitute at one or more, or one to three positions. Preferable examples of the halogen include a fluorine atom.

R⁵ is preferably a hydrogen atom or C₁₋₄ alkyl, and more preferably a hydrogen atom, methyl, or ethyl.

Preferable examples of R² in the case that Y is R² include a C₁₋₄ alkyl optionally substituted with one or more substituents selected from Group 2 or Group 2' mentioned above, phenyl optionally substituted with one or more substituents selected from Group 3 or Group 3' mentioned above, or a 5- or 6-membered heteroaryl optionally substituted with one or more substituents selected from Group 3 in [6] or [7] mentioned above. Specific examples of the heteroaryl include furan-2-yl and triazin-1-yl, each optionally substituted.

Examples of R² in an embodiment in the case that Y is R² include a C₁₋₄ alkyl, and preferable examples thereof include methyl and ethyl.

Examples of R² in the case that Y is R² in an embodiment include a C₁₋₄ alkyl substituted with one or more, preferably one to three substituents selected from Group 4 in the following, and preferable examples thereof include a C₁₋₄ alkyl, methyl, and ethyl, each substituted with one to three substituents selected from Group 4 in the following.

### [Group 4]

Carboxy, hydroxy, amino, a C₁₋₄ alkoxy, a C₁₋₄ alkylcarbonyl, a C₁₋₄ alkylcarbonyloxy, a C₁₋₄ alkoxycarbonyl, carbamoyl optionally substituted with one or two C₁₋₄ alkyls, phenyl optionally substituted with one to five groups selected from Group 3, a 5- or 6-membered heteroaryl containing one to three endocyclic heteroatoms selected from an oxygen atom, a nitrogen atom, and a sulfur atom and optionally substituted with one to four groups selected from Group 3, a 3- to 6-membered cycloalkyl optionally substituted with one to three groups selected from Group 3, a 4- to 8-membered aliphatic heterocyclic group containing one to four endocyclic heteroatoms selected from an oxygen atom, a nitrogen atom, and a sulfur atom and optionally substituted with one to three groups selected from Group 3, and a fluorine atom.

Examples of the compound represented by the formula (2) in an embodiment include a compound having the following characteristics (A1) to (A5) or a salt thereof:
(A1) Z¹, Z³, and Z⁵ are each methyl, and Z², Z⁴, and Z⁶ are each a hydrogen atom;
(A2) X is O or O-O;
(A3) Y is hydroxy, oxo, carboxy, carbamoyl, a fluorine atom, OR¹, OCOR¹, SR¹, SO₂R¹, R², NHCOR¹, or NR³R⁴;
(A4) R¹ or R² is an optionally substituted C₁₋₈ alkyl, optionally substituted phenyl, an optionally substituted 5- or 6-membered cycloalkyl, or an optionally substituted 5- or 6-membered heteroaryl, wherein the substituents are selected from a fluorine atom, amino, carboxy, a C₁₋₈ alkoxy, a C₁₋₈ alkylcarbonyloxy, and phenyl; and
(A5) R³ and R⁴ each represent a methyl group, or are combined with the adjacent nitrogen atom to form morpholine, thiomorpholine, thiomorpholin-1-oxide, thiomorpholine-1,1-dioxide, piperazine, indoline, or tetrahydroisoquinoline.

Examples of the compound represented by the formula (1), formula (2), formula (3-1), formula (3-2), formula (3-3), formula (4-1), formula (4-2), or formula (4-3) in an embodiment include a compound having the following characteristics (B1) and (B2) or a salt thereof:
(B1) X is O-O; and
(B2) Y is NR⁵SO₂R¹.

Examples of the compound represented by the formula (1), formula (2), formula (3-1), formula (3-2), formula (3-3), formula (4-1), formula (4-2), or formula (4-3) in an embodiment include a compound having the following characteristics (B3) and (B4) in addition to (B1) and (B2) in the above or a salt thereof:
(B3) R¹ is an optionally substituted C₁₋₈ alkyl, optionally substituted phenyl, an optionally substituted 5- or 6-membered cycloalkyl, or an optionally substituted 5- or 6-membered heteroaryl, wherein the substituents are selected from a fluorine atom, amino, carboxy, a C₁₋₈ alkoxy, a C₁₋₈ alkylcarbonyloxy, and phenyl; and
(B4) R⁵ is a hydrogen atom.

Specific examples of the compound represented by the formula (1), formula (2), formula (3-1), formula (3-2), formula (3-3), formula (4-1), formula (4-2), or formula (4-3) are compounds of Examples 1-1 to 1-35.

The scope of the compound of the present invention includes a salt of the above-described compound represented by the formula (1). The salt is not limited unless the salt affects, for example, the survival or differentiation of cells in cell culture, and examples thereof include a salt acceptable as a pharmaceutical raw material.

Examples of "salt" include acid addition salts and base addition salts. Examples of acid addition salts include inorganic acid salts such as hydrochlorides, hydrobromides, sulfates, hydroiodides, nitrates, and phosphates, and organic acid salts such as citrates, oxalates, phthalates, fumarates, maleates, succinates, malates, acetates, formates, propionates, benzoates, trifluoroacetates, methanesulfonates, benzenesulfonates, para-toluenesulfonates, and camphorsulfonates. Examples of base addition salts include inorganic base salts such as sodium salts, potassium salts, calcium salts, magnesium salts, barium salts, and aluminum salts, and organic base salts with trimethylamine, triethylamine, pyridine, picoline, 2,6-lutidine, ethanolamine, diethanolamine, triethanolamine, tromethamine, [tris(hydroxymethyl)methylamine], tert-butylamine, cyclohexylamine, dicyclohexylamine, or N,N-dibenzylethylamine. Additional examples of "salt" include amino acid salts with a basic amino acid or acidic amino acid such as arginine, lysine, ornithine, aspartic acid, and glutamic acid.

In attempting to obtain a salt of the compound of the present invention, if the compound of the present invention in the form of a salt is generated, the salt can be directly purified; if the compound of the present invention is generated as a free form, a salt can be formed by a common method involving dissolving or suspending the free form in an appropriate organic solvent followed by adding an acid or base thereto.

Deuterated products obtained by converting any one or two or more ¹H atoms in the compound of the present invention into ²H (D) atoms are also included in the scope of the compound of the present invention.

The compound of the present invention may exist in the form of a hydrate and/or a solvate with a given solvent (e.g., a solvate with ethanol), and hence such hydrates and/or solvates are also included in the scope of the compound of the present invention. Further, all of the tautomers of the compound of the present invention, all of the existing stereoisomers thereof, and those in all modes of crystal form, and mixtures of them are also included in the present invention.

In the case that Y represents oxo or hydroxy in the formula (1), formula (2), formula (3-1), formula (3-2), formula (3-3), formula (4-1), formula (4-2), and formula (4-3), for example, when there exists a tautomer represented by the following formula, the scope of the compound in which Y represents hydroxy or compound in which Y represents oxo includes, in concept, the corresponding tautomers.

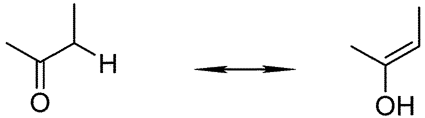

While there may exist optical isomers, which involves a chiral center, atropisomers, which involves axial or planar chirality caused by restriction of intramolecular rotation, and other stereoisomers, tautomers, and geometric isomers for the compound of the present invention in some cases, all the possible isomers and mixtures thereof, including those mentioned, are included in the scope of the present invention.

Specifically, if any of the compounds represented by the formula (1), formula (2), formula (3-1), formula (3-2), formula (3-3), formula (4-1), formula (4-2), and formula (4-3) has a chiral center the configuration of which is not explicitly shown, the scope of it can include single stereoisomers and mixtures of a plurality of stereoisomers.

For example, optical isomers of any of the compounds represented by the formula (3-1), formula (3-2), formula (3-3), formula (4-1), formula (4-2), and formula (4-3), and mixtures of the compound and an optical isomer thereof (e.g., a racemate) also fall within the scope of the present invention.

In particular, optical isomers and atropisomers can be each obtained as a racemate, or as an optically active substance if an optically active starting raw material or intermediate is used. If necessary, the corresponding raw material, intermediate, or final product being a racemate can be physically or chemically resolved into optical enantiomers thereof by using a known separation method such as a method using an optically active column and a fractional crystallization method in an appropriate stage of a production method shown below. Specifically, in a diastereomer method, for example, two diastereomers are formed from a racemate through a reaction with an optically active resolver. These different diastereomers can be resolved by using a known method such as fractional crystallization because they generally have different physical characteristics.

### Methods for Producing Compound

Methods for producing the compound of the present invention will be described in the following, but the way of producing the compound of the present invention is not limited thereto.

The compound represented by the formula (1) can be a known compound, or produced from a known compound by a chemical synthesis method well known to those skilled in the art.

Specific examples of such known compounds include compounds described in Examples in the present specification: artemisinin (Example 1-33), artemether (Example 1-30), artenimol (Example 1-29), artemotil (Example 1-31), artesunate (Example 1-32), and compounds shown in Examples 1-1 to 1-28, 1-34, and 1-35. Artemether, artenimol, artemotil, and artesunate are each a resultant of derivatization of artemisinin, and can be produced from artemisinin by semisynthesis. For example, artemether and artemotil can be produced according to a method described in Tetrahedron Letters 2002, 43, 7235-7237, artesunate can be produced according to a method described in J. Med. Chem. 1988, 31, 645-650, and artenimol can be produced according to a method described in Chem Commun 2014, 50, 12652-12655. Likewise, the other compounds are known to the public through publication, or can be produced from artemisinin by semisynthesis.

For example, the compound represented by the formula (1), formula (2), formula (3-1), formula (3-2), formula (3-3), formula (4-1), formula (4-2), or formula (4-3) can be produced from a known compound such as artenimol and artemisinin by semisynthesis.

In the following, description will be made with reference to examples of the compound represented by the formula (1); however, the compound represented by the formula (2), formula (3-1), formula (3-2), formula (3-3), formula (4-1), formula (4-2), or formula (4-3) can also be produced in accordance with the following production method.

For example, a compound in which Y is hydroxy, a halogen, a hydrogen atom, carboxy, COOR¹, or R² (a heteroaryl such as furan, or an alkyl such as methyl) can be produced according to the following reaction formula or a similar method: 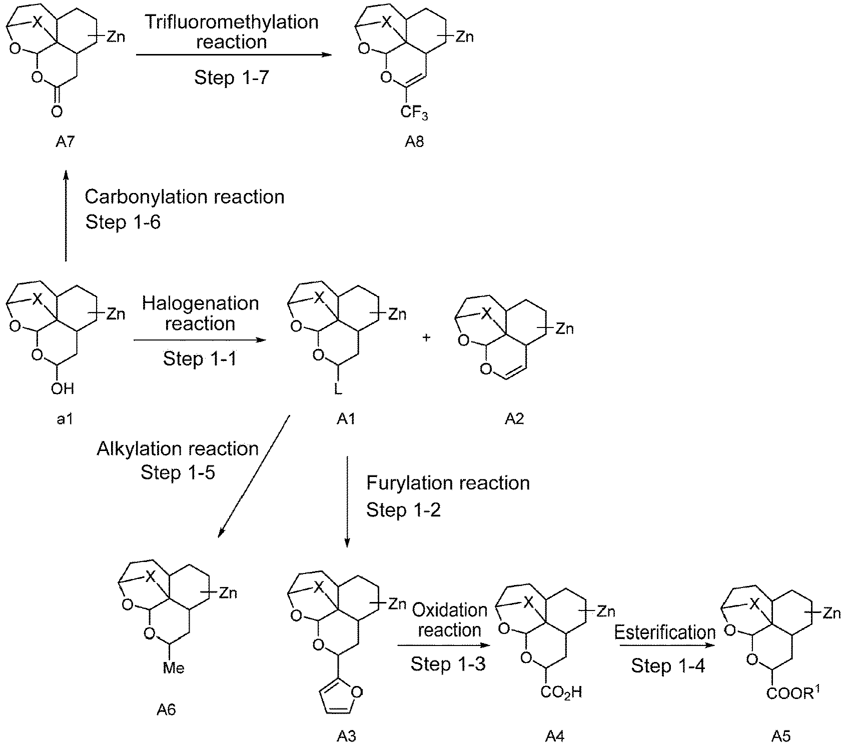 wherein X, Z, n, and R¹ are as defined in the formula (1), and L indicates a leaving group such as a halogen.

For compound a1 as the starting raw material, artenimol, deoxydihydroartemisinin, and the like are known compounds and available. Artenimol can be synthesized, for example, according to a method described in Chem Commun 2014, 50, 12652-12655, and dihydroartemisinic acid, which is described in the literature, can be chemically synthesized, for example, according to a method described in Tetrahedron 2016, 72 (32), 4931-4937. Deoxydihydroartemisinin can be chemically synthesized from artenimol as a raw material, for example, according to a method described in ChemMedChem 2012, 7 (12), 2204-2226. Thus, various types of a1 can be produced with reference to the methods described in them.

Compounds A1 and A2 can be produced by reacting compound a1 with a fluorinating reagent. An appropriate solvent is selected from exemplary solvents shown later and the like, and preferable examples of the solvent include dichloromethane. Examples of the fluorinating reagent include N,N-diethylaminosulfur trifluoride. The reaction time is typically 5 minutes to 72 hours, and preferably 30 minutes to 24 hours. The reaction temperature is typically -78°C to 100°C, and preferably - 30°C to 40°C.

Compound A3 can be produced by reacting compound A1 with furan in an appropriate solvent in the presence of a Lewis acid. Examples of the Lewis acid include boron trifluoride-diethyl ether complex. An appropriate solvent is selected from exemplary solvents shown later and the like, and preferable examples of the solvent include dichloromethane. The reaction time is typically 5 minutes to 72 hours, and preferably 30 minutes to 24 hours. The reaction temperature is typically -78°C to 20°C, and preferably -78°C to -20°C. With use of another heteroaryl in place of furan, such a compound that R² is a heteroaryl in the formula (1) can be produced.

Compound A4 can be produced by reacting compound A3 with a specific periodate in the presence of a metal catalyst. Examples of the metal catalyst include ruthenium dioxide. Examples of the periodate include sodium periodate and potassium periodate. An appropriate solvent is selected from exemplary solvents shown later and the like, and preferable examples of the solvent include acetonitrile, carbon tetrachloride, and water. The reaction time is typically 5 minutes to 72 hours, and preferably 30 minutes to 24 hours. The reaction temperature is typically -20°C to 70°C, and preferably -0°C to 40°C.

Compound A5 can be produced by reacting compound A4 with the corresponding alcohol in the presence or absence of a specific condensing agent and/or base in an appropriate solvent, wherein a catalyst is used, as necessary. Various condensing agents for use in conventional methods can be used as the condensing agent, and preferable examples of the condensing agent include 1-ethyl-3-(3-dimethylaminopropyl)carbodiimide (including the hydrochloride thereof). An appropriate base is selected from exemplary bases shown later and the like, and preferable examples of the base include diisopropylethylamine and triethylamine. Examples of the catalyst include 4-dimethylaminopyridine. An appropriate solvent is selected from exemplary solvents shown later and the like, and preferable examples of the solvent include tetrahydrofuran, dimethylformamide, and dichloromethane. The reaction time is typically 5 minutes to 72 hours, and preferably 30 minutes to 24 hours. The reaction temperature is typically 0°C to 200°C, and preferably 0°C to 100°C.

Compound A6 can be produced by reacting compound A1 with trimethylaluminum in an appropriate solvent. An appropriate solvent is selected from exemplary solvents shown later and the like, and preferable examples of the solvent include toluene and dichloromethane. The reaction time is typically 5 minutes to 72 hours, and preferably 30 minutes to 24 hours. The reaction temperature is typically -78°C to 50°C, and preferably -40°C to 30°C. Likewise, with use of a reagent well known to those skilled in the art such as an alkylating agent, for example, such a compound that R² in the formula (1) is an alkyl can be produced.

Compound A7 can be produced, for example, according to a method described in Organic Letters 2007, 9, 21, 4107-4110 or ACS Catalysis 2017, 7, 3, 1998-2001, or by a combination of them.

Compound A8 can be produced, for example, according to a method of J. Org. Chem. 2002, 67, 4, 1253-1260. Specifically, the method is such that a specific nucleophilic reagent is reacted with the carbonyl group of compound A7 in the presence or absence of a proper additive, the tertiary alcohol as the product is converted into a leaving group, and then dehydration is performed. Examples of the nucleophilic reagent include trifluoromethyltrimethylsilane and Grignard reagents. Examples of the additive include tetrabutylammonium fluoride. Examples of the reagent for converting into a leaving group include thionyl chloride. An appropriate solvent is selected from exemplary solvents shown later and the like, and preferable examples of the solvent include tetrahydrofuran and pyridine. The reaction time is typically 5 minutes to 72 hours, and preferably 30 minutes to 24 hours. The reaction temperature is typically -78°C to 150°C, and preferably -40°C to 120°C.

For example, a compound in which Y is SR¹, SOR¹, or SO₂R¹ can be produced by the following method: 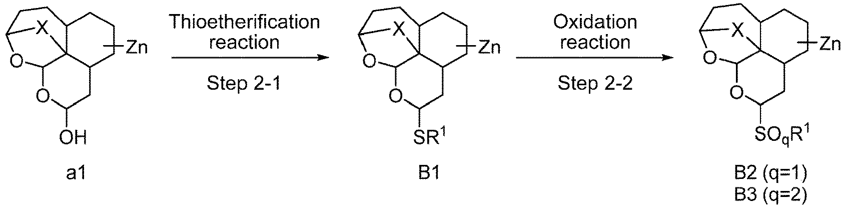 wherein X, Z, n, and R¹ are as defined in the formula (1), and q is 1 or 2.

Compound B1 can be produced by reacting compound a1 with the corresponding disulfide compound in an appropriate solvent in the presence of a Lewis acid and a reducing agent. Examples of the Lewis acid include boron trifluoride-diethyl ether complex. Examples of the reducing agent include triphenylphosphine. An appropriate solvent is selected from exemplary solvents shown later and the like, and preferable examples of the solvent include acetonitrile. The reaction time is typically 5 minutes to 72 hours, and preferably 30 minutes to 24 hours. The reaction temperature is typically -50°C to 100°C, and preferably - 20°C to 50°C.

Compounds B2 and B3 can be produced by reacting compound B1 with trifluoroacetic anhydride and a urea/hydrogen peroxide reagent in an appropriate solvent in the presence of a base. Examples of the base include sodium hydrogen carbonate. An appropriate solvent is selected from exemplary solvents shown later and the like, and preferable examples of the solvent include acetonitrile. The reaction time is typically 5 minutes to 72 hours, and preferably 30 minutes to 24 hours. The reaction temperature is typically -40°C to 50°C, and preferably -30°C to 30°C.

For example, a compound in which Y is R² (an aryl such as phenyl) or NR³R⁴ can be produced by the following method: 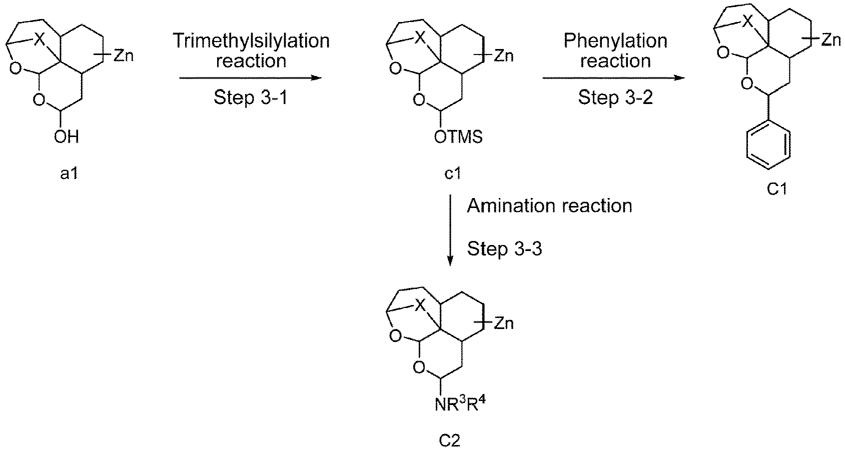 wherein X, Z, n, R³, and R⁴ are as defined in the formula (1), and TMS means trimethylsilyl.

Compound c1 can be produced by reacting compound a1 with trimethylsilyl chloride in an appropriate solvent in the presence of a base. An appropriate base is selected from exemplary bases shown later and the like, and preferable examples of the base include diisopropylethylamine and triethylamine. An appropriate solvent is selected from exemplary solvents shown later and the like, and preferable examples of the solvent include dichloromethane. The reaction time is typically 5 minutes to 72 hours, and preferably 30 minutes to 24 hours. The reaction temperature is typically -50°C to 100°C, and preferably -20°C to 50°C.

Compound C1 can be produced by reacting compound c1 with phenylmagnesium bromide in an appropriate solvent in the presence of trimethylsilyl bromide. An appropriate solvent is selected from exemplary solvents shown later and the like, and preferable examples of the solvent include tetrahydrofuran. The reaction time is typically 5 minutes to 72 hours, and preferably 30 minutes to 24 hours. The reaction temperature is typically -50°C to 100°C, and preferably -20°C to 70°C. Likewise, with use of a reagent well known to those skilled in the art such as arylmagnesium bromide, for example, such a compound that R² in the formula (1) is an aryl can be produced.

Compound C2 can be produced by reacting compound c1 with the corresponding amine in an appropriate solvent in the presence of trimethylsilyl bromide. An appropriate solvent is selected from exemplary solvents shown later and the like, and preferable examples of the solvent include tetrahydrofuran and dichloromethane. The reaction time is typically 5 minutes to 72 hours, and preferably 30 minutes to 24 hours. The reaction temperature is typically -50°C to 100°C, and preferably -20°C to 70°C.

For example, a compound in which Y is OCOR¹ can be produced by the following method: 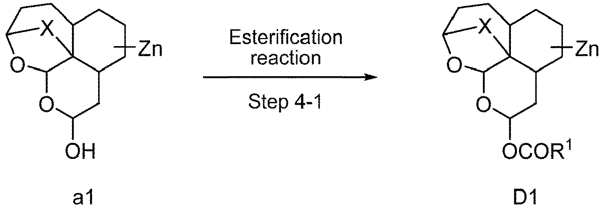 wherein X, Z, n, and R¹ are as defined in the formula (1).

For example, compound D1 is produced by reacting compound a1 with a specific imidating reagent in the presence of a specific base in an appropriate solvent to obtain an imidate compound and then treating with carboxylic acid in the presence or absence of a Lewis acid. Examples of the imidating reagent to be used in the step of imidation include trichloroacetonitrile and trifluoro-N-phenylacetimidoyl chloride. Examples of the base to be used in the imidoylation step include organic bases such as diazabicycloundecene and triethylamine and inorganic bases such as sodium hydride and potassium carbonate, whereas selection is appropriately made according to the imidoylating reagent to be used. Preferable examples of the carboxylic acid to be used in the step of carboxylic acid treatment include benzoic acid and acetic acid, and more preferable examples thereof include benzoic acid. Preferable examples of the Lewis acid to be used in the step of carboxylic acid treatment include boron trifluoride-diethyl ether complex. An appropriate solvent for use in the imidoylation step is selected from exemplary solvents shown later and the like, and preferable examples of the solvent include a halogen-containing solvent and an ether solvent, and more preferable examples thereof include dichloromethane and tetrahydrofuran. The reaction time is typically 5 minutes to 48 hours, and preferably 10 minutes to 2 hours. The reaction temperature is typically -78°C to 100°C, and preferably -10°C to 30°C. Alternatively, the production is performed by reacting with the corresponding acyl halide, the corresponding carboxylic acid, the corresponding acid anhydride, or the like in the presence or absence of a specific base, wherein a condensing agent and a catalyst are used, as necessary. An appropriate base is selected from exemplary bases shown later and the like, and preferable examples of the base include diisopropylethylamine and triethylamine. Various condensing agents for use in conventional methods can be used as the condensing agent, and preferable examples of the condensing agent include 1-ethyl-3-(3-dimethylaminopropyl)carbodiimide (including the hydrochloride thereof). Examples of the catalyst include 4-dimethylaminopyridine. An appropriate solvent is selected from exemplary solvents shown later and the like, and preferable examples of the solvent include tetrahydrofuran, dimethylformamide, and dichloromethane. The reaction time is typically 5 minutes to 72 hours, and preferably 30 minutes to 24 hours. The reaction temperature is typically 0°C to 200°C, and preferably 0°C to 100°C. The present reaction can be performed, for example, by using a method described in Eur. J. Org. Chem. 2002, 113-132 in the same manner as described therein.

For example, a compound in which Y is NR⁵COR¹, NR⁵SO₂R¹, NR⁵CONR³R⁴, or 1,2,3-triazole can be produced by the following method: 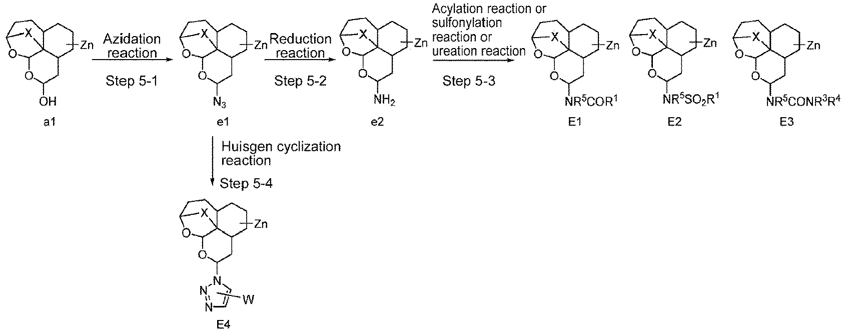 wherein X, Z, n, R¹, R³, R⁴, and R⁵ are as defined in the formula (1), and W is a substituent listed in [Group 1] in [6].

Compound e1 can be produced by reacting compound a1 with sodium azide in an appropriate solvent in the presence of trimethylsilyl bromide. An appropriate solvent is selected from exemplary solvents shown later and the like, and preferable examples of the solvent include dichloromethane. The reaction time is typically 5 minutes to 72 hours, and preferably 30 minutes to 24 hours. The reaction temperature is typically -50°C to 70°C, and preferably -20°C to 40°C.

Compound e2 can be produced by reacting compound e1 with an appropriate reducing agent in an appropriate solvent. Examples of the reducing agent include triphenylphosphine. An appropriate solvent is selected from exemplary solvents shown later and the like, and preferable examples of the solvent include tetrahydrofuran and water. The reaction time is typically 5 minutes to 72 hours, and preferably 30 minutes to 24 hours. The reaction temperature is typically -20°C to 120°C, and preferably 0°C to 80°C.

Compound E1 is produced by reacting compound e2 with the corresponding acyl halide, the corresponding carboxylic acid, the corresponding carboxylic anhydride, or the like in the presence or absence of a specific base, wherein a condensing agent and a catalyst are used, as necessary. An appropriate base is selected from exemplary bases shown later and the like, and preferable examples of the base include diisopropylethylamine and triethylamine. Various condensing agents for use in conventional methods can be used as the condensing agent, and preferable examples of the condensing agent include 1-ethyl-3-(3-dimethylaminopropyl)carbodiimide (including the hydrochloride thereof). Examples of the catalyst include 4-dimethylaminopyridine. An appropriate solvent is selected from exemplary solvents shown later and the like, and preferable examples of the solvent include tetrahydrofuran, dimethylformamide, and dichloromethane. The reaction time is typically 5 minutes to 72 hours, and preferably 30 minutes to 24 hours. The reaction temperature is typically 0°C to 200°C, and preferably 0°C to 100°C.

Compound E2 is produced by reacting compound e2 with the corresponding sulfonyl chloride in the presence of a specific base in an appropriate solvent. Examples of the base include triethylamine, pyridine, and 2,4,6-collidine. An appropriate solvent is selected from exemplary solvents shown later and the like, and preferable examples of the solvent include dichloromethane, tetrahydrofuran, and acetonitrile. The reaction time is typically 5 minutes to 72 hours, and preferably 30 minutes to 24 hours. The reaction temperature is typically 0°C to 200°C, and preferably 0°C to 80°C.

Compound E3 is produced by reacting compound e2 with the corresponding isocyanate, or treating compound e2 with a carbonylating reagent such as a phosgene derivative, carbonyldiimidazole, and chloroformate followed by reacting with the corresponding amine, in the presence or absence of a base in an appropriate solvent. An appropriate solvent is selected from exemplary solvents shown later and the like, and preferable examples of the solvent include dichloromethane and tetrahydrofuran. The reaction time is typically 5 minutes to 72 hours, and preferably 30 minutes to 24 hours. The reaction temperature is typically 0°C to 200°C, and preferably 0°C to 80°C.

Each of compound E1, E2, and E3 except the case that R⁵ is a hydrogen atom can be produced by introducing the corresponding R⁵ into compound e2 by using a method well known to those skilled in the art and then treating according to the above method, or by treating a compound being compound E1, E2, or E3 in which R³ is a hydrogen atom with a reactant such as R⁵-Cl and R⁵-Br, as appropriate, in the presence of a base.

Compound E4 is produced by reacting compound e1 with the corresponding alkyne compound in an appropriate solvent; this is what is called Huisgen cyclization reaction. While various conditions for this reaction have been reported, the reaction more smoothly proceeds by allowing a metal catalyst to act thereon in an appropriate solvent. An appropriate solvent is selected from exemplary solvents shown later and the like, and preferable examples of the solvent include dichloromethane and water. Monovalent copper is preferable as the metal catalyst, and a method of treating copper(II) sulfate with ascorbic acid to generate monovalent copper is more preferable. The reaction time is typically 5 minutes to 72 hours, and preferably 30 minutes to 24 hours. The reaction temperature is typically 0°C to 200°C, and preferably 20°C to 80°C.

For example, a compound in which Y is OR¹ can be produced by the following method: 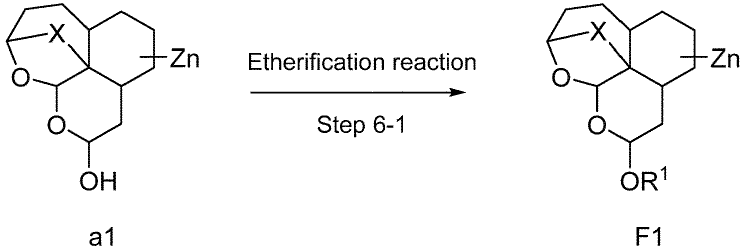 wherein X, Z, n, and R¹ are as defined in the formula (1).

Compound F1 is produced by reacting compound a1 with the corresponding alcohol in the presence of a specific Lewis acid in an appropriate solvent. An appropriate Lewis acid is selected from exemplary Lewis acids shown later and the like, and preferable examples of the Lewis acid include boron trifluoride-diethyl ether complex, trimethylsilyl chloride, and silver perchlorate. An appropriate solvent is selected from exemplary solvents shown later and the like, and preferable examples of the solvent include dichloromethane and diethyl ether. The reaction time is typically 5 minutes to 48 hours, and preferably 10 minutes to 24 hours. The reaction temperature is typically -78°C to 100°C, and preferably -78°C to 50°C.

For example, a compound in which Y is cyano, carbamoyl, or aminomethyl can be produced by the following method: 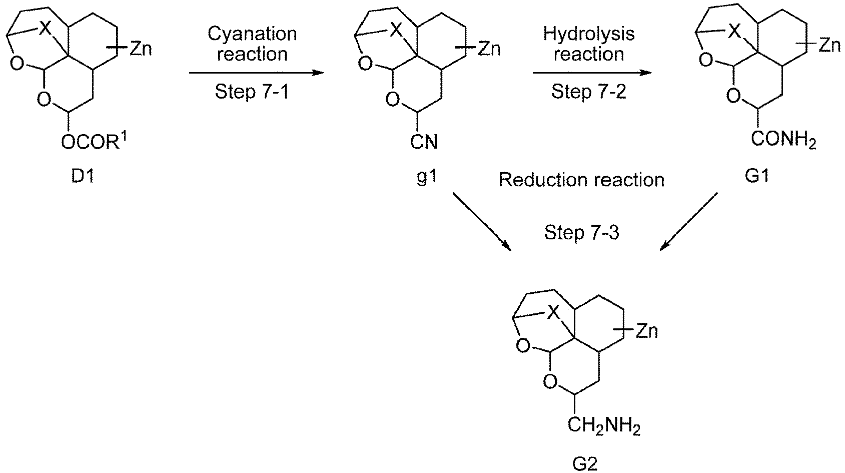 wherein X, Z, n, and R¹ are as defined in the formula (1), and in this case R¹ is preferably phenyl or methyl, and more preferably phenyl.

Compound g1 is produced by reacting compound D1 with a specific cyanizing reagent in the presence or absence of a specific Lewis acid in an appropriate solvent. An appropriate Lewis acid is selected from exemplary Lewis acids shown later and the like, and preferable examples of the Lewis acid include tin tetrachloride. Examples of the cyanizing reagent include trimethylsilyl cyanide, sodium cyanide, and potassium cyanide. An appropriate solvent is selected from exemplary solvents shown later and the like, and preferable examples of the solvent include a halogen-containing solvent and dimethylformamide. The reaction time is typically 5 minutes to 48 hours, and preferably 10 minutes to 24 hours. The reaction temperature is typically -78°C to 100°C, and preferably -78°C to 50°C.

Compound G1 can be produced by reacting compound g1 with a specific base in an appropriate solvent in the presence or absence of hydrogen peroxide. An appropriate base is selected from exemplary bases shown later and the like, and examples of the base include potassium carbonate and potassium hydroxide. An appropriate solvent is selected from exemplary solvents shown later and the like, and preferable examples of the solvent include tetrahydrofuran, tert-butyl alcohol, and water. The reaction time is typically 5 minutes to 72 hours, and preferably 30 minutes to 24 hours. The reaction temperature is typically 0°C to 100°C, and preferably 20°C to 70°C.

Compound G2 can be produced by reacting compound g1 or compound G1 with a specific reducing agent in an appropriate solvent in the presence of a Lewis acid. Examples of the Lewis acid include boron trifluoride-diethyl ether complex and triruthenium dodecacarbonyl. Examples of the reducing agent include a hydride reducing agent and a hydrosilane reducing agent, and more preferable examples of the reducing agent include sodium borohydride and 1,1,3,3-tetramethyldisiloxane. An appropriate solvent is selected from exemplary solvents shown later and the like, and preferable examples of the solvent include tetrahydrofuran and toluene. The reaction time is typically 5 minutes to 72 hours, and preferably 30 minutes to 24 hours. The reaction temperature is typically 0°C to 200°C, and preferably 20°C to 100°C.

For example, a compound in which Y is mercapto can be produced by the following method: 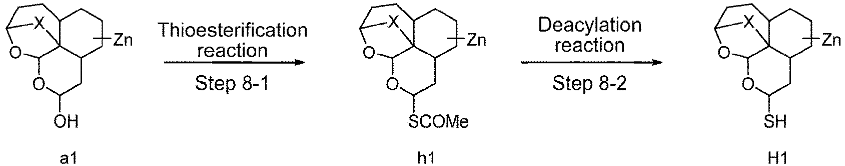 wherein X, Z, and n are as defined in the formula (1).

Compound h1 is produced by reacting compound a1 with thioacetic acid in the presence of a specific Lewis acid in an appropriate solvent. An appropriate Lewis acid is selected from exemplary Lewis acids shown later and the like, and preferable examples of the Lewis acid include boron trifluoride-diethyl ether complex. An appropriate solvent is selected from exemplary solvents shown later and the like, and preferable examples of the solvent include dichloromethane. The reaction time is typically 5 minutes to 48 hours, and preferably 10 minutes to 24 hours. The reaction temperature is typically -78°C to 100°C, and preferably -78°C to 50°C.

Compound H1 is produced by reacting compound h1 with a base in an appropriate solvent. An appropriate base is selected from exemplary bases shown later, and preferable examples of the base include sodium ethoxide. An appropriate solvent is selected from exemplary solvents shown later and the like, and preferable examples of the solvent include ethanol. The reaction time is typically 5 minutes to 48 hours, and preferably 10 minutes to 24 hours. The reaction temperature is typically -78°C to 150°C, and preferably -20°C to 80°C.

For example, a compound in which Y is represented by COR¹ can be produced by the following method: 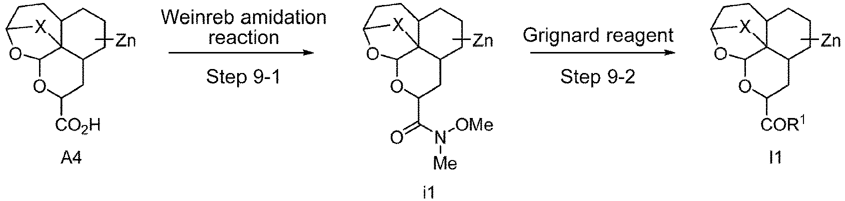 wherein X, Z, n, and R¹ are as defined in the formula (1).

Compound i1 is produced by reacting compound A4 with N,O-dimethylhydroxylamine or a salt thereof in the presence or absence of a specific condensing agent and/or base in an appropriate solvent, wherein a catalyst is used, as necessary. Various condensing agents for use in conventional methods can be used as the condensing agent, and preferable examples of the condensing agent include 1-ethyl-3-(3-dimethylaminopropyl)carbodiimide (including the hydrochloride thereof). An appropriate base is selected from exemplary bases shown later and the like, and preferable examples of the base include diisopropylethylamine and triethylamine. Examples of the catalyst include 4-dimethylaminopyridine. An appropriate solvent is selected from exemplary solvents shown later and the like, and preferable examples of the solvent include tetrahydrofuran, dimethylformamide, and dichloromethane. The reaction time is typically 5 minutes to 72 hours, and preferably 30 minutes to 24 hours. The reaction temperature is typically 0°C to 200°C, and preferably 0°C to 100°C.

Compound I1 is produced by reacting compound i1 with a Grignard reagent in an appropriate solvent. An appropriate solvent is selected from exemplary solvents shown later and the like, and preferable examples of the solvent include tetrahydrofuran and diethyl ether. The reaction time is typically 5 minutes to 48 hours, and preferably 10 minutes to 24 hours. The reaction temperature is typically -78°C to 150°C, and preferably -20°C to 80°C.

Selection should be made on timely basis for each of the bases to be used in the steps of the above production methods, for example, according to the types of the reaction and raw material compound, and examples of the bases include alkali bicarbonates such as sodium bicarbonate and potassium bicarbonate, alkali carbonates such as sodium carbonate and potassium carbonate, metal hydrides such as sodium hydride and potassium hydride, alkali metal hydroxides such as sodium hydroxide and potassium hydroxide, alkali metal alkoxides such as sodium methoxide and sodium t-butoxide, organometallic bases such as butyllithium and lithium diisopropylamide, and organic bases such as triethylamine, diisopropylethylamine, pyridine, 4-dimethylaminopyridine (DMAP), and 1,8-diazabicyclo[5.4.0]-7-undecene (DBU).

Selection should be made on timely basis for each of the solvents to be used in the steps of the above production methods, for example, according to the types of the reaction and raw material compound, and examples of the solvents include alcohols such as methanol, ethanol, and isopropanol, ketones such as acetone and methyl ketone, halogenated hydrocarbons such as methylene chloride and chloroform, ethers such as tetrahydrofuran (THF) and dioxane, aromatic hydrocarbons such as toluene and benzene, aliphatic hydrocarbons such as hexane and heptane, esters such as ethyl acetate and propyl acetate, amides such as N,N-dimethylformamide (DMF) and N-methyl-2-pyrrolidone, sulfoxides such as dimethyl sulfoxide (DMSO), and nitriles such as acetonitrile, and these solvents can be used singly or as a mixture of two or more of them. For some types of reaction, an organic base may be used as a solvent.

The compound of the present invention, for example, represented by the formula (1), or an intermediate thereof can be separated and/or purified by a method known to those skilled in the art. Examples thereof include extraction, partition, reprecipitation, column chromatography (e.g., silica gel column chromatography, ion-exchange column chromatography, or preparative liquid chromatography), and recrystallization.

Applicable as recrystallization solvent are, for example, alcoholic solvents such as methanol, ethanol, and 2-propanol, ether solvents such as diethyl ether, ester solvents such as ethyl acetate, aromatic hydrocarbon solvents such as benzene and toluene, ketone solvents such as acetone, halogen-containing solvents such as dichloromethane and chloroform, hydrocarbon solvents such as hexane, aprotic solvents such as dimethylformamide and acetonitrile, water, and mixed solvents or the like of them. As another purification method, for example, a method described in Experimental Chemistry (The Chemical Society of Japan, ed., Maruzen Publishing Co., Ltd.) Vol. 1 can be used. Determination of the molecular structure of the compound of the present invention can be readily performed through a spectrometric technique such as a nuclear magnetic resonance method, an infrared absorption method, and circular dichroism spectrometry, and mass spectrometry with reference to the structure derived from the corresponding raw material compound.

Any of the intermediates and the final products in the above production methods may be derivatized into another compound included in the scope of the present invention by appropriately converting its functional group, in particular, extending a specific side chain, for example, from amino, a hydroxy group, carbonyl, or a halogen, and performing the above-mentioned protection and deprotection at that time, as necessary. Specifically, if each group may be substituted in the compound represented by the formula (1), formula (2), formula (3-1), formula (3-2), formula (3-3), formula (4-1), formula (4-2), or formula (4-3), the compound can be produced through appropriate selection of a reagent to be used as a raw material, conversion of a functional group, or extension of a side chain, by a method well known to those skilled in the art. Conversion of a functional group and extension of a side chain can be achieved through a conventional, common method (e.g., see Comprehensive Organic Transformations, R. C. Larock, John Wiley & Sons Inc. (1999)). As appropriate, protection and deprotection can be performed, for example, according to a method described in Protective Groups in Organic Synthesis (Theodora W. Greene, Peter G. M. Wuts, published by John Wiley & Sons, Inc., 1999).

The compound of the present invention represented by the formula (1) or any other formula or a pharmaceutically acceptable salt thereof may undergo asymmetrization or have a substituent with an asymmetric carbon, and there are optical isomers for such a compound. The scope of the compound of the present invention includes mixtures of such isomers and isolated isomers, which can be produced according to a common method. Examples of the production method include methods using a raw material having an asymmetric point and methods of introducing asymmetrization in the middle of production.

For example, optical isomers of the compound represented by the formula (3-1) can be produced by a method described in Angewandte Chemie International Edition 2018, 57, 8293-8296 (e.g., such a compound that Y is oxo). The compound represented by the formula (3-2) can be produced, for example, by a method described in Organic Process Research & Development 2007, 11, 3, 336-340, or Bioorganic & Medicinal Chemistry Letters 2010, 20, 14, 4112-4115.

In the case of optical isomers, for example, optical isomers can be obtained by using an optically active raw material or by performing optical resolution or the like in an appropriate stage of the production process. Examples of optical resolution methods in the case that the compound represented by the formula (1) or an intermediate thereof has a basic functional group include a diastereomer method to form a salt in an inert solvent (e.g., an alcoholic solvent such as methanol, ethanol, and 2-propanol, an ether solvent such as diethyl ether, an ester solvent such as ethyl acetate, a hydrocarbon solvent such as toluene, an aprotic solvent such as acetonitrile, or a mixed solvent of two or more selected from the solvents presented) with use of an optically active acid (e.g., a monocarboxylic acid such as mandelic acid, N-benzyloxyalanine, and lactic acid, a dicarboxylic acid such as tartaric acid, o-diisopropylidenetartaric acid, and malic acid, a sulfonic acid such as camphorsulfonic acid and bromocamphorsulfonic acid). If the compound of the present invention represented by the formula (1) or an intermediate thereof has an acidic functional group such as a carboxyl group, optical resolution may be performed by forming a salt with use of an optically active amine (e.g., an organic amine such as 1-phenylethylamine, quinine, quinidine, cinchonidine, cinchonine, and strychnine).

A temperature in the range of -50°C to the boiling point of the solvent, preferably in the range of 0°C to the boiling point of the solvent, more preferably in the range of room temperature to the boiling point of the solvent, is selected as the temperature to form a salt. It is desirable for enhancing the optical purity to temporarily raise the temperature nearly to the boiling point of the solvent. The yield can be enhanced by cooling, as necessary, in collecting a salt precipitated. It is appropriate that the amount of usage of the optically active acid or amine be in the range of approximately 0.5 to approximately 2.0 equivalents, preferably in the range around 1 equivalent, with respect to the substrate. As necessary, crystals may be recrystallized in an inert solvent (e.g., an alcoholic solvent such as methanol, ethanol, and 2-propanol; an ether solvent such as diethyl ether; an ester solvent such as ethyl acetate; a hydrocarbon solvent such as toluene; an aprotic solvent such as acetonitrile; or a mixed solvent of two or more selected from the solvents presented) to obtain an optically active salt of high purity. Further, a salt obtained by optical resolution may be treated with an acid or a base by using a common method to obtain a free form, as necessary.

Among the raw materials and intermediates in the production methods described above, those for which no production method is described are commercially available compounds, or can be synthesized from commercially available compounds by a method known to those skilled in the art, or a method based thereon.

### Culture Medium and Culture Conditions

The culture medium to culture a cell population including hematopoietic stem cells can be a culture medium suitable for maintenance culture of hematopoietic stem cells, and may be, for example, a culture medium prepared by adding Stem Cell Factor (SCF, manufactured by PeproTech, Inc.) and Thrombopoietin (TPO, manufactured by PeproTech, Inc.) to StemSpan SFEM culture medium (manufactured by STEMCELL Technologies Inc.), StemPro 34 (manufactured by Life Technologies), HemEx-Type 9A (manufactured by Nipro Corporation), Ham's F12 culture medium (Ham's Nutrient Mixture F12), DMEM (Dulbecco's Modified Eagle's Medium), RPMI1640 culture medium, IMDM culture medium (Iscove's Modified Dulbecco's Medium), or the like.

The culture medium contains the compound represented by the formula (1) or a salt thereof. Examples of the compound represented by the formula (1) include artemether, artemisinin, artenimol, artemotil, and artesunate. The concentration of each compound in the culture medium can be any concentration that allows hematopoietic stem cells to grow with the self-renewal capacity and multipotency maintained, and may be, for example, 0.01 µM to 100 µM, and preferably 0.03 µM to 10 µM, 0.1 µM to 10 µM, 0.1 µM to 3 µM, or 0.1 µM to 1 µM. The concentrations of artemether and artemisinin may be each 0.1 µM to 10 mM, and preferably 0.3 µM to 10 µM or 1 µM to 10 µM. Much lower concentrations may be employed for human hematopoietic stem cells. For example, the concentration of artemether may be 0.001 µM to 10 µM, 0.003 µM to 10 µM, 0.01 µM to 10 µM, or 0.03 µM to 10 µM.

The culture medium may contain, as appropriate, a component other than the compound such as serum, a fatty acid or fat, an amino acid, a vitamin, a growth factor, a cytokine, insulin, transferrin, an antioxidant, 2-mercaptoethanol, pyruvic acid, a buffer, an inorganic salt, and an antibiotic. Serum may be added to reach a serum concentration of 25% or less, preferably of 5% to 15%, more preferably of 8% to 10%.

Herein, the term "serum-containing culture medium" means a culture medium containing non-processed or unpurified serum, and the term "serum-free culture medium" means a culture medium containing no non-processed or unpurified serum. The serum-free culture medium may contain a serum substitute. Examples of serum substitutes include products appropriately containing albumin, transferrin, a fatty acid, a collagen precursor, a trace element, 2-mercaptoethanol, 3-thioglycerol, or an equivalent of any of them. Commercially available products of serum substitutes may be used. Examples thereof include Knockout (TM) Serum Replacement (KSR: manufactured by Life Technologies), Chemically-defined Lipid concentrated (manufactured by Life Technologies), and Glutamax (TM, manufactured by Life Technologies).

The conditions for culturing hematopoietic stem cells are not limited, and, for example, culture may be performed in a CO₂ incubator at 37°C and 5% CO₂. In seeding a cell population including hematopoietic stem cells, the cell population may be in a concentration of 10 cells/mL to 1 × 10⁸ cells/mL. Preferably, the cell population may be in a concentration of 100 cells/mL to 1 × 10⁷ cells/mL.

### Cell Population After Culture

The cell population obtained by culturing by the culture method of the present invention (herein, occasionally referred to as "the cell population after culture") is a cell population including hematopoietic stem cells. In contrast to conventional culture methods, in which most hematopoietic stem cells disappear through differentiation, the culture method of the present invention causes at least some hematopoietic stem cells not to differentiate, allowing hematopoietic stem cells to grow with the self-renewal capacity and multipotency maintained.

In an embodiment, the proportion of the hematopoietic stem cell count to the total cell count in the cell population after culture is 0.1%, 0.5%, 1%, 5%, 10%, 20%, or 30% or more, preferably 50% or more, more preferably 60% or more, 70% or more, 80% or more, 85% or more, or 90% or more of the proportion of the hematopoietic stem cell count to the total cell count in the cell population before culture. The proportion of the long-term hematopoietic stem cell count to the total cell count in the cell population after culture is preferably 0.1%, 0.5%, 1%, 5%, 10%, 20%, or 30% or more, preferably 50% or more, more preferably 60% or more, 70% or more, 80% or more, 85% or more, or 90% or more of the proportion of the long-term hematopoietic stem cell count to the total cell count in the cell population before culture. The proportion of hematopoietic stem cells in the cell population obtained by culturing by the culture method of the present invention can vary with the proportion of hematopoietic stem cells in the cell population before culture. In addition, the presence of hematopoietic stem cells can be functionally (e.g., on multipotency) evaluated by the above-described method. Alternatively, the proportion of hematopoietic stem cells in the cell population can be roughly estimated through comparison with isolated hematopoietic stem cells.

It follows that the present invention includes a method for maintaining the proportion of long-term hematopoietic stem cells in a cell population including long-term hematopoietic stem cells at 0.1%, 0.5%, 1%, 5%, 10%, 20%, or 30% or more, preferably at 50% or more, more preferably at 60% or more, 70% or more, 80% or more, 85% or more, or 90% or more.

In an embodiment, the number of hematopoietic stem cells in the cell population after culture is 1.2 or more times, 1.5 or more times, or 2 or more times, preferably 10 or more times, more preferably 20 or more times, 50 or more times, or 100 or more times the number of hematopoietic stem cells in the cell population before culture. In an embodiment, the number of hematopoietic stem cells in the cell population after culture is 1.2 or more times, 1.5 or more times, or 2 or more times, preferably 10 or more times, more preferably 20 or more times, 50 or more times, or 100 or more times the number of hematopoietic stem cells in a cell population obtained by a culture method under culture conditions matched except that the compound of the present invention is not contained. Preferably, the number of long-term hematopoietic stem cells in the cell population after culture is 1.2 or more times, 1.5 or more times, or 2 or more times, preferably 10 or more times, more preferably 20 or more times, 50 or more times, or 100 or more times the number of long-term hematopoietic stem cells in the cell population before culture. In an embodiment, the number of long-term hematopoietic stem cells in the cell population after culture is 1.2 or more times, 1.5 or more times, or 2 or more times, preferably 10 or more times, more preferably 20 or more times, 50 or more times, or 100 or more times the number of long-term hematopoietic stem cells in a cell population obtained by a culture method under culture conditions matched except that the compound of the present invention is not contained. That is, it is only needed for the hematopoietic stem cell count or long-term hematopoietic stem cell count to be maintained or increase as compared with that of a proper comparative sample.

Thus, the present invention includes a method for increasing the cell count of hematopoietic stem cells in a cell population including hematopoietic stem cells by 1.2 or more times, 1.5 or more times, or 2 or more times, preferably by 10 or more times, more preferably by 20 or more times, 50 or more times, or 100 or more times as compared with that of the above-mentioned comparative sample. In addition, the present invention includes a method for increasing the cell count of long-term hematopoietic stem cells in cell population including long-term hematopoietic stem cells by 1.2 or more times, 1.5 or more times, or 2 or more times, preferably by 10 or more times, more preferably by 20 or more times, 50 or more times, or 100 or more times as compared with that of the above-mentioned comparative sample.

Further, the present invention includes a culture method involving increasing the hematopoietic stem cell count in a cell population including hematopoietic stem cells by 1.2 or more times, 1.5 or more times, or 2 or more times, preferably by 10 or more times, more preferably by 20 or more times, 50 or more times, or 100 or more times as compared with that of the above-mentioned comparative sample, and maintaining the proportion of hematopoietic stem cells to the total cell count at 0.1%, 0.5%, 1%, 5%, 10%, 20%, or 30% or more, preferably at 50% or more, more preferably at 60% or more, 70% or more, 80% or more, 85% or more, or 90% or more.

Further, the present invention includes a culture method involving increasing the cell count of long-term hematopoietic stem cells in a cell population including long-term hematopoietic stem cells by 1.2 or more times, 1.5 or more times, or 2 or more times, preferably by 10 or more times, more preferably by 20 or more times, 50 or more times, or 100 or more times as compared with that of the above-mentioned comparative sample, and maintaining the proportion of long-term hematopoietic stem cells to the total cell count at 0.1%, 0.5%, 1%, 5%, 10%, 20%, or 30% or more, preferably at 50% or more, more preferably at 60% or more, 70% or more, 80% or more, 85% or more, or 90% or more.

Further, the present invention includes a culture method involving amplifying cells for forming a CFU-GEMM colony in CFU assay. The present invention includes, as an embodiment, a culture method, wherein the CFU-GEMM colony count given by a cell population obtained by the culture method of the present invention increases to 1.2 or more times, 1.5 or more times, 2 or more times, 5 or more times, or 10 or more times the CFU-GEMM colony count given by a comparative sample. The comparative sample in this situation may be, for example, a cell population obtained by a culture method under culture conditions matched except that the compound of the present invention is not contained, or the cell population before culture. Thus, the culture methods according to [1] to [11] in the above can be regarded as methods for culturing one or more cells or cell population having an ability to form a mixed colony derived from hematopoietic stem cells (CFU-GEMM).

Moreover, the present invention includes a culture method involving amplifying a cell capable of being engrafted for a long period of time (for 1 month or more, preferably for 2 months or more, 3 months or more, or 4 months or more after transplantation) in transplantation experiment for immunocompromised NOG mice exposed to a lethal dose of radiation. For the methods for culturing one or more human hematopoietic stem cells, as described above, the proportion of human CD45-positive cells in the blood of a NOG mouse can be used as an index, and those skilled in the art could arbitrarily set dates to perform evaluation of the index after transplantation (for example, 1 month, 2 months, 3 months, or 4 months after transplantation). The present invention includes, as an embodiment, a culture method, wherein the proportion of human CD45-positive cells when a cell population obtained by the culture method of the present invention is transplanted increases to 1.2 or more times, 1.5 or more times, 2 or more times, or 10 or more times the proportion of human CD45-positive cells when a comparative sample is transplanted. The comparative sample in this situation may be, for example, a cell population obtained by a culture method under culture conditions matched except that the compound of the present invention is not contained, or the cell population before culture. Thus, the culture methods according to [1] to [11] in the above can be regarded as methods for culturing a cell population that increases the ability to produce mature blood cells when being transplanted into a mammal. Alternatively, the culture methods according to [1] to [11] in the above can be regarded as methods for culturing a cell population that increases at least one of, preferably all of the abilities of engraftment potential to the bone marrow, multipotency, and bone marrow reconstruction potential.

The cell population including hematopoietic stem cells obtained by culturing by the culture method of the present invention may be immediately used for transplantation, or cryopreserved if it is not used immediately. If cells collected from blood are frozen, for example, the cryopreservation method may involve separation and removal of erythrocyte, plasma, and leukocyte fractions, and use of a preservation solution containing 8% dimethyl sulfoxide and 0.8% dextran, or HSC-BANKER GMP grade (manufactured by Nippon Zenyaku Kogyo Co., Ltd.).

It is critical in transplantation treatment that transplanted cells function in a sustained manner in the body of a recipient, and it is important therefor that cells including many hematopoietic stem cells having self-renewal capacity, in particular, long-lasting self-renewal capacity are transplanted. The cell population including hematopoietic stem cells obtained by culturing by the culture method of the present invention include many hematopoietic stem cells, in particular, many long-term hematopoietic stem cells, and has high self-renewal ability and multipotency, hence being superior in long-lasting engraftment potential, multipotency, and bone marrow reconstruction potential in use for hematopoietic stem cell transplantation. Transplantation of undifferentiated cell fractions such as long-term hematopoietic stem cells is expected to provide GVHD-suppressing effect.

### [Culture]

The present invention provides, in an aspect, a culture of a cell population including hematopoietic stem cells, comprising: (1) a cell population including hematopoietic stem cells obtained by culturing by the culture method of the present invention; and (2) a medium necessary for maintaining the viability of a hematopoietic stem cell population. It is preferable that the hematopoietic stem cells be long-term hematopoietic stem cells.

The term "culture" as used herein means a liquid containing a medium necessary for maintaining viability and a cell population, and optionally containing a biological substance further added or produced by the cell population. Examples of the biological substance include, but are not limited to, cytokines and chemokines.

In an embodiment, the proportion of hematopoietic stem cells (cells expressing the above-mentioned markers, for example, CD34-positive CD38-negative cells) to the total cell count in the cell population in the culture is 0.1%, 0.5%, 1%, 5%, 10%, 20%, or 30% or more, and preferably 50% or more, 60% or more, 70% or more, 80% or more, 85% or more, or 90% or more. In another embodiment, the proportion of the long-term hematopoietic stem cell count to the total cell count in the cell population in the culture is 0.1%, 0.5%, 1%, 5%, 10%, 20%, or 30% or more, and preferably 50% or more, 60% or more, 70% or more, 80% or more, 85% or more, or 90% or more.

Examples of the "medium necessary for maintaining viability" as used herein include culture media and physiological buffer solutions, whereas the medium is not limited as long as the cell population including hematopoietic stem cells survives, and those skilled in the art could select appropriate one. An example is the above-described culture medium containing, as a minimum essential medium, a culture medium that is commonly used in animal cell culture. For example, fetal bovine serum, human serum, horse serum, insulin, transferrin, lactoferrin, cholesterol, ethanolamine, sodium selenite, monothioglycerol, 2-mercaptoethanol, bovine serum albumin, sodium pyruvate, polyethylene glycol, vitamins, amino acids, agar, agarose, collagen, methylcellulose, and so on can be contained in the culture medium. The medium may contain the compound represented by the formula (1) or a salt thereof. The concentration of the compound or a derivative thereof, or a salt of any of them may be in the above-mentioned concentration range. The concentration may be, for example, 0.001 µM tο 100 µM, 0.01 µM to 100 µM, and preferably 0.003 µM to 100 µM, or 0.03 µM to 10 µM.

The "medium necessary for maintaining viability" as used herein may further contain cytokines or substances that act on hematopoietic stem cells. Examples of the cytokines include, but are not limited to, IL-1, IL-3, IL-6, IL-11, G-CSF, GM-CSF, SCF, FLT3-L, thrombopoietin (TPO), erythropoietin, and analogs of them. The "analogs" as used herein include all sorts of structural variants of cytokines and growth factors having natural-like biological activity. Examples of the substances that act on hematopoietic stem cells include, but are not limited to, pyrimido[4,5-b]indole derivatives described in Patent Literature 2, which are UM171 ((1r,4r)-N1-(2-benzyl-7-(2-methyl-2H-tetrazol-5-yl)-9H-pyrimido[4,5-b]indol-4-yl)cyclohexane-1,4-diamine) and a derivative thereof (e.g., UM729 (methyl 4-((3-(piperidin-1-yl)propyl)amino)-9H-pyrimido[4,5-b] indole-7-carboxylate). The effects of UM171 and the like that maintain or amplify hematopoietic stem cells have been reported (Patent Literature 2, Non Patent Literature 2).

The culture medium to be used in the culture method of the present invention may contain UM171 and a derivative thereof. For the derivative, for example, reference can be made to Patent Literature 2 (WO 2013/110198).

### [Production Method]

The method for producing one or more hematopoietic stem cells comprises: a step of preparing a cell population including hematopoietic stem cells; and a step of culturing the cell population including hematopoietic stem cells, wherein the cell population including hematopoietic stem cells is cultured by the culture method of the present invention.

The method of the present invention for producing one or more hematopoietic stem cells in an embodiment comprises:
(1) a step of preparing a cell population including hematopoietic stem cells;
(2) a step of culturing a cell population including the hematopoietic stem cells; and
(3) a step of collecting hematopoietic stem cells.

### Step (1)

In step (1) of the production method of the present invention, specifically, in the step of preparing a cell population including hematopoietic stem cells, the cell population including hematopoietic stem cells can be prepared by using a method that would be well known to those skilled in the art. For example, the cell population can be prepared by collection from blood (peripheral blood or umbilical cord blood) or bone marrow, or artificial production from pluripotent stem cells (examples: iPS cells, ES cells). The cell population including hematopoietic stem cells is also available as a commercially available product.

In a method of collection from bone marrow, an appropriate volume of bone marrow is collected from a mammal such as a human, a monkey, a mouse, and a rat, a buffer obtained by adding thermally deactivated bovine serum (manufactured by Gibco) at a final concentration of 2% and EDTA solution at a final concentration of 2 mM to Ca²⁺-free and Mg²⁺-free PBS is added, the bone marrow tissue is mechanically broken, and thus the cell population including hematopoietic stem cells is successfully obtained.

In a method of collection from blood, an appropriate volume of blood is collected from a mammal such as a human, a monkey, a mouse, and a rat, a PBMC fraction is prepared according to a conventional method, and thus the cell population including hematopoietic stem cells is successfully obtained. For the blood, either peripheral blood or umbilical cord blood may be used. The blood may be mobilized blood obtained by administering an agent such as granulocyte colony-stimulating factor (G-CSF) according to a conventional method. Hematopoietic stem cells can be mobilized into peripheral blood by administering an agent such as granulocyte colony-stimulating factor (G-CSF).

A method that would be known to those skilled in the art can be used for collection from umbilical cord blood. In an example, a neonate and an umbilical cord are cut out from a mammal such as a human, a monkey, a mouse, and a rat, a needle for collection is inserted into the umbilical cord vascular, and the umbilical cord blood is collected into a bag for collection. Subsequently, erythrocytes are aggregated by addition of hydroxyethyl starch, the resultant is centrifuged, the erythrocytes and plasma are then removed, and thus the cell population including hematopoietic stem cells is successfully obtained.

The cell population including hematopoietic stem cells may be filtered through strainers of 100 µm, 70 µm, and 40 µm. From the cell population filtered, a hematopoietic stem cell and progenitor cell populations may be enriched by staining with a fluorescence-labeled antibody for any of the above hematopoietic stem cell markers and collecting cells positive for the hematopoietic stem cell marker by MACS (magnetic-activated cell sorting), FACS (fluorescence-activated cell sorter), or the like.

The cell types of hematopoietic stem cells, hematopoietic progenitor cells, and others in the resulting cell population including hematopoietic stem cells can be confirmed by performing marker expression analysis with flow cytometry or qRT-PCR for hematopoietic stem cells, hematopoietic progenitor cells, and others. A system that expresses a fluorescent protein such as mCherry may be used for the downstream of a marker gene promoter to collect cells positive for the marker gene (i.e., long-term hematopoietic stem cells) on the basis of expression of the fluorescent protein, as an indicator. Any of the above-mentioned genes can be used as the marker gene.

The proportion of hematopoietic stem cells in the thus-obtained cell population including hematopoietic stem cells is arbitrary, but it is preferable that the proportion be higher, and it is preferable to include hematopoietic stem cells, for example, in a proportion of 0.1%, 0.5%, 1%, 5%, 10%, 20%, 30% or more, 50% or more, 60% or more, 70% or more, 80% or more, 85% or more, or 90% or more. Mouse hematopoietic stem cells can be confirmed, as described above, by performing expression analysis for a hematopoietic stem cell marker through flow cytometer or qRT-PCR.

It is preferable for the thus-obtained cell population including hematopoietic stem cells to include long-term hematopoietic stem cells, and it is preferable to include long-term hematopoietic stem cells, for example, in a proportion of 0.1%, 0.5%, 1%, 5%, 10%, 20%, 30% or more, 50% or more, 60% or more, 70% or more, 80% or more, 85% or more, or 90% or more. Long-term hematopoietic stem cells can be confirmed, as described above, by expression of a maker such a s Hoxb5.

A known method can be used as a method for artificially producing a cell population including hematopoietic stem cells from pluripotent stem cells (for example, iPS cells, ES cells). Examples thereof in an embodiment include a method described in Non Patent Literature shown below. Specifically, embryoid bodies are produced from iPS cells, and CD34-positive FLK1-positive cells are then isolated. Thereafter, ERG, HoxA5, HoxA9, HoxA10, LCOR, RUNX1, and SPI1 are expressed as transcription factors, and thus a cell population including hematopoietic stem cells is successfully obtained (Nature. 2017 25: 545 (7655) 432-438).

Step (1) may include, for example, a step of preculturing a cell population including hematopoietic stem cells and/or a step of collecting hematopoietic stem cells (long-term hematopoietic stem cells) by using a specific marker.

### Step (2)

Step (2) of the production method of the present invention, that is, the culture step is equivalent to that of any method described as the culture method of the present invention. Specifically, the culture step includes culturing in a culture medium containing the compound of the present invention. The resultant of step (2) is a cell population including hematopoietic stem cells (hereinafter, occasionally referred to as "the cell population including hematopoietic stem cells produced by the production method of the present invention"). The cell population including hematopoietic stem cells produced by the production method of the present invention is equivalent to the cell population including hematopoietic stem cells obtained by culturing by the culture method of the present invention.

Those skilled in the art could appropriately set culture conditions for a cell population including hematopoietic stem cells in the culture method of the present invention. The concentration of the compound of the present invention can be set within the above-mentioned range of concentration. Likewise, the culture days is not limited, and may be, for example 1 to 30 days, and preferably 21 days, 14 days, 7 days, or 3 days. Culture conditions including them may be set on the basis of required condition of amplification of hematopoietic stem cells. In an embodiment, culture may be performed until the hematopoietic stem cell count (a cell population defined on the basis of state of positiveness and negativeness for the above-mentioned markers may be applied) increases to 1.2 or more times, 1.5 or more times, or 2 or more times, preferably 10 or more times, more preferably 20 or more times, 50 or more times, or 100 or more times that of the above-mentioned comparative sample. In an embodiment, conditions that allow the CFU-GEMM colony count to increase (for example, conditions that allow the CFU-GEMM colony count to increase to 1.2 or more times, 1.5 or more times, 2 or more times, or 10 or more times that of a comparative sample) in CFU assay may be set.

Culture may be performed with a culture medium further containing any of the above-mentioned cytokines. Specifically, the culture medium may be that containing one or more selected from the group consisting of IL-1, IL-3, IL-6, IL-11, G-CSF, GM-CSF, SCF, FLT3-L, thrombopoietin (TPO), erythropoietin, and analogs of them. In an embodiment, a culture medium containing IL-6, SCF, FLT3-L, and TPO may be used.

Culture may be performed in combination with a known substance that is known to maintain and/or amplify hematopoietic stem cells, unless the known substance inhibits the effects of the compound of the present invention. For example, culture may be performed with a culture medium further containing UM171 or a derivative thereof. The concentration of UM171 or a derivative thereof depends on culture conditions, and those skilled in the art could appropriately set it. In an embodiment, the concentration of UM171 or a derivative thereof may be 1 nM to 10 µM. As described above, the concentration may be set on the basis of the hematopoietic stem cell count or CFU-GEMM colony count, as an index.

### Step (3)

Step (3) of the production method of the present invention is, specifically, a step of collecting hematopoietic stem cells (long-term hematopoietic stem cells), in a collection step, from the cell population including hematopoietic stem cells obtained by culturing in step (2) with use of a specific marker. For example, hematopoietic stem cells or long-term hematopoietic stem cells may be collected from the cell population including hematopoietic stem cells obtained by culturing in step (2) through MACS, FACS, or the like with use of any of the above-mentioned markers for hematopoietic stem cells.

The production method of the present invention may further include, for example, a step of packing the hematopoietic stem cells collected in step (3) in a container (e.g., an infusion bag) and/or a step of freezing the cells obtained.

### [Reagent for Culturing Hematopoietic Stem Cell]

The present invention provides, in an aspect, a reagent for culturing one or more hematopoietic stem cells, comprising: at least one compound represented by any of the formula (1), formula (2), formula (3-1), formula (3-2), formula (3-3), formula (4-1), formula (4-2), and formula (4-3) or a salt thereof.

### [Kit]

The present invention provides, in an aspect, a kit for culturing one or more hematopoietic stem cells, comprising: (1) a culture medium suitable for maintenance culture of hematopoietic stem cells; and (2) a compound represented by any of the formula (1), formula (2), formula (3-1), formula (3-2), formula (3-3), formula (4-1), formula (4-2), and formula (4-3) or a salt thereof (herein, referred to as "the compound of the present invention").

Examples of the compound represented by the formula (1), in other words, preferable examples of the compound of the present invention include the following compounds:
artemether,
artemisinin,
artenimol,
artemotil,
artesunate, and compounds of Examples 1-1 to 1-28 and compounds of Examples 1-33 and 1-34.

Preferable examples of the compound represented by the formula (1) include the following compounds:
artemether,
artemisinin,
artenimol,
artemotil,
artesunate,
(3R,5aS,6R,8aS,9R,10S,12R,12aR)-3,6,9-trimethyldecahydro-12H-3,12-epoxypyrano[4,3-j][1,2]benzodioxepine-10-carboxamide, (3R,5aS,6R,8aS,9R,10R,12R,12aR)-3,6,9-trimethyldecahydro-12H-3,12-epoxypyrano[4,3-j][1,2]benzodioxepine-10-carboxamide, 1-[(3R,5aS,6R,8aS,9R,10S,12R,12aR)-3,6,9-trimethyldecahydro-12H-3,12-epoxypyrano[4,3-j][1,2]benzodioxepin-10-yl]methanamine, 1-[3R,(5aS,6R,8aS,9R,10R,12R,12aR)-3,6,9-trimethyldecahydro-12H-3,12-epoxypyrano[4,3-j][1,2]benzodioxepin-10-yl]methanamine, and (3R,5aS,6R,8aS,9R,10S,12R,12aR)-10-(2-methoxyethoxy)-3,6,9-trimethyldecahydro-12H-3,12-epoxypyrano[4,3-j][1,2]benzodioxepine.

The kit may include UM171 or a derivative thereof. The UM171 or derivative thereof is as described above.

The culture medium suitable for maintenance culture of hematopoietic stem cells is not limited as long as the culture medium is the above-described culture medium.

The kit may include an antibody for isolating hematopoietic stem cells and/or a reagent for sorting cells. The antibody for isolating hematopoietic stem cells can be an antibody for any of the above-mentioned markers, and it is preferable that the antibody for isolating hematopoietic stem cells be labeled with a fluorescent protein or the like. As the reagent for sorting cells, for example, a labeled secondary antibody for use in MACS or FACS or a column for separation is included.

The kit may include (1) a culture medium suitable for maintenance culture of hematopoietic stem cells and (2) the compound represented by the formula (1) or a salt thereof, as a single composition, or as individual compositions.

In an embodiment, the composition containing (2) the compound represented by the formula (1) or a salt thereof can be provided as a culture auxiliary composition for maintenance culture of hematopoietic stem cells separately from (1) the culture medium suitable for maintenance culture of hematopoietic stem cells.

### [Transplantation]

The cell population including hematopoietic stem cells produced by the above-described method or the like can be transplanted into a subject in need of transplantation (for example, a mammal), and the hematopoietic stem cells after being transplanted can be engrafted and produce blood in the bone marrow of the subject (recipient). Examples of the candidate mammal for a subject include a human, a mouse, a rat, a guinea pig, a hamster, a rabbit, a cat, a dog, a sheep, a pig, a bovine, a horse, a goat, and a monkey. The cell population including hematopoietic stem cells is preferably formulated into a pharmaceutical composition in advance of transplantation.

Transplantation of the cell population including hematopoietic stem cells is performed, for example, by intravenous administration (e.g., intravenous infusion).

In the case of transplantation for treatment of blood cancer such as leukemia, pretransplantation treatment such as treatment with an anticancer agent, CAR-T cell therapy, or systemic radiation in combination with an immunosuppressor is occasionally performed for the purpose of destructing cancer cells remaining in the body as much as possible and lowering the immunocompetence of the patient him- or herself to facilitate engraftment of transplanted cells.

### [Pharmaceutical Composition]

The present invention provides, in an aspect, a pharmaceutical composition comprising: an effective amount of a cell population of hematopoietic stem cells produced by the above production method. The effective amount of a cell population for transplantation depends on the purpose of administration, method of administration, and situation of a subject of administration (sex, age, body weight, disease condition, etc.), and can be, for example, 1 × 10⁷ to 1 × 10¹⁰ cells/kg in terms of cell count per patient body weight.

The pharmaceutical composition in an embodiment comprises: an effective amount of the cell population for transplantation produced by the above production method; and a pharmaceutically acceptable carrier.

A physiological aqueous solvent (physiological saline, buffer, serum-free culture medium, etc.) can be used for the pharmaceutically acceptable carrier. As necessary, the pharmaceutical composition may contain, for example, a preservative, stabilizer, reducing agent, or isotonic agent that is commonly used as an accessory component for drugs containing tissue or cells to be transplanted in transplantation therapy. Examples thereof include, but are not limited to, dimethyl sulfoxide, dextran, human serum albumin, acetyltryptophane sodium, sodium chloride, potassium chloride, calcium chloride hydrate, magnesium chloride, sodium hydrogen carbonate, sodium citrate hydrate, and carbon dioxide.

The pharmaceutical composition in an embodiment in the present specification can be formulated into a cell suspension and intravenously administered (intravenous infusion). Provided as an embodiment is an infusion bag including a cell population of hematopoietic stem cells. The infusion bag may include any of the above-mentioned accessory components. For example, the bag can be used after dilution with physiological saline or the like.

The pharmaceutical composition in an embodiment in the present specification is provided in a frozen state. For example, the pharmaceutical composition can be used after thawing in a water bath at 37°C. Dilution with physiological saline or the like may be performed after thawing.

The present invention provides, in an aspect, a pharmaceutical composition for treatment of a disease for which regeneration or augmentation of hematopoietic stem cells is required, specifically, blood cancer such as leukemia. The pharmaceutical composition in an embodiment comprises: an effective amount of the compound of the present invention; and a pharmaceutically acceptable carrier.

### [Therapeutic Drug and Therapeutic Method]

The cell population of hematopoietic stem cells produced by the production method of the present invention is useful for transplantation therapy to supplement hematopoietic stem cells for patients with loss of functions of hematopoietic stem cells because of blood cancer or the like. Accordingly, the present invention provides, in an aspect, a therapeutic drug (for example, a therapeutic drug for blood cancer) for supplementing hematopoietic stem cells, comprising a hematopoietic stem cell produced by the production method of the present invention. A patient who is affected by blood cancer, aplastic anemia, congenital hematopoietic disorder, immunodeficiency syndrome such as acquired immunodeficiency syndrome (AIDS), agammaglobulinemia, myelopathic thrombocytopenia, idiopathic thrombocytopenic purpura (ITP), congenital anemia such as sickle cell disease, or a like disease and in need of transplantation can be treated by transplanting the cell population including hematopoietic stem cells produced by the production method of the present invention to allow the hematopoietic stem cells to produce new blood. Examples of blood cancer include leukemia, malignant lymphoma, and multiple myeloma. Examples of leukemia include acute myeloid leukemia, acute lymphoid leukemia, chronic myeloid leukemia, myelodysplastic syndrome, Adult T-cell Leukemia (ATL), and chronic lymphoid leukemia. Examples of malignant lymphoma include follicular lymphoma, aggressive lymphoma, and Hodgkin's lymphoma.

The present invention provides, in an aspect, a method for treating a disease for which regeneration or augmentation of hematopoietic stem cells is required, specifically, blood cancer such as leukemia, comprising: administering the compound of the present invention itself to a subject.

The present invention will be more specifically described with reference to Reference Examples, Examples, and Test Examples in the following, but, needless to say, the present invention is not limited by them. Compound names shown in Reference Examples and Examples are given by using ACD/Labs 2016.2.2, and are not necessarily according to the IUPAC nomenclature.

For simplification of descriptions in the present specification, abbreviations as shown below are occasionally used in Reference Examples, Examples, and tables in Examples. Regarding abbreviations used for substituents, Me stands for methyl, CN for cyano, Bz for benzoyl, TMS for trimethylsilyl, N₃ for azide, and tert for tertiary. Regarding symbols used for NMR, s stands for a singlet, d for a doublet, t for a triplet, m for a multiplet, and J for a coupling constant. Regarding symbols used for units, M stands for molarity.

Hereinafter, the present invention will be described in detail with reference to Examples; however, the present invention is not limited by them at all.

### Examples

### Example 1-17

### 1-[(3R,5aS,6R,8aS,9R,10S,12R,12aR)-3,6,9-Trimethyldecahydro-12H-3,12-epoxypyrano[4,3-j][1,2]benzodioxepin-10-yl]methanamine

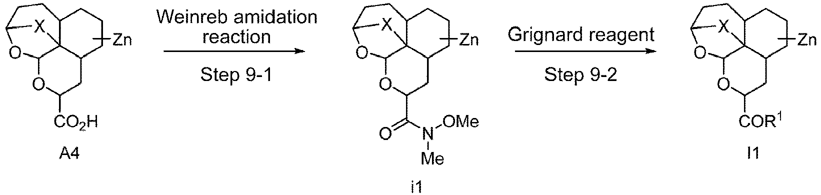

a) Production of (3R,5aS,6R,8aS,9R,10R,12R,12aR)-3,6,9-trimethyldecahydro-12H-3,12-epoxypyrano[4,3-j][1,2]benzodioxepin-10-yl benzoate (compound Q2)
   A dichloromethane (5 mL) of commercially available dihydroartemisinin (Q1, Adamas Reagent Ltd. (catalog code: 01108787), 284 mg), trichloroacetonitrile (156 mg), and diazabicycloundecene (7.58 mg) was stirred at room temperature overnight. A dichloromethane solution (5 mL) of benzoic acid (365 mg) was added to the reaction solution, which was stirred at room temperature for 2 hours. After the completion of the reaction, saturated sodium bicarbonate water (10 mL) and water (10 mL) were added, and the organic layer was dried over sodium sulfate. The solvent was distilled off under reduced pressure to afford compound Q2 (250 mg).
   1H NMR (400 MHz, CDCl₃): δ 8.03-8.01 (2H, m), 7.61 (1H, d, J = 7.4 Hz), 7.49 (2H, d, J = 4.6 Hz), 6.53 (1H, d, J = 3.2 Hz), 5.59 (1H, s), 2.98-2.94 (1H, m), 2.46-2.38 (1H, m), 2.10-1.89 (4H, m), 1.83-1.79 (1H, m), 1.68-1.64 (1H, m), 1.64-1.49 (5H, m), 1.45-1.35 (1H, m), 1.08-1.02 (4H, m), 0.98 (3H, d, J = 7.6 Hz).
b) Production of (3R,5aS,6R,8aS,9R,10S,12R,12aR)-3,6,9-trimethyldecahydro-12H-3,12-epoxypyrano[4,3-j][1,2]benzodioxepine-10-carbonitrile (compound Q3)
   A dichloromethane solution (2 mL) of compound Q2 (100 mg) obtained in the previous experiment and trimethylsilyl cyanide (76.4 mg) was stirred at -78°C for 5 minutes. Thereafter, tin tetrachloride (26.7 mg) was added, and the resultant was stirred at -78°C for 2 hours. The reaction solvent was distilled off under reduced pressure, and the resulting residue was purified by silica gel column chromatography (elution solvent; pentane:ethyl acetate) to afford compound Q3 (40 mg). 1H NMR (400 MHz, CDCl₃): δ 5.55 (1H, s), 4.79 (1H, d, J = 6.0 Hz), 2.93-2.88 (1H, m), 2.43-2.35 (1H, m), 2.12-2.06 (1H, m), 2.01-1.91 (2H, m), 1.87-1.76 (2H, m), 1.67-1.62 (1H, m), 1.56-1.43 (5H, m), 1.42-1.41 (1H, m), 1.09 (3H, d, J = 7.2 Hz), 1.10-0.99 (4H, m).
c) Production of 1-[(3R,5aS,6R,8aS,9R,10S,12R,12aR)-3,6,9-trimethyldecahydro-12H-3,12-epoxypyrano[4,3-j][1,2]benzodioxepin-10-yl]methanamine (Example 1-17)

A tetrahydrofuran solution (4 mL) of compound Q3 (100 mg), sodium borohydride (64.6 mg), and boron trifluoride-diethyl ether complex (115 mg) was stirred at 65°C for 2 hours. After the completion of the reaction, liquid separation and extraction were performed with dichloromethane-sodium bicarbonate water. The organic layer was washed with brine, dried over sodium sulfate, and then subjected to distillation under reduced pressure. A tetrahydrofuran solution (3 mL) of the resulting residue, di-tert-butyl dicarbonate (110 mg), and triethylamine (101 mg) was stirred at room temperature for 2 hours. After the completion of the reaction, the reaction solvent was distilled off under reduced pressure, and purification by silica gel column chromatography (elution solvent; pentane: ethyl acetate) was performed to afford a product (45 mg). A dichloromethane solution (2 mL) of the product (25 mg) and 3 M hydrochloric acid-ethyl acetate (1 mL) was stirred at room temperature for 2 hours. The reaction solvent was distilled off to afford the compound of Example 1-17 (10 mg) as a hydrochloride thereof.
1H NMR (400 MHz, CDCl₃): δ 8.36 (3H, s), 5.56-5.36 (1H, m), 4.65-4.44 (1H, m), 3.26 (2H, s), 3.01-2.78 (1H, m), 2.43-2.11 (1H, m), 2.02-1.91 (2H, m), 1.78-1.76(1H, m), 1.66 (3H, s), 1.48 (3H, s), 1.30-1.19 (3H, m), 0.98-0.87 (7H, m).

### Examples 1-1 and 1-28

### (3R,5aS,6R,8aS,9R,10R,12S,12aR)-10-Fluoro-3,6,9-trimethyldecahydro-12H-3,12-epoxypyrano[4,3-j][1,2]benzodioxepine (Example 1-1) and (3R,5aS,6R,8aS,12R,12aR)-3,6,9-trimethyl-3,4,5,5a,6,7,8,8a-octahydro-12H-3,12-epoxypyrano[4,3-j][1,2]benzodioxepine (Example 1-28)

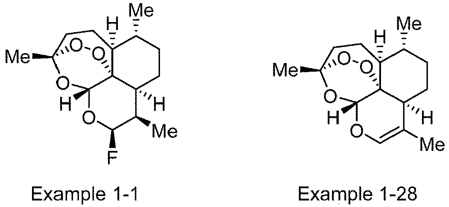

To a dichloromethane solution (24 mL) of Q1 (1.136 g), N,N-diethylaminosulfur trifluoride (0.6 mL) was added dropwise at 0°C, and the resultant was stirred at room temperature for 16 hours. After the completion of the reaction, the resultant was cooled to 0°C, 5% aqueous sodium carbonate solution (20 mL) was added, and the resultant was stirred at room temperature for 2 hours. The organic layer was washed with 1 M aqueous hydrochloric acid solution, sodium bicarbonate water, and water, and then dried over sodium sulfate. The solvent was distilled off under reduced pressure, and the resulting residue was then purified by silica gel column chromatography (elution solvent; pentane: ethyl acetate) to afford the compound of Example 1-1 (283 mg) and the compound of Example 1-28 (59 mg).

### Example 1-1

1H NMR (400 MHz, CDCl₃): 5.60 (1H, d, J = 54.4, 2.0 Hz), 5.56 (1H, d, J = 1.6 Hz), 2.72-2.56 (1H, m), 2.38 (1H, m), 2.09-2.02 (1H, m), 1.95-1.81 (2H, m), 1.72-1.65 (1H, m), 1.62-1.58 (1H, m), 1.55-1.50 (2H, m), 1.50-1.45 (1H, m), 1.43 (3H, s), 1.40-1.32 (1H, m), 1.32-1.23 (1H, m), 0.99 (3H, d, J = 7.6 Hz), 0.94 (3H, d, J = 6.0 Hz).

### Example 1-28

1H NMR (400 MHz, CDCl₃): δ 6.19 (1H, d, J = 1.6 Hz), 5.54 (1H, s), 2.40 (1H, m), 2.10-2.00 (2H, m), 1.95-1.86 (1H, m), 1.73-1.62 (2H, m), 1.59 (3H, s), 1.54-1.51 (1H, m), 1.48-1.38 (5H, m), 1.26-1.03 (2H, m), 0.98 (3H, d, J = 5.6 Hz).

### Example 1-7

### (3R,5aS,6R,8aS,9R,10R,12R,12aR)-10-(Furan-2-yl)-3,6,9-trimethyldecahydro-12H-3,12-epoxypyrano[4,3-j][1,2]benzodioxepine

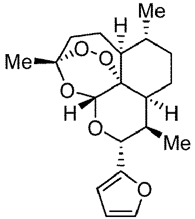

To a dichloromethane solution (10 mL) of the compound of Example 1-1 (285 mg) obtained in the previous experiment and furan (338 mg), boron trifluoride-diethyl ether complex (16.8 mg) was added under a nitrogen atmosphere at -78°C, and the resultant was stirred at - 50°C for 5 hours. After the completion of the reaction, liquid separation and extraction were performed with dichloromethane-sodium bicarbonate water. The solvent of the organic layer was distilled off under reduced pressure, and the resulting residue was purified by reversed-phase column chromatography (elution solvent; acetonitrile:water (with formic acid)) to afford the compound of Example 1-7 (33 mg).

1H NMR (500 MHz, CDCl₃): δ 7.39 (1H, s), 6.33-6.31 (1H, m), 5.38 (1H, s), 4.46 (1H, d, J = 10.5Hz), 2.90-2.82 (1H, m), 2.39(1H, m), 2.08-2.01 (1H, m), 1.93-1.83 (1H, m), 1.77-1.71 (2H, m), 1.66-1.60 (1H, m), 1.53-1.47 (2H, m), 1.42-1.34 (4H, m), 1.33-1.24 (1H, m), 1.11-1.02 (1H, m), 0.98 (4H, d, J = 5.5 Hz), 0.93 (3H, d, J = 7.5 Hz).

### Example 1-8

### (3R,5aS,6R,8aS,9R,10R,12R,12aR)-3,6,9-Trimethyldecahydro-12H-3,12-epoxypyrano[4,3-j][1,2]benzodioxepine-10-carboxylic acid

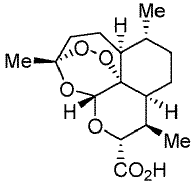

Example 1-7 (90 mg) was dissolved in a mixture of acetonitrile (1 mL), carbon tetrachloride (1 mL), and water (1 mL). Sodium periodate (286 mg) was added, rhodium(III) chloride trihydrate (7 mg) was subsequently added, and the resultant was stirred at room temperature for 3 hours. After the completion of the reaction, liquid separation and extraction were performed with dichloromethane-sodium bicarbonate water, and the solvent of the organic layer was distilled off under reduced pressure. The resulting residue was purified by reversed-phase column chromatography (elution solvent; acetonitrile:water (with formic acid)) to afford the compound of Example 1-8 (35 mg).
1H NMR (500 MHz, CDCl₃): δ 5.37 (1H, s), 4.06 (1H, d, J = 11.5 Hz), 2.61-2.53 (1H, m), 2.39 (1H, m), 2.08-2.02 (1H, m), 1.95- 1.88 (1H, m), 1.80-1.73 (2H, m), 1.64-1.37 (1H, m), 1.50-1.44 (1H, m), 1.43 (3H, s), 1.39-1.25 (3H, m), 1.10-1.02 (1H, m), 0.98 (3H, d, J = 1.5 Hz), 0.97 (3H, s).

Example 1-6

### (3R,5aS,6R,8aS,9R,10R,12R,12aR)-3,6,9,10-Tetramethyldecahydro-12H-3,12-epoxypyrano[4,3-j][1,2]benzodioxepine

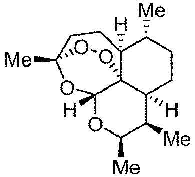

The compound of Example 1-6 (11 mg) was produced from the compound of Example 1-1 (70 mg) by using a method described in Tetrahedron Lett. 1998, 39, 1533-1536.

1H NMR (400 MHz, CDCl₃): δ 5.36 (1H, s), 4.36-4.33 (1H, m), 2.73-2.66 (1H, m), 2.34 (1H, m), 2.06-2.01 (1H, m), 1.93-1.78 (2H, m), 1.70-1.62 (1H, m), 1.60-1.51 (1H, m), 1.51-1.45 (1H, m), 1.45-1.39 (4H, m), 1.36-1.28 (1H, m), 1.26-1.22 (4H, m), 0.99-0.92 (4H, m), 0.86 (3H, d, J = 7.6 Hz).

Example 1-2

### (3R,5aS,6R,8aS,9R, 10S,12R, 12aR)-3,6,9-Trimethyl-10-phenyldecahydro-12H-3,12-epoxypyrano[4,3-j][1,2]benzodioxepine

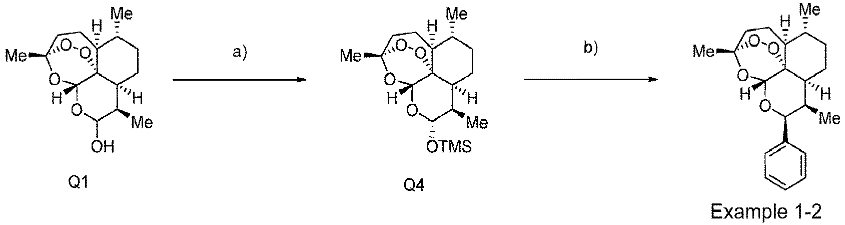

a) Production of trimethyl{[(3R,5aS,6R,8aS,9R,10S,12R,12aR)-3,6,9-trimethyldecahydro-12H-3,12-epoxypyrano[4,3-j][1,2]benzodioxepin-10-yl]oxy}silane (compound Q4)
   To a dichloromethane solution (4 mL) of Q1 (300 mg) and triethylamine (132 mg), chlorotrimethylsilane (142 mg) was added at 0°C, and the resultant was stirred at 0°C for 1.5 hours. After the completion of the reaction, iced water (5 g) was added, the aqueous layer was subjected to extraction with dichloromethane, and the organic layer was dried over sodium sulfate. The solvent was distilled off under reduced pressure, and the resulting residue was then purified by silica gel column chromatography (elution solvent; pentane: ethyl acetate) to afford compound Q4 (70 mg).
   1H NMR (400 MHz, CDCl₃): δ 5.34 (1H, s), 4.78 (1H, d, J = 9.2 Hz), 2.42-2.31(2H, m), 2.06-2.01 (1H, m), 1.93-1.86 (1H, m), 1.79-1.67 (2H, m), 1.57-1.47 (2H, m), 1.44 (3H, s), 1.40-1.30 (3H, m), 1.05-0.95 (4H, m), 0.88 (3H, d, J = 7.2 Hz), 0.21 (9H, s).
b) Production of (3R,5aS,6R,8aS,9R,10S,12R,12aR)-3,6,9-trimethyl-10-phenyldecahydro-12H-3,12-epoxypyrano[4,3-j][1,2]benzodioxepine (Example 1-2)

The compound of Example 1-2 (24.2 mg) was produced from compound Q4 (200 mg) by using a method described in Eur. J. Org. Chem. 2003,2098-2114.
1H NMR (400 MHz, CDCl₃): δ 7.34-7.31 (4H, m), 7.27-7.22 (1H, m), 5.74 (1H, d, J = 6.8 Hz), 5.60 (1H, s), 2.80-2.74 (1H, m), 2.40-2.32 (1H, m), 2.11-2.00 (2H, m), 1.91-1.85 (1H, m), 1.78-1.67 (2H, m), 1.47-1.43 (1H, m), 1.41 (3H, s), 1.37-1.26(4H, m), 1.01 (3H, d, J = 6.0 Hz), 0.53 (3H, d, J = 7.6 Hz).

### Example 1-3 and Example 1-4

### (3R,5aS,6R,8aS,9R,10S,12S,12aR)-3,6,9-Trimethyl-10-(methylsulfanyl)decahydro-12H-3,12-epoxypyrano[4,3-j][1,2]benzodioxepine (Example 1-3) and (3R,5aS,6R,8aS,9R,10R,12S,12aR)-3,6,9-trimethyl-10-(methylsulfanyl)decahydro-12H-3,12-epoxypyrano[4,3-j][1,2]benzodioxepine (Example 1-4)

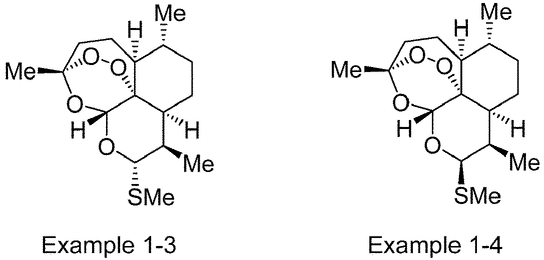

The compound of Example 1-3 (50 mg) and the compound of Example 1-4 (6.8 mg) were produced from Q1 (100 mg) by using a method described in J. Org. Chem. 2004, 69, 984-986.

### Example 1-3

1H NMR (400 MHz, CDCl₃): δ 5.34 (1H, s), 4.48 (1H, d, J = 5.2 Hz), 2.65-2.60 (1H, m), 2.43-2.35 (1H, m), 2.21 (3H, s), 2.07-2.01 (1H, m), 1.94-1.87 (1H, m), 1.78-1.71 (2H, m), 1.64-1.62 (1H, m), 1.56-1.48 (1H, m), 1.45 (3H, s), 1.42-1.33 (2H, m), 1.31-1.23 (1H, m), 1.07-1.03 (1H, m), 0.99-0.94 (6H, m).

### Example 1-4

1H NMR (400 MHz, CDCl₃): δ 5.60 (1H, s), 5.19 (1H, d, J = 5.2 Hz), 3.07-3.03 (1H, m), 2.42-2.34 (1H, m), 2.21 (3H, s), 2.08-2.02 (1H, m), 1.92-1.81 (2H, m), 1.74-1.65 (2H, m), 1.54-1.44 (2H, m), 1.40 (3H, s), 1.39-1.35 (1H, m), 1.26-1.25 (1H, m), 0.98-0.92 (7H, m).

### Example 1-5

### (3R,5aS,6R,8aS,9R,10S,12S,12aR)-10-(Methanesulfonyl)-3,6,9-trimethyldecahydro-12H-3,12-epoxypyrano[4,3-j][1,2]benzodioxepine

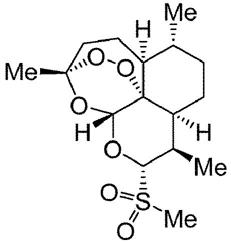

The compound of Example 1-5 (18.5 mg) was produced from the compound of Example 1-3 (55 mg) by using a method described in J. Org. Chem. 2004, 69, 984-986.
1H NMR (400 MHz, CDCl₃): δ 5.40 (1H, s), 4.35 (1H, d, J = 11.2 Hz), 2.96 (3H, s), 2.87-2.82 (1H, m), 2.42-2.34 (1H, m), 2.06-2.01 (1H, m), 1.95-1.88 (1H, m), 1.84-1.73 (2H, m), 1.50-1.40 (4H, m), 1.37-1.26 (4H, m), 1.14 (3H, d, J = 7.2 Hz), 1.10-1.03 (1H, m), 0.97 (3H, d, J = 6.0 Hz).

### Example 1-9

### 4-Phenyl-1-[(3R,5aS,6R,8aS,9R,10R,12R,12aR)-3,6,9-trimethyldecahydro-12H-3,12-epoxypyrano[4,3-j][1,2]benzodioxepin-10-yl]-1H-1,2,3-triazole

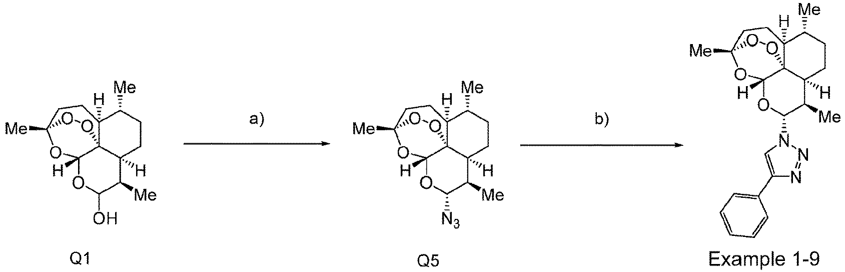

a) Production of (3R,5aS,6R,8aS,9R,10R,12R,12aR)-10-azido-3,6,9-trimethyldecahydro-12H-3,12-epoxypyrano[4,3-j][1,2]benzodioxepine (compound Q5)
   Compound Q5 (30 mg) was produced from Q1 (100 mg) by using a method described in Bioorg. Med. Chem. Lett. 2009, 19, 382-385. 1H NMR (400 MHz, CDCl₃): δ 5.41 (1H, s), 4.63 (1H, d, J = 10.0 Hz), 2.37-2.47 (2H, m), 2.03-2.09 (1H, m), 1.89-1.95 (1H, m), 1.71-1.81 (2H, m), 1.48-1.57 (1H, m), 1.47 (3H, s), 1.25-1.37 (4H, m), 0.89-1.09 (1H, m), 0.86 (3H, d, J = 4.0 Hz), 0.86 (3H, d, J = 2.4 Hz).
b) Production of 4-phenyl-1-[(3R,5aS,6R,8aS,9R,10R,12R,12aR)-3,6,9-trimethyldecahydro-12H-3,12-epoxypyrano[4,3j][1,2]benzodioxepin-10-yl]-1H-1,2,3-triazole

### (Example 1-9)

The compound of Example 1-9 (20 mg) was produced from compound Q5 (90 mg) by using a method described in Bioorg. Med. Chem. Lett. 2009, 19, 382-385.

1H NMR (400 MHz, CDCl₃): δ 7.98 (1H, s), 7.86-7.84 (2H, m), 7.45-7.41 (2H, m), 7.36-7.32 (1H, m), 5.89 (1H, d, J = 10.4 Hz), 5.49 (1H, s), 3.63-3.60 (1H, m), 2.97-2.92 (1H, m), 2.14-1.88 (5H, m), 1.67-1.60 (4H, m), 1.35-1.15 (4H, m), 0.94 (3H, d, J=6.4 Hz), 0.85 (3H, d, J = 7.2 Hz).

### Example 1-10

### N-[(3R,5aS,6R,8aS,9R,10R,12R,12aR)-3,6,9-Trimethyldecahydro-12H-3,12-epoxypyrano[4,3-j][1,2]benzodioxepin-10-yl]acetamide

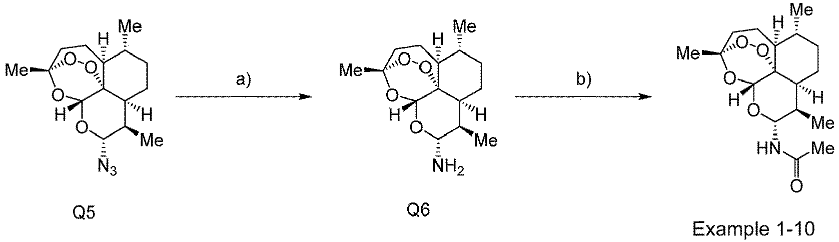

a) Production of (3R,5aS,6R,8aS,9R,10R,12R,12aR)-3,6,9-trimethyldecahydro-12H-3,12-epoxypyrano[4,3-j][1,2]benzodioxepin-10-amine (compound Q6)
   Compound Q6 (87 mg) was produced from compound Q5 (100 mg) by using a method described in Bioorg. Chem. 2016, 66, 63-71. 1H-NMR (400 MHz, CDCl₃) δ: 5.33 (1H, s), 4.20 (1H, d, J = 9.7Hz), 1.94-1.84 (2H, m), 1.75 (3H, m), 1.52 (2H, m), 1.46-1.41 (4H, m), 1.31-1.22 (4H, m), 0.98-0.90 (6H, m).
b) Production of N-[(3R,5aS,6R,8aS,9R,10R,12R,12aR)-3,6,9-trimethyldecahydro-12H-3,12-epoxypyrano[4,3-j][1,2]benzodioxepin-10-yl]acetamide (Example 1-10)

A dichloromethane solution (5.0 mL) of compound Q6 (87 mg), acetic anhydride (15.6 mg), and 4-dimethylaminopyridine (44.9 mg) was stirred at room temperature for 15 hours. After the completion of the reaction, liquid separation and extraction were performed with dichloromethane-5% hydrochloric acid water, and the organic layer was dried over sodium sulfate, and then subjected to distillation under reduced pressure. The resulting residue was purified by silica gel column chromatography (elution solvent; dichloromethane:methanol) to afford the compound of Example 1-10 (43 mg).
1H-NMR (500 MHz, CDCl₃) δ:6.01 (1H, d, J = 9.7 Hz), 5.42 (1H, s), 5.34 (1H, t, J = 10.2 Hz), 2.42-2.34 (2H, m), 2.03 (3H, s), 1.92-1.88 (1H, m), 1.80-1.77 (2H, m), 1.72 (1H, d, J = 3.3 Hz), 1.57-1.52 (1H, m), 1.44 (3H, s), 1.29 (1H, d, J = 4.2 Hz), 1.28-1.25 (2H, m), 1.13-1.04 (1H, m), 1.02 (1H, d, J = 12.2 Hz), 0.97 (3H, d, J = 6.3 Hz), 0.87 (3H, d, J = 7.2 Hz).

### Example 1-11

### N-[(3R,5aS,6R,8aS,9R,10R,12R,12aR)-3,6,9-Trimethyldecahydro-12H-3,12-epoxypyrano[4,3-j][1,2]benzodioxepin-10-yl]methanesulfonamide

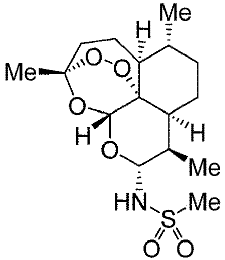

To a dichloromethane solution of compound Q6 (20 mg), methanesulfonyl chloride (24.1 mg) and triethylamine (21.3 mg) were added at 0°C, and the resultant was stirred at room temperature for 17 hours. After the completion of the reaction, liquid separation and extraction were performed with dichloromethane-sodium bicarbonate water, and the organic layer was washed with 1 M aqueous hydrochloric acid solution, dried over sodium sulfate, and then subjected to distillation under reduced pressure. The resulting residue was purified by reversed-phase column chromatography (elution solvent; acetonitrile: water (with formic acid)) to afford the compound of Example 1-11 (14 mg).
1H-NMR (400 MHz, CDCl₃): δ 5.37 (1H, s), 5.05 (1H, d, J = 10.5 Hz), 4.78 (1H, t, J = 10.4 Hz), 3.18 (3H, s), 2.44-2.33 (2H, m), 2.08-2.01 (1H, m), 1.95-1.88 (1H, m), 1.78-1.73 (2H, m), 1.53-1.49 (2H, m), 1.42 (3H, s), 1.39-1.28 (3H, m), 1.02 (1H, d, J = 14.9 Hz), 0.96 (6H, m).

### Example 1-13

### 4-[(3R,5aS,6R,8aS,9R,10R,12R,12aR)-3,6,9-Trimethyldecahydro-12H-3,12-epoxypyrano[4,3-j][1,2]benzodioxepin-10-yl]morpholine

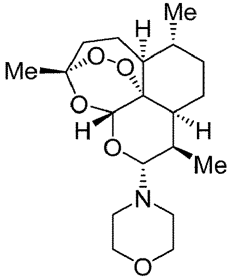

The compound of Example 1-13 (24 mg) was obtained from compound Q4 (150 mg) by using a method described in Angew. Chem. Int. Ed. 2006, 45, 2082-2088.

1H NMR (400 MHz, CDCl₃): δ 5.30 (1H, s), 4.00 (1H, d, J = 10.0 Hz), 3.76-3.66 (4H, m), 3.03-2.98 (2H, m), 2.71-2.66 (2H, m), 2.63-2.57 (1H, m), 2.41-2.33 (1H, m), 2.06-2.00 (1H, m), 1.92-1.85 (1H, m), 1.77-1.69 (2H, m), 1.58-1.48 (2H, m), 1.42 (3H, s), 1.40-1.32 (2H, m), 1.26-1.22 (1H, m), 1.08-1.01 (1H, m), 0.97 (3H, d, J = 6.0 Hz), 0.84 (3H, d, J = 7.6 Hz).

### Example 1-12

### (3R,5aS,6R,8aS,9R, 10R, 12R,12aR)-N,N,3,6,9-Pentamethyldecahydro-12H-3,12-epoxypyrano[4,3-j][1,2]benzodioxepin-10-amine

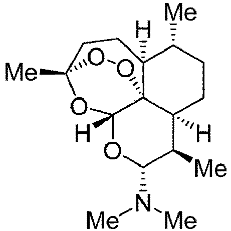

The compound of Example 1-12 was obtained by performing reaction and treatment with use of the corresponding raw material compound in the same manner as in Example 1-13.
¹H-NMR (400 MHz, CDCl₃): δ 5.31 (1 H, s), 4.05 (1H, d, J = 10.2 Hz), 2.56-2.51 (1H, m), 2.42 (6H, s), 2.40-2.33 (1H, m), 2.06-2.00 (1H, m), 1.91-1.88 (1H, m), 1.76-1.76 (1H, m), 1.57-1.54 (2H, m), 1.42(3H, d, *J* = 8.8 Hz), 1.42-1.37 (1H, m), 1.27-1.21 (2H, m), 1.09 (1H, m), 1.04-0.98 (1H, m), 0.95 (3H, d, J = 6.3 Hz), 0.82 (3H, d, J = 7.2 Hz).

### Example 1-14

### (3R,5aS,6R,8aS,9R,10S,12R,12aR)-3,6,9-Trimethyldecahydro-12H-3,12-epoxypyrano[4,3-j][1,2]benzodioxepin-10-yl acetate

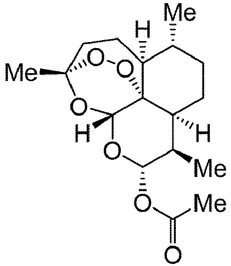

The compound of Example 1-14 (28 mg) was produced from Q1 (100 mg) by using a method described in Eur. J. Org. Chem. 2002, 113-132.

1H NMR (400 MHz, CDCl₃): δ 5.81 (1H, d, J = 10.0 Hz), 5.47 (1H, s), 2.62-2.56 (1H, m), 2.44-2.36 (1H, m), 2.15 (3H, s), 2.09-2.03 (1H, m), 1.95-1.88 (1H, m), 1.83-1.72 (2H, m), 1.68-1.62 (1H, m), 1.56-1.48 (1H, m), 1.46 (3H, s), 1.44-1.40 (1H, m), 1.34-1.27 (2H, m), 1.09-1.02 (1H, m), 0.99 (3H, d, J = 6.0 Hz), 0.88 (3H, d, J = 6.8 Hz).

### Example 1-15

### (3R,5aS,6R,8aS,9R,10S,12R,12aR)-3,6,9-Trimethyldecahydro-12H-3,12-epoxypyrano[4,3-j][1,2]benzodioxepine-10-carboxamide

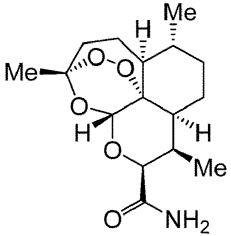

The compound of Example 1-15 (50 mg) was obtained from compound Q3 (100 mg) by using a method described in Chem Med Chem 2016, 11, 1469-1479.
1H NMR (400 MHz, CDCl₃): δ 6.58 (1H, s), 5.50-5.34 (2H, m), 4.83 (1H, d, J = 6.4 Hz), 2.94-2.88 (1H, m), 2.41-2.33 (1H, m), 2.09-1.95 (2H, m), 1.87-1.67 (3H, m), 1.43 (3H, s), 1.32-1.22 (4H, m), 1.13 (3H, d, J = 7.6 Hz), 1.00-0.97 (4H, m).

### Example 1-16

### (3R,5aS,6R,8aS,9R,10R,12R,12aR)-3,6,9-Trimethyldecahydro-12H-3,12-epoxypyrano[4,3-j][1,2]benzodioxepine-10-carboxamide

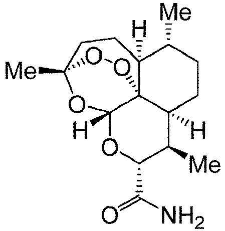

The compound of Example 1-16 (10 mg) was obtained from compound Q3 (30 mg) by using a method described in Chem Med Chem 2016, 11, 1469-1479.
1H NMR (400 MHz, CDCl₃): δ 6.58 (1H, s), 5.35-5.34 (2H, m), 3.93 (1 H, d, J = 11.2 Hz), 2.56-2.50 (1H, m), 2.43-2.37 (1H, m), 2.09-2.04 (1H, m), 1.97-1.90 (1H, m), 1.81-1.74 (2H, m), 1.56-1.48 (2H, m), 1.45 (3H, s), 1.41-1.28 (3H, m), 1.12-1.05 (1H, m), 1.00-0.97 (6H, m).

### Example 1-18

### 1-[(3R,5aS,6R,8aS,9R,10R,12R,12aR)-3,6,9-Trimethyldecahydro-12H-3,12-epoxypyrano[4,3-j][1,2]benzodioxepin-10-yl]methanamine

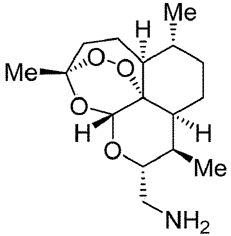

A toluene solution (8 mL) of the compound of Example 1-16 (400 mg), triruthenium dodecacarbonyl (24.5 mg) and 1,1,3,3-tetramethyldisiloxane (686 mg) was stirred at 50°C overnight. After the completion of the reaction, water was added, and extraction was performed with dichloromethane. The organic layer was dried over sodium sulfate, and then subjected to distillation under reduced pressure. A dichloromethane solution (8 mL) of the resulting residue, di-tert-butyl dicarbonate (766 mg), and triethylamine (590 mg) was stirred at room temperature for 5 hours. After the completion of the reaction, the reaction solvent was distilled off under reduced pressure, and purification by silica gel column chromatography (elution solvent; pentane:ethyl acetate) was performed to afford a product (150 mg). A dichloromethane solution (2.5 mL) of the product (75 mg) and hydrochloric acid-ethyl acetate (1.5 mL) was stirred at room temperature for 5 hours. The reaction solvent was distilled off to afford the compound of Example 1-18 (28.6 mg) as a hydrochloride thereof.
1H NMR (400 MHz, CDCl₃): δ 8.31 (3H, s), 5.37-5.32 (1H, m), 3.98-3.96 (1H, m), 3.35-3.34 (1H, m), 3.08-3.09 (1H, m), 2.40-2.33 (2H, m), 2.10-2.01 (2H, m), 1.74-1.68 (2H, m), 1.56-1.37 (7H, m), 1.30-1.26 (1H, m), 1.04-1.02 (1H, m), 0.97 (3H, m), 0.90-0.85 (3H, m).

### Example 1-19

### (3R,5aS,6R,8aS,9R,10S,12R,12aR)-3,6,9-Trimethyl-10-[(propan-2-yl)oxy]decahydro-12H-3,12-epoxypyrano[4,3-j][1,2]benzodioxepine

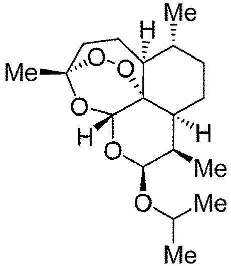

To a dichloromethane solution (1 mL) of Q1 (100 mg) and isopropanol (42.1 mg), boron trifluoride-diethyl ether complex (99.6 mg) was added at 15°C, and the resultant was stirred at 15°C for 16 hours. The reaction solution was purified by silica gel column chromatography (elution solvent; pentane: ethyl acetate) to afford the compound of Example 1-19 (22 mg).

1H NMR (400 MHz, CDCl₃): 5.46 (1H, s), 4.90 (1H, d, J = 3.2 Hz), 4.03-3.97 (1H, m), 2.64-2.60 (1H, m), 2.43-2.35 (1H, m), 2.08-2.03 (1H, m), 1.93-1.82 (2H, m), 1.78-1.72 (1H, m), 1.68-1.62 (1H, m), 1.55-1.51 (1H, m), 1.46 (3H, s), 1.39-1.32 (1H, m), 1.30-1.26 (1H, m), 1.23-1.16 (4H, m), 1.10 (3H, d, J = 6.4 Hz), 0.98-0.93 (4H, m), 0.90 (3H, d, J = 7.2 Hz).

### Examples 1-20, 1-23, and 1-24

Compounds shown in Table 1 were each obtained by performing reaction and treatment with use of the corresponding raw material compound in the same manner as in Example 1-19.

**[Table 1]**

| 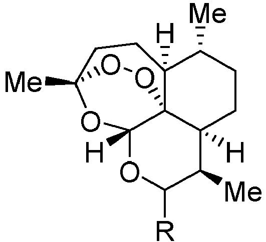 | | |
|---|---|---|
| Example | R | Compound name |
| 1-20 | 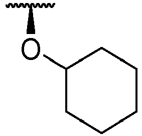 | (3R,5aS,6R,8aS,9R,10S,12R,12aR)-10-(cyclohexyloxy)-3,6,9-trimethyldecahydro-12H-3,12-epoxypyrano[4,3-j][1,2]benzodioxepine |
| 1-23 | 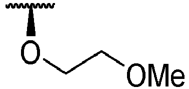 | (3R,5aS,6R,8aS,9R,10S,12R,12aR)-10-(2-methoxyethoxy)-3,6,9-trimethyldecahydro-12H-3,12-epoxypyrano[4,3-j][1,2]benzodioxepine |
| 1-24 | 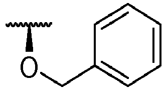 | (3R,5aS,6R,8aS,9R,10S,12R,12aR)-10-(benzyloxy)-3,6,9-trimethyldecahydro-12H-3,12-epoxypyrano[4,3-j][1,2]benzodioxepine |

### Example 1-20

1H NMR (400 MHz, CDCl₃): 5.47 (1H, s), 4.94 (1H, d, J = 4.0 Hz), 3.75-3.71 (1H, m), 2.65-2.60 (1H, m), 2.43-2.35 (1H, m), 2.08-2.02(1H, m), 1.96-1.86 (2H, m), 1.79-1.73 (2H, m), 1.72-1.62 (3H, m), 1.57-1.48 (2H, m), 1.46 (3H, s), 1.40-1.26 (8H, m), 0.97 (3H, d, J = 6.4 Hz), 0.95-0.85 (5H, m).

### Example 1-23

1H NMR (400 MHz, CDCl₃): δ 5.46 (1H, s), 4.85 (1H, d, J = 3.6 Hz), 3.99-3.94 (1H, m), 3.64-3.52 (3H, m), 3.39 (1H, s), 2.67-2.62 (1H, m), 2.43-2.35 (1H, m), 2.08-2.03 (1H, m), 1.94-1.74 (3H, m), 1.67-1.62 (1H, m), 1.51-1.46 (4H, m), 1.40-1.21 (3H, m), 0.98-0.87 (7H, m).

### Example 1-24

1H NMR (400 MHz, CDCl₃): δ 7.39-7.30 (5H, m), 5.49 (1H, s), 4.95-4.92 (2H, m), 4.53 (1H, d, J = 12.0 Hz), 2.72-2.68 (1H, m), 2.45-2.37 (1H, m), 2.10-2.04 (1H, m), 1.94-1.79 (3H, m), 1.67-1.62 (1H, m), 1.56-1.51 (1H, m), 1.49 (3H, s), 1.38-1.24(3H, m), 0.98-0.96 (6H, m), 0.92-0.88 (1H, m).

### Example 1-22

### (3R,5aS,6R,8aS,9R,10S,12R,12aR)-3,6,9-Trimethyl-10-(2,2,2-trifluoroethoxy)decahydro-12H-3,12-epoxypyrano[4,3-j][1,2]benzodioxepine

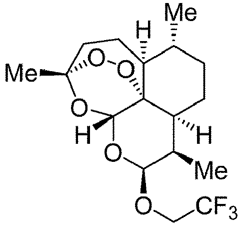

To a toluene solution (5 mL) of Q1 (100 mg) and 2,2,2-trifluoroethanol (351 mg), triphenylphosphine (184 mg) and diethyl azodicarboxylate (119 mg) were added at 0°C, and the resultant was stirred at 0°C for 2 hours. After the completion of the reaction, the reaction solution was subjected to distillation under reduced pressure, and the resulting residue was purified by silica gel column chromatography (elution solvent; pentane: ethyl acetate) to afford the compound of Example 1-22 (35 mg).
1H NMR (400 MHz, CDCl₃): δ 5.43 (1H, s), 4.90 (1H, d, J = 3.2 Hz), 4.20-4.10 (1H, m), 3.95-3.85 (1H, m), 2.74-2.66 (1H, m), 2.44-2.36 (1H, m), 2.10-2.04 (1H, m), 1.95-1.88 (1H, m), 1.84-1.76 (1H, m), 1.74-1.66 (2H, m), 1.57-1.49 (2H, m), 1.46 (3H, s), 1.40-1.24 (2H, m), 0.99-0.97 (6H, m), 0.95-0.89 (1H, m).

### Examples 1-21 and 1-25

Compounds shown in Table 2 were each obtained by performing reaction and treatment with use of the corresponding raw material compound in the same manner as in Example 1-19.

**[Table 2]**

| 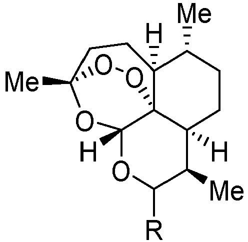 | | |
|---|---|---|
| Example | R | Compound name |
| 1-21 | 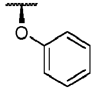 | (3R,5aS,6R,8aS,9R,10S,12R,12aR)-3,6,9-trimethyl-10-phenoxydecahydro-12H-3,12-epoxypyrano[4,3-j][1,2]benzodioxepine |
| 1-25 | 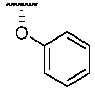 | (3R,5aS,6R,8aS,9R,10R,12R,12aR)-3,6,9-trimethyl-10-phenoxydecahydro-12H-3,12-epoxypyrano[4,3-j][1,2]benzodioxepine |

### Example 1-21

1H NMR (400 MHz, CDCl₃): δ 7.33-7.29 (2H, m), 7.15-7.13 (2H, m), 7.03-7.00 (1H, m), 5.54-5.52 (2H, m), 2.84-2.81 (1H, m), 2.44-2.38 (1H, m), 2.08-1.88 (4H, m), 1.75-1.70 (1H, m), 1.61-1.59 (1H, m), 1.55-1.46 (4H, m), 1.41-1.36 (1H, m), 1.34-1.30 (1H, m), 1.04 (3H, d, J = 6.0 Hz),

1.01-0.96 (4H, m).

### Example 1-25

1H NMR (400 MHz, CDCl₃): δ 7.33-7.28 (2H, m), 7.14-7.12 (2H, m), 7.04-7.00 (1H, m), 5.51 (1H, s), 5.07 (1H, d, J = 10.0 Hz), 2.78-2.73 (1H, m), 2.47-2.39 (1H, m), 2.09-2.04 (1H, m), 1.91-1.97 (1H, m), 1.86-1.80 (1H, m), 1.76-1.71 (1H, m), 1.69-1.65 (1H, m), 1.56-1.49 (1H, m), 1.46 (3H, s), 1.42-1.28 (3H, m), 1.11-1.04 (1H, m), 1.02-1.00 (6H, m).

### Example 1-26 and Example 1-27

### 2-{[(3R,5aS,6R,8aS,9R,10S,12R,12aR)-3,6,9-Trimethyldecahydro-12H-3,12-epoxypyrano[4,3-j][1,2]benzodioxepin-10-yl]oxy}ethyl acetate (Example 1-26) and 2-{[(3R,5aS,6R,8aS,9R,10R,12R,12aR)-3,6,9-trimethyldecahydro-12H-3,12-epoxypyrano[4,3-j][1,2]benzodioxepin-10-yl]oxy}ethyl acetate (Example 1-27)

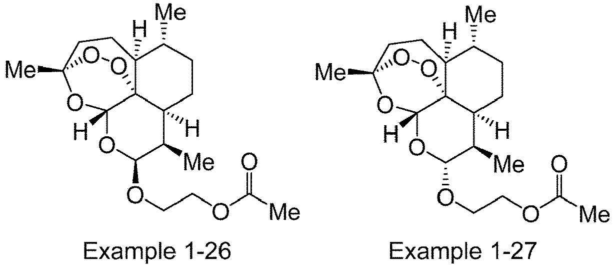

### Example 1-26

### 2-{[(3R,5aS,6R,8aS,9R,10S,12R,12aR)-3,6,9-Trimethyldecahydro-12H-3,12-epoxypyrano[4,3-j][1,2]benzodioxepin-10-yl]oxy}ethyl acetate

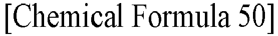

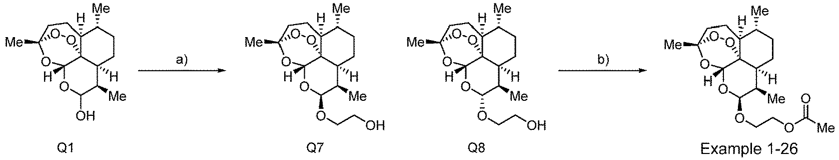

a) Production of 2-{[(3R,5aS,6R,8aS,9R,10S,12R,12aR)-3,6,9-trimethyldecahydro-12H-3,12-epoxypyrano[4,3-j][1,2]benzodioxepin-10-yl]oxy}ethan-1-ol (compound Q7) and 2-{[(3R,5aS,6R,8aS,9R,10R,12R,12aR)-3,6,9-trimethyldecahydro-12H-3,12-epoxypyrano[4,3-j][1,2]benzodioxepin-10-yl]oxylethan-1-ol (compound Q8)

To a dichloromethane solution (4 mL) of Q1 (200 mg) and ethylene glycol (130 mg), boron trifluoride-diethyl ether complex (56.5 mg) was added at room temperature, and the resultant was stirred at room temperature overnight. After the completion of the reaction, saturated sodium bicarbonate water (5 mL) and dichloromethane were added, the organic layer was washed with water, and the solvent was distilled off under reduced pressure. The resulting residue was purified by silica gel column chromatography (elution solvent; pentane: ethyl acetate) to afford compound Q7 (54 mg) and compound Q8 (62 mg).

### Compound Q7

1H NMR (400 MHz, CDCl₃) δ: 5.46 (1H, s), 4.86 (1H, d, J = 3.6 Hz), 3.93-3.88 (1H, m), 3.78-3.75 (2H, m), 3.68-3.63 (1H, m), 2.73-2.66 (1H, m), 2.39 (1H, m), 2.08-2.03 (1H, m), 1.94-1.87 (1H, m), 1.81-1.78 (2H, m), 1.75-1.71 (1H, m), 1.69-1.63 (1H, m), 1.54-1.47 (2H, m), 1.45 (3H, s), 1.39-1.30 (2H, m), 1.00-0.93 (7H, m).

### Compound Q8

1H NMR (400 MHz, CDCl₃) δ: 5.39 (1H, s), 4.49 (1H, d, J = 9.2 Hz), 3.89-3.87 (2H, m), 3.77-3.71 (2H, m), 2.51-2.36 (2H, m), 2.08-2.02 (1H, m), 1.95-1.88 (1H, m), 1.83-1.77 (1H, m), 1.75-1.70 (1H, m), 1.59-1.64 (1H, m), 1.53-1.49 (1H, m), 1.44 (3H, s), 1.36-1.24 (4H, m), 1.07-1.01 (1H, m), 0.98 (3H, d, J = 5.6 Hz), 0.94 (3H, d, J = 6.8 Hz).

b) Production of 2-{[(3R,5aS,6R,8aS,9R,10S,12R,12aR)-3,6,9-trimethyldecahydro-12H-3,12-epoxypyrano[4,3-j][1,2]benzodioxepin-10-yl]oxy}ethyl acetate (Example 1-26)

To a dichloromethane solution (3 mL) of compound Q7 (54 mg) obtained in the previous experiment, acetic anhydride (83.7 mg) and triethylamine (82.9 mg) were added, and the resultant was stirred at room temperature overnight. After the completion of the reaction, dichloromethane and water were added, the solvent of the organic layer was distilled off under reduced pressure, and the resulting residue was purified by silica gel column chromatography (elution solvent; pentane:ethyl acetate) to afford Example 1-26 (29.1 mg).
1H NMR (400 MHz, CDCl₃) δ: 5.45 (1H, s), 4.84 (1H, d, J = 3.2 Hz), 4.30-4.20 (2H, m), 4.05-4.00 (1H, m), 3.69-3.64 (1H, m), 2.69-2.62 (1H, m), 2.39 (1H, m), 2.10-2.03 (4H, m), 1.94-1.88 (1H, m), 1.86-1.75 (2H, m), 1.67-1.64 (1H, m), 1.51-1.49 (1H, m), 1.46 (3H, s), 1.34-1.24 (3H, m), 0.98-0.88 (7H, m).

### Example 1-27

### 2-{[(3R,5aS,6R,8aS,9R,10R,12R,12aR)-3,6,9-Trimethyldecahydro-12H-3,12-epoxypyrano[4,3-j][1,2]benzodioxepin-10-yl]oxy}ethyl acetate

The compound of Example 1-27 was obtained by performing reaction and treatment with use of the corresponding raw material compound, Q8, in the same manner as in Example 1-26.

1H NMR (400 MHz, CDCl₃) δ: 5.36 (1H, s), 4.50 (1H, d, J = 9.2 Hz), 4.26 (2H, t, J = 5.0 Hz), 4.14-4.09 (1H, m), 3.76-3.70 (1H, m), 2.49-2.36 (2H, m), 2.09 (3H, s), 2.07-2.02 (1H, m), 1.94-1.87 (1H, m), 1.82-1.76 (1H, m), 1.73-1.69 (1H, m), 1.60-1.55 (1H, m), 1.52-1.51 (1H, m), 1.49 (3H, s), 1.34-1.27 (3H, m), 1.07-0.97 (4H, m), 0.90 (3H, d, J = 7.2 Hz).

### Example 1-34

### (3R,3aS,6R,6aS,9S,10aS,10bR)-3,6,9-Trimethyloctahydro-10aH-9,10b-epoxypyrano[4,3,2-jk][2]benzoxepin-2(3H)-one

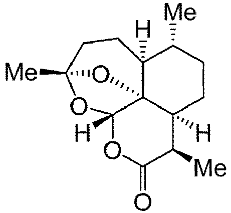

A compound purchased from Sigma-Aldrich Co. LCC (catalog code: CDS010483) was used.

### Example 1-35

### (3R,5aS,6R,8aS,9R,10R,12R,12aR)-10-Methoxy-3,6,9-trimethyldecahydro-12H-3,12-epoxypyrano[4,3-j][1,2]benzodioxepine

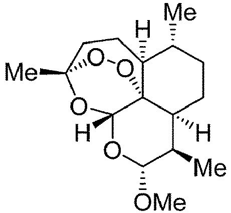

A compound purchased from Toronto Research Chemicals, Inc. (catalog code: A777410) was used.

### Housing of Mice

Mice with insertion of a nucleic acid sequence containing three copies of a gene encoding the fluorescent protein mCherry to the C terminus of the mouse endogenous HoxB5 gene (Hoxb5-tri-mCherry) were used (triple-mCherry Hoxb5 knock-in mice; Non Patent Literature 1). The mice were housed under environmental conditions such that the room temperature and the humidity were 20 to 26°C and 40 to 70%, respectively, under light for 12 hours and under dark for 12 hours (illumination: 8:00 a.m. to 8:00 p.m.).

### Collection of Bone Marrow Cells

Bone marrow cells were collected from the tibia, femur, humerus, and pelvis in each side of each of the mice, and gathered in a mortar. A buffer obtained by adding thermally deactivated bovine serum (manufactured by Gibco) at a final concentration of 2% and EDTA at a final concentration of 2 mM to Ca²⁺-free and Mg²⁺-free PBS was added, and the resultant was broken with a pestle to obtain a cell population including hematopoietic stem cells.

### Classification of Mouse Long-Term Hematopoietic Stem Cells by Flow Cytometry

First, the thus-obtained cell population including hematopoietic stem cells was passed through strainers (meshes) of 100 µm, 70 µm, and 40 µm. The cell population was stained with an APC-binding anti-c-Kit (2B8) and fractionated with anti-APC magnetic beads and an LS column (both manufactured by Miltenyi Biotec) to obtain a cell population enriched with hematopoietic stem cells and progenitor cells.

Subsequently, cells being c-Kit-positive were stained with antibodies for the following cell surface markers.
Cell surface markers: Sca-1, Flk2, CD150, CD34, Ter-119, B220, CD3, CD4, CD8a, Gr-1, CD11b, IL-7R, and CD16/32

Staining with the antibodies was performed at 4°C, and the cells were incubated for 30 minutes. For staining with the CD34 antibody, the cells were incubated for 90 minutes. Dead cell staining was performed with SYTOX Red Dead Cell Stain (manufactured by Life Technologies) in accordance with a method recommended by the manufacturer.

Flow cytometry analysis and cell classification (cell sorting) were performed with a FACS Aria II cell sorter (manufactured by BD Biosciences), wherein Lineage-negative, c-Kit-positive, Sca-1-positive, Flk2-negative, CD34-negative or weakly CD34-positive, CD150-positive, and mCherry-positive fractions were regarded as long-term hematopoietic stem cells, and analysis was performed with the software FlowJo (manufactured by Tree Star, Inc.).

The cells after classification were seeded in a 96-well plate. A cell culture medium was prepared by adding Stem Cell Factor (SCF, manufactured by PeproTech, Inc.) and Thrombopoietin (TPO, manufactured by PeproTech, Inc.) to StemSpan SFEM culture medium (manufactured by STEMCELL Technologies Inc.) and added in advance to a cell culture 96-well plate (manufactured by BD Biosciences) at 100 µL/well, and the cells sorted with the FACS Aria II cell sorter (BD Biosciences) were then seeded at 10 cells/well.

### [Example 2: Primary Screening of Compounds]

The test compounds each in the form of 10 mM DMSO solution were diluted with the cell culture medium to a final concentration of 1 µM, and added to the cells seeded after classification through medium exchange to a final volume of 200 µL/well. Thereafter, the cells were cultured in a CO₂ incubator under conditions of 37°C and 5% CO₂ for 1 week.

After culture, the cells were stained with the antibodies for the surface markers c-Kit and Sca-1, Ter-119, B220, CD3, CD4, CD8a, Gr-1, and CD11b, and the ratio and post-growth cell count of a KLS fraction (c-Kit⁺, Lin⁻, Sca-1⁺) or an mCherry-positive fraction given with use of a FACS Aria II cell sorter were analyzed by using FlowJo (BD Biosciences). The mCherry-positive fraction is a cell fraction expressing the Hoxb5 gene, and the KLS fraction is a cell fraction being c-Kit-positive, Lin-negative, and Sca-1-positive, and includes hematopoietic stem cells and multipotent progenitor cells as main components.

Compounds that provided a KLS-positive rate of 30% or more or an mCherry-positive rate of 30% or more, and a cell count of 10 cells or more in the analysis were regarded as hit compounds. The hit compounds were the following five compounds. The KLS-positive rate is the proportion (%) of KLS (c-Kit⁺, Lin⁻, Sca-1⁺) cell count to the total viable cell count, and mCherry-positive rate is the proportion (%) of mCherry-positive cell count to the total viable cell count. 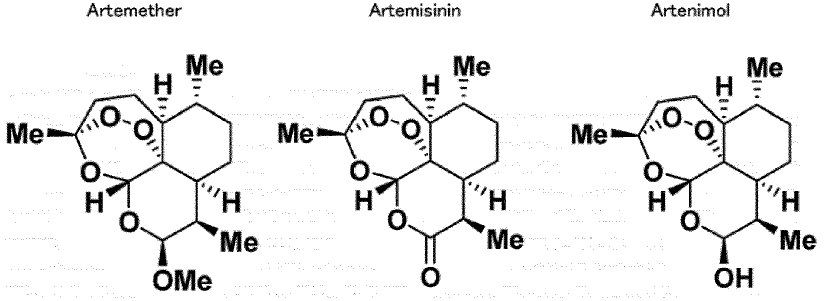 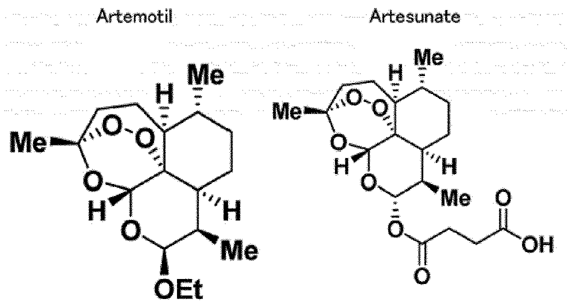 (In each formula, Me represents a methyl group, and Et represents an ethyl group.)

### [Example 3: Reproducibility Test and Concentration Dependence Test for Hit Compounds]

A DMSO solution of each of the five hit compounds was adjusted with the cell culture medium to five final concentrations (0.1 µM, 0.3 µM, 1 µM, 3 µM, 10 µM), and cell culture was performed at n = 2 by the same method as in Example 1. Viable cell counts, KLS-positive rates, and mCherry-positive rates were analyzed, and the results are shown in Table 3. The cell culture and evaluation criteria were the same as those in Example 2.

**[Table 3]**

| | | Concentration of compound (µM) | | | | |
|---|---|---|---|---|---|---|
| | | 10 | 3 | 1 | 0.3 | 0.1 |
| Artemether | Viable cell count (cell) | 108.5 | 115 | 1170.5 | 2632 | 1109 |
| | KLS-positive rate (%) | 65.9 | 50.3 | 21.4 | 22.05 | 50.25 |
| | mCherry-positive rate (%) | 94.45 | 98.05 | 57.85 | 36.85 | 67.2 |
| Artemisinin | Viable cell count (cell) | 282.5 | 229 | 728 | 1020 | 445.5 |
| | KLS-positive rate (%) | 52 | 50.8 | 46.85 | 18.2 | 33.2 |
| | mCherry-positive rate (%) | 80 | 81.3 | 60.85 | 28.15 | 49.25 |
| Artenimol | Viable cell count (cell) | 0 | 0 | 666.5 | 2268 | 1563 |
| | KLS-positive rate (%) | 0 | 0 | 20.05 | 18 | 8.92 |
| | mCherry-positive rate (%) | 0 | 0 | 84.8 | 42.4 | 16.9 |
| Artemotil | Viable cell count (cell) | 0 | 8 | 249 | 1529 | 1073 |
| | KLS-positive rate (%) | 0 | 66.65 | 30.8 | 21.95 | 28.85 |
| | mCherry-positive rate (%) | 0 | 100 | 92.85 | 47.65 | 57.4 |
| Artesunate | Viable cell count (cell) | 0.5 | 0.5 | 266 | 540 | 1065.5 |
| | KLS-positive rate (%) | 0 | 0 | 45.3 | 46.4 | 36.3 |
| | mCherry-positive rate (%) | 0 | 50 | 91.2 | 35.1 | 23.59 |

From Table 3, we were able to confirm high mCherry-positive cell ratios for cells on day 7 of culture in each culture medium containing a tested compound, although the effects of each compound differed among different concentrations. From this finding, it was understood that those compounds allow long-term hematopoietic stem cells to be cultured and grow with the self-renewal capacity and multipotency maintained.

In addition to the five compounds (Examples 1-29, 1-30, 1-31, 1-32, and 1-33: artenimol, artemether, artemotil, artesunate, and artemisinin, respectively), a DMSO solution of each of the compounds of Examples 1-1 to 1-28, 1-34, and 1-35 was adjusted with the cell culture medium to five final concentrations (0.1 µM, 0.3 µM, 1 µM, 3 µM, 10 µM), and cell culture was performed at n = 2 by the same method as in Example 2. Viable cell counts, KLS-positive rates, and mCherry-positive rates were analyzed, and the results are shown in Table 4. The cell culture and evaluation criteria were the same as those in Example 2.

**[Table 4]**

| Example | Evaluation item | Concentration of compound (µM) | | | | |
|---|---|---|---|---|---|---|
| | | 10 | 3 | 1 | 0.3 | 0.1 |
| 1-1 | Viable cell count (cell) | 0.0 | 0.0 | 0.5 | 0.0 | 477.0 |
| | KLS-positive rate (%) | 0.0 | 0.0 | 0.0 | 0.0 | 46.5 |
| | mCherry-positive rate (%) | 0.0 | 0.0 | 50.0 | 0.0 | 0.5 |
| 1-2 | Viable cell count (cell) | 0.0 | 0.5 | 0.5 | 0.5 | 340.5 |
| | KLS-positive rate (%) | 0.0 | 0.0 | 0.0 | 50.0 | 16.0 |
| | mCherry-positive rate (%) | 0.0 | 50.0 | 0.0 | 50.0 | 59.9 |
| 1-3 | Viable cell count (cell) | 3.5 | 139.0 | 299.5 | 324.0 | 445.0 |
| | KLS-positive rate (%) | 83.4 | 24.2 | 20.3 | 18.6 | 15.8 |
| | mCherry-positive rate (%) | 100.0 | 97.0 | 38.6 | 4.7 | 8.0 |
| 1-4 | Viable cell count (cell) | 7.5 | 87.5 | 181.5 | 103.0 | 353.0 |
| | KLS-positive rate (%) | 61.4 | 30.7 | 31.1 | 37.9 | 17.2 |
| | mCherry-positive rate (%) | 90.9 | 100.0 | 85.2 | 44.7 | 8.0 |
| 1-5 | Viable cell count (cell) | 1.5 | 184.0 | 308.0 | 221.5 | 443.5 |
| | KLS-positive rate (%) | 0.0 | 30.8 | 20.0 | 41.7 | 18.2 |
| | mCherry-positive rate (%) | 0.0 | 41.5 | 4.5 | 8.5 | 11.5 |
| 1-6 | Viable cell count (cell) | 37.5 | 215.5 | 183.5 | 190.0 | 147.0 |
| | KLS-positive rate (%) | 47.8 | 17.6 | 19.8 | 20.9 | 34.8 |
| | mCherry-positive rate (%) | 98.4 | 80.8 | 24.4 | 15.6 | 2.0 |
| 1-7 | Viable cell count (cell) | 0.0 | 0.0 | 73.0 | 269.0 | 225.0 |
| | KLS-positive rate (%) | 0.0 | 0.0 | 36.1 | 19.6 | 26.9 |
| | mCherry-positive rate (%) | 0.0 | 0.0 | 96.7 | 62.2 | 42.9 |
| 1-8 | Viable cell count (cell) | 286.5 | 204.5 | 248.0 | 379.5 | 396.5 |
| | KLS-positive rate (%) | 20.6 | 37.3 | 28.7 | 18.2 | 17.3 |
| | mCherry-positive rate (%) | 29.0 | 12.9 | 12.0 | 7.7 | 3.0 |
| 1-9 | Viable cell count (cell) | 491.5 | 479.0 | 625.0 | 500.0 | 627.5 |
| | KLS-positive rate (%) | 27.1 | 14.1 | 34.3 | 31.1 | 10.6 |
| | mCherry-positive rate (%) | 11.4 | 8.1 | 9.4 | 4.5 | 5.9 |
| 1-10 | Viable cell count (cell) | 46.5 | 579.0 | 325.5 | 387.0 | 717.5 |
| | KLS-positive rate (%) | 53.1 | 33.1 | 32.9 | 23.1 | 19.7 |
| | mCherry-positive rate (%) | 99.3 | 38.5 | 9.1 | 16.5 | 6.5 |
| 1-11 | Viable cell count (cell) | 1.5 | 31.0 | 196.0 | 267.0 | 452.5 |
| | KLS-positive rate (%) | 100.0 | 32.4 | 26.0 | 17.6 | 13.2 |
| | mCherry-positive rate (%) | 100.0 | 86.2 | 60.4 | 29.2 | 14.3 |
| 1-12 | Viable cell count (cell) | 234.5 | 217.0 | 431.5 | 262.0 | 450.5 |
| | KLS-positive rate (%) | 36.6 | 14.4 | 19.3 | 15.8 | 27.2 |
| | mCherry-positive rate (%) | 12.1 | 19.4 | 8.2 | 15.2 | 7.3 |
| 1-13 | Viable cell count (cell) | 0.0 | 0.0 | 136.5 | 605.0 | 201.0 |
| | KLS-positive rate (%) | 0.0 | 0.0 | 21.0 | 14.7 | 22.1 |
| | mCherry-positive rate (%) | 0.0 | 0.0 | 6.7 | 2.9 | 24.3 |
| 1-14 | Viable cell count (cell) | 0.5 | 1.0 | 119.0 | 340.0 | 497.5 |
| | KLS-positive rate (%) | 0.0 | 50.0 | 47.3 | 30.1 | 20.6 |
| | mCherry-positive rate (%) | 0.0 | 100.0 | 22.1 | 9.0 | 0.2 |
| 1-15 | Viable cell count (cell) | 42.5 | 111.0 | 258.0 | 540.0 | 379.0 |
| | KLS-positive rate (%) | 54.9 | 40.0 | 17.9 | 13.0 | 6.8 |
| | mCherry-positive rate (%) | 97.6 | 87.1 | 47.3 | 13.3 | 7.1 |
| 1-16 | Viable cell count (cell) | 29.0 | 182.5 | 310.0 | 388.5 | 504.5 |
| | KLS-positive rate (%) | 52.6 | 9.2 | 33.4 | 11.6 | 13.3 |
| | mCherry-positive rate (%) | 99.0 | 92.4 | 68.2 | 29.2 | 6.8 |
| 1-17 | Viable cell count (cell) | 80.0 | 315.0 | 641.5 | 902.0 | 947.0 |
| | KLS-positive rate (%) | 51.8 | 24.0 | 19.4 | 16.1 | 15.9 |
| | mCherry-positive rate (%) | 100.0 | 70.1 | 16.1 | 2.5 | 4.9 |
| 1-18 | Viable cell count (cell) | 72.5 | 154.0 | 162.0 | 191.0 | 405.0 |
| | KLS-positive rate (%) | 49.6 | 30.9 | 22.4 | 16.7 | 10.9 |
| | mCherry-positive rate (%) | 98.6 | 80.5 | 19.2 | 15.4 | 23.5 |
| 1-19 | Viable cell count (cell) | 0.5 | 0.0 | 6.0 | 151.0 | 469.0 |
| | KLS-positive rate (%) | 0.0 | 0.0 | 4.2 | 41.7 | 9.5 |
| | mCherry-positive rate (%) | 50.0 | 0.0 | 50.0 | 96.2 | 56.3 |
| 1-20 | Viable cell count (cell) | 0.5 | 0.5 | 1.0 | 0.0 | 0.0 |
| | KLS-positive rate (%) | 0.0 | 0.0 | 0.0 | 0.0 | 0.0 |
| | mCherry-positive rate (%) | 50.0 | 50.0 | 0.0 | 0.0 | 0.0 |
| 1-21 | Viable cell count (cell) | 0.5 | 0.5 | 0.5 | 2.0 | 0.0 |
| | KLS-positive rate (%) | 0.0 | 0.0 | 0.0 | 0.0 | 0.0 |
| | mCherry-positive rate (%) | 50.0 | 50.0 | 0.0 | 50.0 | 0.0 |
| 1-22 | Viable cell count (cell) | 0.5 | 0.5 | 0.5 | 12.5 | 216.0 |
| | KLS-positive rate (%) | 0.0 | 50.0 | 0.0 | 87.0 | 26.7 |
| | mCherry-positive rate (%) | 0.0 | 50.0 | 0.0 | 100.0 | 90.9 |
| 1-23 | Viable cell count (cell) | 63.5 | 252.0 | 729.0 | 509.0 | 712.5 |
| | KLS-positive rate (%) | 42.3 | 23.2 | 5.3 | 15.4 | 12.4 |
| | mCherry-positive rate (%) | 100.0 | 97.3 | 72.7 | 46.5 | 17.9 |
| 1-24 | Viable cell count (cell) | 0.5 | 0.0 | 0.5 | 0.5 | 18.0 |
| | KLS-positive rate (%) | 0.0 | 0.0 | 0.0 | 0.0 | 73.6 |
| | mCherry-positive rate (%) | 0.0 | 0.0 | 50.0 | 50.0 | 94.1 |
| 1-25 | Viable cell count (cell) | 0.0 | 0.5 | 0.0 | 0.5 | 181.5 |
| | KLS-positive rate (%) | 0.0 | 50.0 | 0.0 | 0.0 | 40.0 |
| | mCherry-positive rate (%) | 0.0 | 50.0 | 0.0 | 0.0 | 52.3 |
| 1-26 | Viable cell count (cell) | 30.5 | 43.5 | 20.0 | 48.5 | 13.5 |
| | KLS-positive rate (%) | 28.9 | 26.9 | 55.4 | 68.5 | 56.1 |
| | mCherry-positive rate (%) | 100.0 | 100.0 | 96.7 | 95.0 | 80.7 |
| 1-27 | Viable cell count (cell) | 17.5 | 6.0 | 6.0 | 14.5 | 122.0 |
| | KLS-positive rate (%) | 49.8 | 90.0 | 100.0 | 75.0 | 45.4 |
| | mCherry-positive rate (%) | 100.0 | 100.0 | 100.0 | 94.7 | 44.3 |
| 1-28 | Viable cell count (cell) | 138.0 | 283.0 | 522.0 | 305.0 | 275.5 |
| | KLS-positive rate (%) | 41.2 | 16.5 | 10.5 | 13.5 | 21.5 |
| | mCherry-positive rate (%) | 96.4 | 80.3 | 35.5 | 15.0 | 12.9 |
| 1-29 | Viable cell count (cell) | 0.5 | 4.5 | 248.0 | 215.0 | 469.5 |
| | KLS-positive rate (%) | 50.0 | 80.0 | 11.3 | 13.7 | 19.4 |
| | mCherry-positive rate (%) | 50.0 | 100.0 | 3.9 | 6.0 | 13.3 |
| 1-30 | Viable cell count (cell) | 65.0 | 44.5 | 135.5 | 184.0 | 238.0 |
| | KLS-positive rate (%) | 71.4 | 57.4 | 40.1 | 38.4 | 35.3 |
| | mCherry-positive rate (%) | 98.2 | 100.0 | 99.0 | 100.0 | 71.2 |
| 1-31 | Viable cell count (cell) | 1.5 | 2.5 | 19.5 | 194.0 | 201.5 |
| | KLS-positive rate (%) | 25.0 | 87.5 | 62.3 | 19.7 | 26.5 |
| | mCherry-positive rate (%) | 50.0 | 100.0 | 98.3 | 96.3 | 84.9 |
| 1-32 | Viable cell count (cell) | 1.0 | 238.5 | 9.0 | 355.5 | 597.5 |
| | KLS-positive rate (%) | 0.0 | 21.2 | 90.6 | 12.4 | 21.0 |
| | mCherry-positive rate (%) | 25.0 | 53.3 | 100.0 | 6.0 | 9.4 |
| 1-33 | Viable cell count (cell) | 178.0 | 85.0 | 268.0 | 309.5 | 268.5 |
| | KLS-positive rate (%) | 16.1 | 26.1 | 20.3 | 11.2 | 20.2 |
| | mCherry-positive rate (%) | 78.8 | 81.8 | 56.8 | 23.5 | 28.1 |
| 1-34 | Viable cell count (cell) | 70.0 | 268.0 | 713.5 | 389.0 | 136.5 |
| | KLS-positive rate (%) | 27.5 | 27.7 | 21.8 | 28.4 | 77.3 |
| | mCherry-positive rate (%) | 0.4 | 14.6 | 15.2 | 18.3 | 11.7 |
| 1-35 | Viable cell count (cell) | 121.5 | 104.5 | 554.0 | 544.5 | 763.5 |
| | KLS-positive rate (%) | 46.7 | 48.7 | 17.9 | 19.9 | 13.8 |
| | mCherry-positive rate (%) | 99.6 | 92.2 | 74.0 | 48.5 | 3.0 |

From Table 4, we were able to confirm mCherry-positive cell ratios for cells on day 7 of culture in each culture medium containing a tested compound, although the effects of each compound differed among different concentrations. This finding revealed that those compounds all allow long-term hematopoietic stem cells to be cultured and grow with the self-renewal capacity and multipotency maintained. For a compound that gave particularly preferable results, evaluation was carried out with use of human cells as described below.

### [Example 4: Confirmation Test by Gene Expression of Mouse HoxB5]

Cells cultured in the presence of artemether were evaluated by gene expression of mouse HoxB5. Cells were cultured in the same manner as in Example 3, and the cells after culture were treated with a Single Cell to CT qRT-PCR kit (manufactured by Life Technologies) according to the instruction of the kit. The cells treated were washed once with PBS, a cell-lysing solution was then added thereto, and the resultant was subjected to RNA extraction followed by reverse transcription reaction for cDNA synthesis, amplified through Taqman Gene Expression Assay in 14 cycles, and diluted with 1× TE buffer (pH 8.0). Real-time PCR was performed with the following Taqman probes at 95°C for 3 seconds and at 60°C for 30 seconds in 40 cycles to amplify the sample (HoxbB5: Mm00657672 (manufactured by Thermo Fisher Scientific), Gapdh: Mm99999915-g1 (manufactured by Thermo Fisher Scientific)). All the thermal cycler operations were analyzed by using a QuantStudio 6 (manufactured by Applied Biosystems) (Table 5).

**[Table 5]**

| | Concentration of compound (µM) | | | | | |
|---|---|---|---|---|---|---|
| | 10 | 3 | 1 | 0.3 | 0.1 | 0.03 |
| mHoxB5 Ct value | 27.62 | 28.99 | 31.18 | 28.79 | 30.57 | 30.72 |
| mGAPDH Ct value | 17.68 | 13.90 | 13.02 | 14.47 | 13.23 | 13.12 |
| Ct value difference | 9.94 | 15.10 | 18.16 | 14.31 | 17.34 | 17.60 |

From Table 5, we were able to confirm, by allowing the compound to act, the expression of the long-term hematopoietic stem cell marker HoxB5 in cells on day 7 of culture under culture conditions such that the mouse HoxB5 Ct value was 30 or less and the ΔCT value was 16 or less. This finding suggested, also in terms of expression of a gene marker, that the present compound has a function to maintain long-term hematopoietic stem cells in vitro.

### [Example 5: Combination Test with UM171]

A DMSO solution of artemether was adjusted with the cell culture medium to three final concentrations (0.1 µM, 1 µM, 10 µM), and a DMSO solution of UM171 was adjusted with the cell culture medium to four final concentrations (0.1 µM, 0.3 µM, 1 µM, 3 µM), and cell culture was performed at n = 2 by the same method as in Example 1. Viable cell counts, KLS-positive rates, and mCherry-positive rates were analyzed, and the results are shown in Table 6. The cell culture and evaluation criteria were the same as those in Example 2.

**[Table 6]**

| Compound | Evaluation item | UM171 (µM) | | | |
|---|---|---|---|---|---|
| | | 0.1 | 0.3 | 1 | 3 |
| DMSO | Viable cell count (cell) | 508.0 | 281.0 | 24.5 | 82.5 |
| | KLS-positive rate (%) | 41.0 | 71.6 | 98.0 | 82.2 |
| | mCherry-positive rate (%) | 4.7 | 17.1 | 38.9 | 16.2 |
| Artemether 0.1 µM | Viable cell count (cell) | 430.0 | 257.0 | 28.5 | 101.5 |
| | KLS-positive rate (%) | 52.0 | 42.0 | 93.4 | 60.6 |
| | mCherry-positive rate (%) | 51.8 | 78.1 | 100.0 | 82.4 |
| Artemether 1 µM | Viable cell count (cell) | 269.0 | 151.0 | 23.0 | 23.5 |
| | KLS-positive rate (%) | 33.1 | 48.6 | 93.7 | 67.3 |
| | mCherry-positive rate (%) | 96.3 | 99.1 | 100.0 | 100.0 |
| Artemether 10 µM | Viable cell count (cell) | 122.0 | 38.0 | 9.5 | 19.0 |
| | KLS-positive rate (%) | 56.7 | 91.1 | 95.9 | 94.7 |
| | mCherry-positive rate (%) | 99.6 | 100.0 | 100.0 | 96.9 |

Table 6 showed that, without artemether, the increasing effect of UM171 on the mCherry-positive cell ratio was limited. However, an effect that increases the KLS-positive cell ratio was found for UM171. On the other hand, it was found that culture in a culture medium containing artemether and UM171 resulted in enhanced mCherry-positive cell ratios and KLS-positive cell ratios as compared with culture with any one of the substances alone. These findings revealed that artemether and UM171 exhibit a combinational effect.

### [Example 6: Confirmation Test with Human Cells for Compounds]

### Collection of Human Cord Blood-Derived Mononuclear Cells

Human cord blood-derived mononuclear cells were subjected to removal of erythrocytes and concentration treatment by using EasySep RBC Depletion Reagent (manufactured by STEMCELL Technologies Inc.) to obtain a cell population including hematopoietic stem cells.

### Classification of Human Hematopoietic Stem Cells (CD34-Positive Cells) by Flow Cytometry

First, a CD34-positive cell fraction was partially purified from the thus-obtained cell population including hematopoietic stem cells by using an EASYSep Human Cord Blood CD34 Positive Selection Kit II (manufactured by STEMCELL Technologies Inc.). The concentrated CD34-positive cells in a cell population enriched with hematopoietic stem cells and progenitor cells were stained with antibodies for the following cell surface markers.
Cell surface markers: CD34, CD11b, CD14, CD19, CD20, CD235ab, CD3, CD4, CD56, CD8a.

Staining with the antibodies was performed at 4°C, and the cells were incubated for 30 minutes. For staining with the CD34 antibody, the cells were incubated for 90 minutes. Dead cell staining was performed with SYTOX Blue Dead Cell Stain (manufactured by Life Technologies) in accordance with a method recommended by the manufacturer.

Flow cytometry analysis and cell classification (cell sorting) were performed with a FACS Aria III cell sorter (manufactured by BD Biosciences), wherein fractions being Lineage-negative and CD34-positive were regarded as hematopoietic stem cells, and analysis was performed with the software FlowJo (manufactured by Tree Star, Inc.).

The cells after classification were seeded in a 96-well plate. A cell culture medium was prepared by adding Stem Cell Factor (SCF, manufactured by PeproTech, Inc.), Thrombopoietin (TPO, manufactured by PeproTech, Inc.), L-Glutamine, Penicillin, Streptomycin (manufactured by Thermo Fisher Scientific), and Insulin-Transferrin-Selenium-Ethanolamine (manufactured by Thermo Fisher Scientific) to IMDM culture medium (manufactured by Thermo Fisher Scientific) and added in advance to a cell culture 96-well plate (manufactured by Corning Incorporated) at 200 µL/well, and the cells sorted with the FACS Aria III cell sorter (BD Biosciences) were then seeded at 30 cells/well.

Each of artemether and the compounds of Example 1-23, Example 1-16, and Example 1-7 was prepared with the cell culture medium at nine final concentrations (0.001 µM, 0.003 µM, 0.01 µM, 0.03 µM, 0.1 µM, 0.3 µM, 1 µM, 3 µM, 10 µM), and added to the above human cells seeded after classification to a final volume of 200 µL/well to act thereon. UM171 (35 nM) was added to some of them to examine the presence or absence of synergistic effect. Thereafter, the cells were cultured in a CO₂ incubator under conditions of 37°C and 5% CO₂ for 1 week. Viable cell counts and CD34-positive cell counts were analyzed.

Figure 1 and Figure 2 show viable cell counts and CD34-positive cell counts for culture under (UM171+/-) in the presence of artemether. Tables 7 to 11 show viable cell counts and CD34-positive cell counts for culture in the presence of a tested compound(s).

After culture, the cells were stained with the antibodies for the surface markers CD34, CD11b, CD14, CD19, CD20, CD235ab, CD3, CD4, CD56, and CD8a, and the visible cell count and the ratio and post-growth cell count of a CD34 fraction (CD34⁺, Lin⁻) were measured by using a FACS Aria III cell sorter, and analyzed by using FlowJo (BD Biosciences). The CD34 fraction is a fraction including hematopoietic stem cells and multipotent progenitor cells as main components.

From Tables 7 to 11, we were able to confirm increase in the visible cell count and the cell count of the CD34 fraction for cells on day 7 of culture in each culture medium containing a tested compound(s), although the effects of each compound differed among different concentrations. In addition, we were also able to confirm the synergistic effect of use of UM171 in combination. From these findings with human cells, it was confirmed that those compounds allow hematopoietic stem cells to be cultured and grow with the self-renewal capacity and multipotency maintained.

**[Table 7]**

| Culture condition | Viable cell count (cell) | CD34-positive cell count (cell) |
|---|---|---|
| Before culture | 180 | 180 |
| DMSO | 786 | 53 |
| Artemether (0.001 µM) | 484 | 59 |
| Artemether (0.003 µM) | 921 | 88 |
| Artemether (0.01 µM) | 498 | 57 |
| Artemether (0.03 µM) | 298 | 23 |
| Artemether (0.1 µM) | 555 | 45 |
| Artemether (0.3 µM) | 550 | 61 |
| Artemether (1 µM) | 479 | 35 |
| Artemether (3 µM) | 380 | 26 |
| Artemether (10 µM) | 250 | 27 |

**[Table 8]**

| Culture condition | Viable cell count (cell) | CD34-positive cell count (cell) |
|---|---|---|
| Before culture | 180 | 180 |
| DMSO | 90 | 14 |
| UM171 (35 nM) and artemether (0.001 µM) | 301 | 36 |
| UM171 (35 nM) and artemether (0.003 µM) | 765 | 102 |
| UM171 (35 nM) and artemether (0.01 µM) | 323 | 43 |
| UM171 (35 nM) and artemether (0.03 µM) | 216 | 56 |
| UM171 (35 nM) and artemether (0.1 µM) | 376 | 93 |
| UM171 (35 nM) and artemether (0.3 µM) | 240 | 9 |
| UM171 (35 nM) and artemether (1 µM) | 31 | 0 |

**[Table 9]**

| Culture condition | Viable cell count (cell) | CD34-positive cell count (cell) |
|---|---|---|
| Before culture | 180 | 180 |
| DMSO | 665 | 44 |
| Example 1-23 (0.001 µM) | 1581 | 156 |
| Example 1-23 (0.003 µM) | 797 | 65 |
| Example 1-23 (0.01 µM) | 1152 | 72 |
| Example 1-23 (0.03 µM) | 901 | 63 |
| Example 1-23 (0.1 µM) | 537 | 28 |
| Example 1-23 (0.3 µM | 462 | 19 |
| Example 1-23 (1 µM) | 480 | 16 |
| Example 1-23 (3 µM) | 269 | 15 |
| Example 1-23 (10 µM) | 333 | 0 |

**[Table 10]**

| Culture condition | Viable cell count (cell) | CD34-positive cell count (cell) |
|---|---|---|
| Before culture | 180 | 180 |
| DMSO | 1098 | 129 |
| Example 1-16 (0.001 µM) | 1306 | 143 |
| Example 1-16 (0.003 µM) | 1224 | 173 |
| Example 1-16 (0.01 µM) | 1301 | 116 |
| Example 1-16 (0.03 µM) | 590 | 130 |
| Example 1-16 (0.1 µM) | 811 | 92 |
| Example 1-16 (0.3 µM) | 549 | 32 |
| Example 1-16 (1 µM) | 368 | 13 |
| Example 1-16 (3 µM) | 300 | 13 |
| Example 1-16 (10 µM) | 264 | 3 |

**[Table 11]**

| Culture condition | Viable cell count (cell) | CD34-positive cell count (cell) |
|---|---|---|
| Before culture | 180 | 180 |
| DMSO | 1321 | 145 |
| Example 1-7 (0.001 µM) | 1314 | 175 |
| Example 1-7 (0.003 µM) | 1013 | 113 |
| Example 1-7 (0.01 µM) | 1115 | 150 |
| Example 1-7 (0.03 µM) | 905 | 86 |
| Example 1-7 (0.1 µM) | 693 | 80 |
| Example 1-7 (0.3 µM) | 439 | 5 |
| Example 1-7 (1 µM) | 196 | 4 |
| Example 1-7 (3 µM) | 202 | 0 |
| Example 1-7 (10 µM) | 181 | 0 |

### [Example 7: Functional Test with Human Cells for Compounds]

Each of artemether and the compounds of Example 1-23, Example 1-16, and Example 1-7 was prepared with the cell culture medium at nine final concentrations (0.001 µM, 0.003 µM, 0.01 µM, 0.03 µM, 0.1 µM, 0.3 µM, 1 µM, 3 µM, 10 µM), and added to the above human cells seeded after classification to a final volume of 200 µL/well to act thereon. UM171 (35 nM) was added to some of them to examine the presence or absence of synergistic effect. Thereafter, the cells were cultured in a CO₂ incubator under conditions of 37°C and 5% CO₂ for 1 week, collected, and then seeded in a 96-well plate to place one CD34-positive cell in each well by using a FACS Aria III cell sorter, and cultured with a MethoCult H4435 (STEMCELL Technologies Inc.) for 2 weeks. After culture, each well was photographed with an optical microscope (manufactured by KEYENCE CORPORATION), and the colony count was determined by visual observation and the form of each colony was determined by visual observation. Colony Formation Unit-Granulocytes, Erythroids, Macrophages, Megakaryocytes (CFU-GEMM), which are derived from myeloid progenitor cells, the erythroid burst colony-forming cells Burst Forming Unit-Erythroids (BFU-E), the granulocyte/macrophage colony-forming cells Colony Formation Unit-Granulocytes, Macrophages (CFU-GM) were determined on the basis of the forms of colonies, and the numbers were counted.

The results are shown in Tables 12 to 16. Figure 3 shows CFU-GEMM colony counts for culture under (UM171+/-) in the presence of artemether. From Tables 12 to 16 and Figure 3, we were able to confirm increase in the CFU-GEMM colony count was found. In addition, we were also able to confirm the synergistic effect of use of UM171 in combination was found. From these findings with human cells, it was confirmed that those compounds allow hematopoietic stem cells and hematopoietic progenitor cells to be cultured and grow with the self-renewal capacity and multipotency maintained.

**[Table 12]**

| Culture condition | Colony count (colony) after culture (CFU-GEMM) |
|---|---|
| Before culture | 28.5 |
| DMSO | 26.2 |
| Artemether (0.001 µM) | 40.3 |
| Artemether (0.003 µM) | 61.4 |
| Artemether (0.01 µM) | 33.2 |
| Artemether (0.03 µM) | 34.8 |
| Artemether (0.1 µM) | 37.0 |
| Artemether (0.3 µM) | 36.7 |
| Artemether (1 µM) | 63.9 |
| Artemether (3 µM) | 31.7 |
| Artemether (10 µM) | 0.0 |

**[Table 13]**

| Culture condition | Colony count (colony) after culture (CFU-GEMM) |
|---|---|
| Before culture | 20.3 |
| DMSO | 12.6 |
| UM171 (35 nM) and artemether (0.001 µM) | 40.1 |
| UM171 (35 nM) and artemether (0.003 µM) | 204.0 |
| UM171 (35 nM) and artemether (0.01 µM) | 53.8 |
| UM171 (35 nM) and artemether (0.03 µM) | 46.8 |
| UM171 (35 nM) and artemether (0.1 µM) | 94.0 |
| UM171 (35 nM) and artemether (0.3 µM) | 40.0 |
| UM171 (35 nM) and artemether (1 µM) | 0.0 |

**[Table 14]**

| Culture condition | Colony count (colony) after culture (CFU-GEMM) |
|---|---|
| Before culture | 9.0 |
| DMSO | 16.6 |
| Example 1-23 (0.001 µM) | 171.3 |
| Example 1-23 (0.003 µM) | 39.9 |
| Example 1-23 (0.01 µM) | 76.8 |
| Example 1-23 (0.03 µM) | 22.5 |
| Example 1-23 (0.1 µM) | 13.4 |
| Example 1-23 (0.3 µM) | 3.9 |
| Example 1-23 (1 µM) | 12.0 |
| Example 1-23 (3 µM) | 4.5 |
| Example 1-23 (10 µM) | 0.0 |

**[Table 15]**

| Culture condition | Colony count (colony) after culture (CFU-GEMM) |
|---|---|
| Before culture | 20.3 |
| DMSO | 73.2 |
| Example 1-16 (0.001 µM) | 130.6 |
| Example 1-16 (0.003 µM) | 112.2 |
| Example 1-16 (0.01 µM) | 65.1 |
| Example 1-16 (0.03 µM) | 68.8 |
| Example 1-16 (0.1 µM) | 40.6 |
| Example 1-16 (0.3 µM) | 22.9 |
| Example 1-16 (1 µM) | 15.7 |
| Example 1-16 (3 µM) | 10.0 |
| Example 1-16 (10 µM) | 8.8 |

**[Table 16]**

| Culture condition | Colony count (colony) after culture (CFU-GEMM) |
|---|---|
| Before culture | 20.3 |
| DMSO | 33.0 |
| Example 1-7 (0.001 µM) | 43.8 |
| Example 1-7 (0.003 µM) | 33.8 |
| Example 1-7 (0.01 µM) | 74.3 |
| Example 1-7 (0.03 µM) | 83.0 |
| Example 1-7 (0.1 µM) | 64.1 |
| Example 1-7 (0.3 µM) | 19.5 |
| Example 1-7 (1 µM) | 0.0 |
| Example 1-7 (3 µM) | 0.0 |
| Example 1-7 (10 µM) | 0.0 |

### [Example 8: In Vivo Engraftment Test with Human Cells]

Each of artemether and the compound of Example 1-23 was prepared with the cell culture medium at a final concentration of 0.003 µM or 0.01 µM, and added to the above human cells seeded after classification (166 cells were seeded per well) to a final volume of 200 µL/well to act thereon. Thereafter, for each condition, 10000 cells in total of a CD34-positive fraction were cultured in a CO₂ incubator under conditions of 37°C and 5% CO₂ for 1 week, and the cell count was determined. Among the cells after culture, 10000 cells, a number equal to the number of cells before culture, were transplanted into an immunocompromised NOG mouse exposed to a lethal dose of radiation. As a comparative sample, 10000 fresh cord blood-derived CD34-positive cells (uncultured) were transplanted. For peripheral blood donor chimerism at each specified time after transplantation, the human CD45-positive cell count and the mouse CD45-positive cell count were determined by using a human-specific CD45 antibody and a mouse-specific CD45 antibody, and the peripheral blood donor chimerism was represented as the proportion of human CD45-positive cells to the total of human CD45-positive cells and mouse CD45-positive cells (chimerism rate).

Table 17 shows peripheral blood donor chimerism 1 month, 2 months, and 3 months after transplantation, and Table 18 shows those 1 month and 2 months after transplantation.

The results of the transplantation experiment with the same numbers of transplanted cells showed that the cells cultured ex vivo in the presence of artemether or the compound of Example 1-23 exhibited chimerism rates comparable to those of the cells before culture or chimerism rates higher than those of the corresponding DMSO control. In addition, the total cell count increased from before culture. The chimerism rate was significantly higher than that of the corresponding DMSO control (about 10 times). Thus, we were able to confirm from the in vivo engraftment test that the present compounds allow growth culture of long-term hematopoietic stem cells having engraftment potential with their functions maintained.

**[Table 17]**

| Culture condition | Viable cell count (cell) | Donor chimerism (%) | | |
|---|---|---|---|---|
| | | Period after transplantation (month) | | |
| | | 1 | 2 | 3 |
| Fresh | 9960 | 1.243 ± 0.975 | 4.251 ± 5.035 | 4.996 ± 3.932 |
| DMSO | 198409 | 0.081 ± 0.124 | 0.118 ±0.184 | 0.204 ± 0.445 |
| Artemether (0.003 µM | 95954 | 0.041 ± 0.022 | 0.605 ± 0.95 | 2.108 ± 2.479 |

**[Table 18]**

| Culture condition | Viable cell count (cell) | Donor chimerism (%) | | |
|---|---|---|---|---|
| | | Period after transplantation (month) | | |
| | | 1 | 2 | 3 |
| Fresh | 9960 | 0.286 ± 0.329 | 1.302 ± 0.926 | - |
| DMSO | 20262 | 0.014^{∗} | 0.091^{∗} | - |
| Example 1-23 (0.01 µM | 20071 | 0.299 ± 0.049 | 0.748 ± 0.585 | - |

| | | | | |
|---|---|---|---|---|
| ^{∗} Carried out at n = 1 | | | | |

## Claims

1. A method for culturing one or more hematopoietic stem cells, comprising:
culturing a cell population including hematopoietic stem cells in a culture medium containing at least one compound represented by formula (1) or a salt thereof: 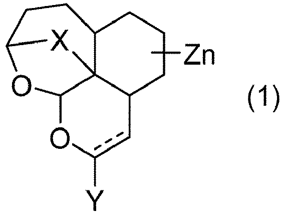 wherein 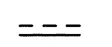
is a single bond or a double bond;
X is O-O or O;
Y is a hydrogen atom, hydroxy, oxo, mercapto, carboxy, carbamoyl, cyano, OR¹, SR¹, SOR¹, SO₂R¹, COR¹, OCOR¹, R², COOR¹, NR³R⁴, NR⁵COR¹, NR⁵SO₂R¹, NR⁵CONR³R⁴, or a halogen;
Z is attached to a carbon at an arbitrary position of the fused ring, and
each Z is independently a C₁₋₆ alkyl;
n is an integer of 0 to 10;
R¹ is an optionally substituted alkyl, an optionally substituted cycloalkyl, an optionally substituted aryl, an optionally substituted heteroaryl, or an optionally substituted aliphatic heterocyclic group;
R² is an optionally substituted alkyl, an optionally substituted alkenyl, an optionally substituted alkynyl, an optionally substituted cycloalkyl, an optionally substituted aryl, an optionally substituted heteroaryl, or an optionally substituted aliphatic heterocyclic group; and
R³, R⁴, and R⁵ are each independently a hydrogen atom, an optionally substituted alkyl, an optionally substituted cycloalkyl, an optionally substituted aliphatic heterocyclic group, an optionally substituted aryl, or
an optionally substituted heteroaryl, wherein R³ and R⁴ can be combined with the adjacent nitrogen atom to form a 4- to 10-membered nitrogen-containing aliphatic heterocyclic ring optionally containing an oxygen atom, a nitrogen atom, or a sulfur atom, additionally constituting one or two rings, and the nitrogen-containing aliphatic heterocyclic ring is optionally substituted.

2. The method for culturing one or more hematopoietic stem cells according to claim 1, wherein the compound of the formula (1) is a compound represented by formula (2): 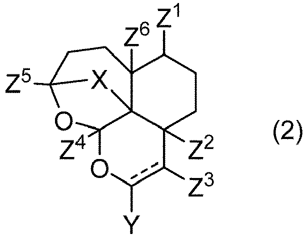 wherein 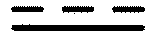
is a single bond or a double bond;
X and Y are as defined in the formula (1); and
Z¹, Z², Z³, Z⁴, Z⁵, and Z⁶ are each independently a hydrogen atom or a C₁₋₆ alkyl.

3. The method for culturing one or more hematopoietic stem cells according to claim 2, wherein the compound of the formula (2) is a compound represented by formula (3-1), (3-2), or (3-3): 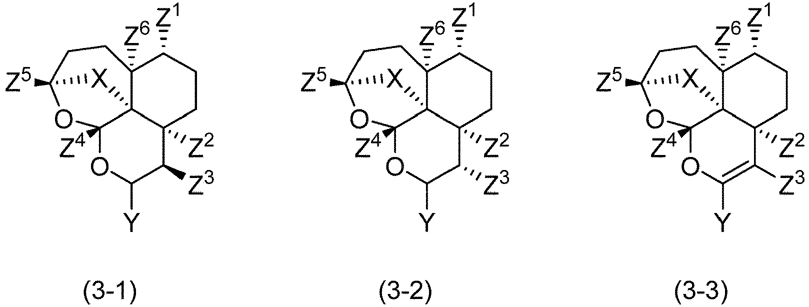 wherein
X and Y are as defined in the formula (1); and
Z¹, Z², Z³, Z⁴, Z⁵, and Z⁶ are each independently a hydrogen atom or a C₁₋₆ alkyl.

4. The method for culturing one or more hematopoietic stem cells according to claim 3, wherein, in the formula (3-1), (3-2), or (3-3), Z¹, Z², Z³, Z⁴, Z⁵, and Z⁶ are each independently a hydrogen atom or methyl.

5. The method for culturing one or more hematopoietic stem cells according to claim 1, wherein the compound of the formula (1) is a compound represented by formula (4-1), (4-2), or (4-3): 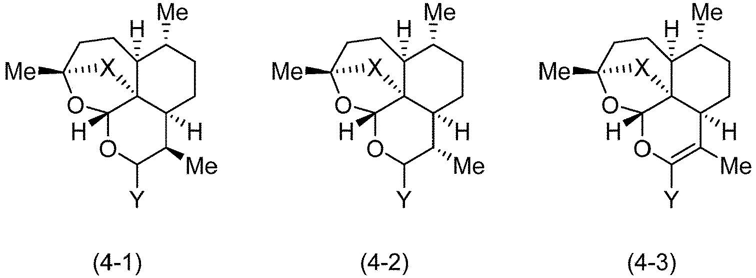 wherein
X and Y are as defined in the formula (1).

6. The method for culturing one or more hematopoietic stem cells according to any one of claims 1 to 5, wherein Y is a hydrogen atom, hydroxy, oxo, carboxy, carbamoyl, OR¹, SR¹, SO₂R¹, COR¹, OCOR¹, R², NR³R⁴, NR⁵COR¹, NR⁵SO₂R¹, or a fluorine atom.

7. The method for culturing one or more hematopoietic stem cells according to any one of claims 1 to 6, wherein,
if 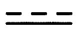
is a double bond, then Y is a hydrogen atom or hydroxy, and
if 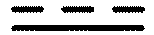
is a single bond, then
Y is hydroxy, oxo, carboxy, carbamoyl, OR¹, SR¹, SO₂R¹, OCOR¹, R², NR³R⁴, NR⁵COR¹, or a fluorine atom;
R¹ is a C₁₋₈ alkyl optionally substituted with one to seven substituents selected from Group 1, a C₃₋₈ cycloalkyl optionally substituted with one to three groups selected from Group 1, phenyl optionally substituted with one to five groups selected from Group 1, a 4- to 10-membered aliphatic heterocyclic group containing one to four endocyclic heteroatoms selected from an oxygen atom, a nitrogen atom, and a sulfur atom and optionally substituted with one to three groups selected from Group 1, or a 5- to 10-membered heteroaryl containing one to three endocyclic heteroatoms selected from an oxygen atom, a nitrogen atom, and a sulfur atom and optionally substituted with one to four groups selected from Group 1;
R² is a C₁₋₈ alkyl optionally substituted with one to seven substituents selected from Group 2, a C₁₋₈ alkenyl optionally substituted with one to seven substituents selected from Group 2, a C₃₋₈ cycloalkyl optionally substituted with one to three groups selected from Group 1, phenyl optionally substituted with one to five groups selected from Group 1, a 4-to 10-membered aliphatic heterocyclic group containing one to four endocyclic heteroatoms selected from an oxygen atom, a nitrogen atom, and a sulfur atom and optionally substituted with one to three groups selected from Group 1, or a 5- to 10-membered heteroaryl containing one to three endocyclic heteroatoms selected from an oxygen atom, a nitrogen atom, and a sulfur atom and optionally substituted with one to four groups selected from Group 1;
R³ and R⁴ are each independently a hydrogen atom, a C₁₋₆ alkyl optionally substituted with one to seven substituents selected from Group 1, or phenyl optionally substituted with one to five groups selected from Group 1, wherein R³ and R⁴ can be combined with the adjacent nitrogen atom to form a 3- to 8-membered nitrogen-containing aliphatic heterocyclic ring optionally containing an oxygen atom, a nitrogen atom, or a sulfur atom, additionally constituting one or two rings, and the nitrogen-containing aliphatic heterocyclic ring is optionally substituted with one to three substituents selected from substituents of Group 1 shown below; and
R⁵ is a hydrogen atom or a C₁₋₃ alkyl:
[Group 1]
carboxy, hydroxy, a C₁₋₈ alkoxy, a C₁₋₈ alkylcarbonyl, a C₁₋₈ alkylcarbonyloxy, a C₁₋₈ alkoxycarbonyl, mercapto, a C₁₋₈ alkylsulfanyl, a C₁₋₈ alkylsulfonyl, carbamoyl optionally substituted with one or two C₁₋₈ alkyls, amino optionally substituted with one or two C₁₋₈ alkyls, C₁₋₈ alkylcarbonylamino, C₁₋₈ alkylsulfonylamino, phenyl optionally substituted with one to five groups selected from Group 3, a 5- or 6-membered heteroaryl containing one to three endocyclic heteroatoms selected from an oxygen atom, a nitrogen atom, and a sulfur atom and optionally substituted with one to four groups selected from Group 3, a C₃₋₈ cycloalkyl optionally substituted with one to three groups selected from Group 3, a 4- to 10-membered aliphatic heterocyclic group containing one to four endocyclic heteroatoms selected from an oxygen atom, a nitrogen atom, and a sulfur atom and optionally substituted with one to three groups selected from Group 3, and a halogen;
[Group 2]
carboxy, hydroxy, a C₁₋₈ alkoxy, a C₁₋₈ alkylcarbonyl, a C₁₋₈ alkylcarbonyloxy, a C₁₋₈ alkoxycarbonyl, mercapto, a C₁₋₈ alkylsulfanyl, a C₁₋₈ alkylsulfonyl, carbamoyl optionally substituted with one or two C₁₋₈ alkyls, amino, phenyl optionally substituted with one to five groups selected from Group 3, a 5- or 6-membered heteroaryl containing one to three endocyclic heteroatoms selected from an oxygen atom, a nitrogen atom, and a sulfur atom and optionally substituted with one to four groups selected from Group 3, a C₃₋₈ cycloalkyl optionally substituted with one to three groups selected from Group 3, a 4- to 8-membered aliphatic heterocyclic group containing one to four endocyclic heteroatoms selected from an oxygen atom, a nitrogen atom, and a sulfur atom and optionally substituted with one to three groups selected from Group 3, and a halogen; and
[Group 3]
carboxy, hydroxy, a C₁₋₈ alkyl, a C₁₋₈ alkoxy, a C₁₋₈ alkylcarbonyl, a C₁₋₈ alkylcarbonyloxy, a C₁₋₈ alkoxycarbonyl, mercapto, a C₁₋₈ alkylsulfanyl, a C₁₋₈ alkylsulfonyl, amino optionally substituted with one or two C₁₋₈ alkyls, carbamoyl optionally substituted with one or two C₁₋₈ alkyls, a C₁₋₈ alkylcarbonylamino, a C₁₋₈ alkylsulfonylamino, and a halogen.

8. The method for culturing one or more hematopoietic stem cells according to any one of claims 1 to 7, wherein 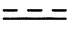
is a single bond;
Y is hydroxy, oxo, carboxy, carbamoyl, OR¹, SR¹, SO₂R¹, OCOR¹, R², NR³R⁴, NR⁵COR¹, NR⁵SO₂R¹, or a fluorine atom;
R¹ is a C₁₋₄ alkyl optionally substituted with one to three groups selected from Group 1', a C₃₋₆ cycloalkyl optionally substituted with one to three groups selected from Group 1', phenyl optionally substituted with one to three groups selected from Group 1', a 4- to 6-membered aliphatic heterocyclic group containing one to three endocyclic heteroatoms selected from an oxygen atom, a nitrogen atom, and a sulfur atom and optionally substituted with one to three groups selected from Group 1', or a 5- or 6-membered heteroaryl containing one to three endocyclic heteroatoms selected from an oxygen atom, a nitrogen atom, and a sulfur atom and optionally substituted with one to three groups selected from Group 1';
R² is a C₁₋₄ alkyl optionally substituted with one to three groups selected from Group 2', a C₃₋₆ cycloalkyl optionally substituted with one to three groups selected from Group 1', phenyl optionally substituted with one to three groups selected from Group 1', a 4- to 6-membered aliphatic heterocyclic group containing one to three endocyclic heteroatoms selected from an oxygen atom, a nitrogen atom, and a sulfur atom and optionally substituted with one to three groups selected from Group 1', or a 5- or 6-membered heteroaryl containing one to three endocyclic heteroatoms selected from an oxygen atom, a nitrogen atom, and a sulfur atom and optionally substituted with one to three groups selected from Group 3';
R³ is a hydrogen atom or a C₁₋₃ alkyl;
R⁴ is a hydrogen atom, a C₁₋₄ alkyl optionally substituted with one to three groups selected from Group 1', a C₃₋₆ cycloalkyl optionally substituted with one to three groups selected from Group 1', phenyl optionally substituted with one to three groups selected from Group 1', a 4- to 6-membered aliphatic heterocyclic group containing one to three endocyclic heteroatoms selected from an oxygen atom, a nitrogen atom, and a sulfur atom and optionally substituted with one to three groups selected from Group 1', or a 5- or 6-membered heteroaryl containing one to three endocyclic heteroatoms selected from an oxygen atom, a nitrogen atom, and a sulfur atom and optionally substituted with one to three groups selected from Group 1',
wherein R³ and R⁴ can be combined with the adjacent nitrogen atom to form a 3- to 8-membered nitrogen-containing aliphatic heterocyclic ring optionally containing an oxygen atom, a nitrogen atom, or a sulfur atom, additionally constituting one or two rings, and the nitrogen-containing aliphatic heterocyclic ring is optionally substituted with one to three groups selected from substituents of Group 1' shown below; and
R⁵ is a hydrogen atom:
[Group 1']
a fluorine atom, hydroxy, carboxy, amino, a C₁₋₄ alkoxy, a C₁₋₄ alkylcarbonyloxy, phenyl optionally substituted with one to three groups selected from Group 3', a C₃₋₆ cycloalkyl optionally substituted with one to three groups selected from Group 3', and a 4- to 6-membered aliphatic heterocyclic group containing one to three endocyclic heteroatoms selected from an oxygen atom, a nitrogen atom, and a sulfur atom and optionally substituted with one to three groups selected from Group 3';
[Group 2']
a fluorine atom, hydroxy, carboxy, amino, a C₁₋₄ alkoxy, a C₁₋₄ alkylcarbonyloxy, a C₁₋₄ alkoxycarbonyl, carbamoyl optionally substituted with one or two C₁₋₈ alkyls, phenyl optionally substituted with one to three groups selected from Group 3', a C₃₋₆ cycloalkyl optionally substituted with one to three groups selected from Group 3', and a 4- to 6-membered aliphatic heterocyclic group containing one to three endocyclic heteroatoms selected from an oxygen atom, a nitrogen atom, and a sulfur atom and optionally substituted with one to three groups selected from Group 3'; and
[Group 3']
a fluorine atom, hydroxy, and a C₁₋₄ alkyl.

9. The method for culturing one or more hematopoietic stem cells according to any one of claims 1 to 8, wherein X is O-O.

10. The method for culturing one or more hematopoietic stem cells according to claim 1, wherein the compound of the formula (1) is a compound selected from the group consisting of the following compounds:
artemether;
artemisinin;
artenimol;
artemotil;
artesunate;
(3R,5aS,6R,8aS,9R,10R,12S,12aR)-10-fluoro-3,6,9-trimethyldecahydro-12H-3,12-epoxypyrano[4,3-j][1,2]benzodioxepine;
(3R,5aS,6R,8aS,9R,10S,12R,12aR)-3,6,9-trimethyl-10-phenyldecahydro-12H-3,12-epoxypyrano[4,3-j][1,2]benzodioxepine;
(3R,5aS,6R,8aS,9R,10S,12S,12aR)-3,6,9-trimethyl-10-(methylsulfanyl)decahydro-12H-3,12-epoxypyrano[4,3-j] [1,2]benzodioxepine;
(3R,5aS,6R,8aS,9R,10R,12S,12aR)-3,6,9-trimethyl-10-(methylsulfanyl)decahydro-12H-3,12-epoxypyrano[4,3-j][1,2]benzodioxepine;
(3R,5aS,6R,8aS,9R,10S,12S,12aR)-10-(methanesulfonyl)-3,6,9-trimethyldecahydro-12H-3,12-epoxypyrano[4,3-j][1,2]benzodioxepine;
(3R,5aS,6R,8aS,9R,10R,12R,12aR)-3,6,9,10-tetramethyldecahydro-12H-3,12-epoxypyrano[4,3-j][1,2]benzodioxepine;
(3R,5aS,6R,8aS,9R,10R,12R,12aR)-10-(furan-2-yl)-3,6,9-trimethyldecahydro-12H-3,12-epoxypyrano[4,3-j][1,2]benzodioxepine;
(3R,5aS,6R,8aS,9R,10R,12R,12aR)-3,6,9-trimethyldecahydro-12H-3,12-epoxypyrano[4,3-j][1,2]benzodioxepine-10-carboxylic acid;
4-phenyl-1-[(3R,5aS,6R,8aS,9R,10R,12R,12aR)-3,6,9-trimethyldecahydro-12H-3,12-epoxypyrano[4,3-j][1,2]benzodioxepin-10-yl]-1H-1,2,3-triazole;
N-[(3R,5aS,6R,8aS,9R,10R,12R,12aR)-3,6,9-trimethyldecahydro-12H-3,12-epoxypyrano[4,3-j][1,2]benzodioxepin-10-yl]acetamide;
N-[(3R,5aS,6R,8aS,9R,10R,12R,12aR)-3,6,9-trimethyldecahydro-12H-3,12-epoxypyrano[4,3-j][1,2]benzodioxepin-10-yl]methanesulfonamide;
(3R,5aS,6R,8aS,9R,10R,12R,12aR)-N,N,3,6,9-pentamethyldecahydro-12H-3,12-epoxypyrano[4,3-j][1,2]benzodioxepin-10-amine;
4-[(3R,5aS,6R,8aS,9R,10R,12R,12aR)-3,6,9-trimethyldecahydro-12H-3,12-epoxypyrano[4,3-j][1,2]benzodioxepin-10-yl]morpholine;
(3R,5aS,6R,8aS,9R,10S,12R,12aR)-3,6,9-trimethyldecahydro-12H-3,12-epoxypyrano[4,3-j][1,2]benzodioxepin-10-yl acetate;
(3R,5aS,6R,8aS,9R,10S,12R,12aR)-3,6,9-trimethyldecahydro-12H-3,12-epoxypyrano[4,3-j][1,2]benzodioxepine-10-carboxamide;
(3R,5aS,6R,8aS,9R,10R,12R,12aR)-3,6,9-trimethyldecahydro-12H-3,12-epoxypyrano[4,3-j][1,2]benzodioxepine-10-carboxamide;
1-[(3R,5aS,6R,8aS,9R,10S,12R,12aR)-3,6,9-trimethyldecahydro-12H-3,12-epoxypyrano[4,3-j][1,2]benzodioxepin-10-yl]methanamine;
1-[(3R,5aS,6R,8aS,9R,10R,12R,12aR)-3,6,9-trimethyldecahydro-12H-3,12-epoxypyrano[4,3-j][1,2]benzodioxepin-10-yl]methanamine;
(3R,5aS,6R,8aS,9R,10S,12R,12aR)-3,6,9-trimethyl-10-[(propan-2-yl)oxy]decahydro-12H-3,12-epoxypyrano[4,3-j][1,2]benzodioxepine;
(3R,5aS,6R,8aS,9R,10S,12R,12aR)-10-(cyclohexyloxy)-3,6,9-trimethyldecahydro-12H-3,12-epoxypyrano[4,3-j][1,2]benzodioxepine;
(3R,5aS,6R,8aS,9R,10S,12R,12aR)-3,6,9-trimethyl-10-phenoxydecahydro-12H-3,12-epoxypyrano[4,3-j][1,2]benzodioxepine;
(3R,5aS,6R,8aS,9R,10S,12R,12aR)-3,6,9-trimethyl-10-(2,2,2-trifluoroethoxy)decahydro-12H-3,12-epoxypyrano[4,3-j][1,2]benzodioxepine;
(3R,5aS,6R,8aS,9R,10S,12R,12aR)-10-(2-methoxyethoxy)-3,6,9-trimethyldecahydro-12H-3,12-epoxypyrano[4,3-j][1,2]benzodioxepine;
(3R,5aS,6R,8aS,9R,10S,12R,12aR)-10-(benzyloxy)-3,6,9-trimethyldecahydro-12H-3,12-epoxypyrano[4,3-j][1,2]benzodioxepine;
(3R,5aS,6R,8aS,9R,10R,12R,12aR)-3,6,9-trimethyl-10-phenoxydecahydro-12H-3,12-epoxypyrano[4,3-j][1,2]benzodioxepine;
2-{[(3R,5aS,6R,8aS,9R,10S,12R,12aR)-3,6,9-trimethyldecahydro-12H-3,12-epoxypyrano[4,3-j][1,2]benzodioxepin-10-yl]oxy}ethyl acetate;
2-{[(3R,5aS,6R,8aS,9R,10R,12R,12aR)-3,6,9-trimethyldecahydro-12H-3,12-epoxypyrano[4,3-j][1,2]benzodioxepin-10-yl]oxy}ethyl acetate;
(3R,5aS,6R,8aS,12R,12aR)-3,6,9-trimethyl-3,4,5,5a,6,7,8,8a-octahydro-12H-3,12-epoxypyrano[4,3-j][1,2]benzodioxepine;
(3R,3aS,6R,6aS,9S,10aS,10bR)-3,6,9-trimethyloctahydro-10aH-9,10b-epoxypyrano[4,3,2-jk] [2]benzoxepin-2(3H)-one; and
(3R,5aS,6R,8aS,9R,10R,12R,12aR)-10-methoxy-3,6,9-trimethyldecahydro-12H-3,12-epoxypyrano[4,3-j][1,2]benzodioxepine.

11. The method for culturing one or more hematopoietic stem cells according to claim 1, wherein the compound of the formula (1) is selected from the group consisting of artemether, artemisinin, artenimol, artemotil, and artesunate.

12. The method for culturing one or more hematopoietic stem cells according to any one of claims 1 to 11, wherein the hematopoietic stem cell is a CD34-positive cell.

13. The method for culturing one or more hematopoietic stem cells according to any one of claims 1 to 12, wherein the hematopoietic stem cell is a long-term hematopoietic stem cell.

14. The method for culturing one or more hematopoietic stem cells according to any one of claims 1 to 12, wherein the hematopoietic stem cell is a Hoxb5-positive mouse hematopoietic stem cell.

15. The method for culturing one or more hematopoietic stem cells according to any one of claims 1 to 14, wherein a proportion of hematopoietic stem cells to total cell count in the cell population after culture is 10% or more of a proportion of hematopoietic stem cells to total cell count in the cell population before culture.

16. The method for culturing one or more hematopoietic stem cells according to any one of claims 1 to 15, wherein a number of hematopoietic stem cells in the cell population after culture is three or more times a number of hematopoietic stem cells in the cell population before culture.

17. The method for culturing one or more hematopoietic stem cells according to any one of claims 1 to 16, wherein the culture medium is a culture medium further containing UM171 or a derivative thereof.

18. A method for producing one or more hematopoietic stem cells, comprising:
a step of preparing a cell population including hematopoietic stem cells; and
a step of culturing the cell population including hematopoietic stem cells, wherein the cell population including hematopoietic stem cells is cultured by the method for culturing one or more hematopoietic stem cells according to any one of claims 1 to 17.

19. A hematopoietic stem cell obtained by culturing by the method for culturing one or more hematopoietic stem cells according to any one of claims 1 to 17.

20. A hematopoietic stem cell produced by the method for producing one or more hematopoietic stem cells according to claim 18.

21. A reagent for culturing one or more hematopoietic stem cells, comprising at least one compound represented by the formula (1) or a salt thereof.
